(19)

**Europäisches Patentamt**
**European Patent Office**
**Office européen des brevets**

(11)  **EP 3 884 502 B1**

(12)  # EUROPEAN PATENT SPECIFICATION

(45) Date of publication and mention
of the grant of the patent:
**09.11.2022 Bulletin 2022/45**

(21) Application number: **19809012.8**

(22) Date of filing: **18.11.2019**

(51) International Patent Classification (IPC):
**G16B 20/10** (2019.01) **G16B 30/10** (2019.01)
**G16B 40/20** (2019.01) **G16B 40/30** (2019.01)
**C12Q 1/6883** (2018.01)

(52) Cooperative Patent Classification (CPC):
**G16B 20/10; C12Q 1/6883; G16B 30/10;**
**G16B 40/20; G16B 40/30;** C12Q 2600/156

(86) International application number:
**PCT/EP2019/081688**

(87) International publication number:
**WO 2020/104394 (28.05.2020 Gazette 2020/22)**

(54) **METHOD AND COMPUTER PROGRAM PRODUCT FOR ANALYSIS OF FETAL DNA BY MASSIVE SEQUENCING**

METHODE UND COMPUTERPROGRAMM PRODUKT ZUR ANALYSE FÖTALER DNA DURCH MASSIVES SEQUENZIEREN

PROCEDE ET PRODUIT PROGRAMME INFORMATIQUE POUR L'ANALYSE DE L'ADN FOETAL PAR SEQUENCAGE MASSIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.11.2018 EP 18382824**
**07.02.2019 EP 19382087**

(43) Date of publication of application:
**29.09.2021 Bulletin 2021/39**

(73) Proprietor: **Sistemas Genómicos, S.L.**
**46980 Valencia (ES)**

(72) Inventors:
- **TRIVIÑO PARDO, Juan Carlos**
  **46980 Valencia (ES)**
- **RODRÍGUEZ CRUZ, Óscar**
  **46980 Valencia (ES)**
- **MIÑAMBRES HERRAIZ, Rebeca**
  **46980 Valencia (ES)**
- **FERNÁNDEZ PEDROSA, María Victoria**
  **46980 Valencia (ES)**
- **COLLADO DÍAZ, María**
  **46980 Valencia (ES)**
- **TENA ROS, Raquel**
  **46980 Valencia (ES)**

- **LOIS OLMO, Sergio**
  **46980 Valencia (ES)**
- **COLLADO MICÓ, María Carmen**
  **Valencia 46980 (ES)**

(74) Representative: **Manuel Illescas y Asociados, S.L.**
**C/ Príncipe de Vergara 197, Oficina 1°A**
**28002 Madrid (ES)**

(56) References cited:
**US-A1- 2018 089 364**

- **ROY STRAVER ET AL: "Calculating the fetal fraction for noninvasive prenatal testing based on genome-wide nucleosome profiles : Direct fetal fraction determination on NIPT data", PRENATAL DIAGNOSIS, vol. 36, no. 7, 20 May 2016 (2016-05-20), pages 614-621, XP055478984, GB ISSN: 0197-3851, DOI: 10.1002/pd.4816**
- **NITSCHE JOSHUA F ET AL: "29: The effect of maternal heparins and/or aspirin on the amount of cell-free fetal DNA in the maternal circulation", AMERICAN JOURNAL OF OBSTETRICS & GYNECOLOGY, vol. 216, no. 1, January 2017 (2017-01), XP029873278, ISSN: 0002-9378, DOI: 10.1016/J.AJOG.2016.11.920**

**Description**

**FIELD OF THE INVENTION**

The present invention belongs to the technical field of Non-invasive Prenatal Diagnosis. In particular, it refers to the methods that use massive sequencing (NGS, *Next Generation Sequencing*) and bioinformatics analysis of the sequences obtained for determining, in a maternal plasma sample containing maternal DNA and fetal DNA, the percentage of fetal DNA, alterations in the number of chromosomes (complete chromosomal fetal aneuploidies), alterations in the number of chromosome fragments (partial fetal aneuploidies) and the absence or presence of Y chromosome, in the fetus.

**BACKGROUND**

During pregnancy, small fragments of fetal DNA from the cytotrophoblast are released into the bloodstream and circulate together with other fragments of maternal DNA in the maternal blood plasma.

The circulating fetal DNA in maternal blood is cell-free DNA (cfDNA). This circulating fetal DNA can be detected in the maternal plasma from 5-7 weeks; however, monitoring tests are more accurate from week 10 since the quantity of circulating fetal DNA increases with gestational age.

Document WO2013132305A1 discloses a method to estimate the fraction of fetal DNA, wherein the size of DNA fragments is calculated, a parameter based on the quantity of DNA fragments of different sizes is calculated, which provides a statistical measurement of the size profile of the DNA fragments, the parameter thus calculated is compared with calibration sample values and the fraction of fetal DNA is determined based on this comparison. The size of the DNA fragments is obtained through massive sequencing and later alignment of the sequences. The calibration samples have known fetal fractions measured according to various techniques. Said document WO2013132305A1 discloses different forms to calculate the parameter, e.g. dividing the number of fragments of a certain size by the total number of fragments, or dividing the number of fragments of a certain size, or within a range of sizes, by the number of fragments of another size or range. Said document discloses that the DNA fragments may be randomly chosen or preferably selected.

Document WO2015061359A1 discloses a method to identify variations in the number of chromosomes (complete chromosomal aneuploidies) and in the number of copies (CNV, *Copy Number Variation,* partial chromosomal aneuploidies) in the fetus. The variations in the number of chromosomes that can be determined with this method include trisomies, monosomies of chromosomes 1-22, X and Y. The method can also determine variations in the number of copies of a sequence of a nucleic acid of interest, i.e. deletions and duplications of a determined size. The method disclosed in said document includes obtaining sequence reads by massive sequencing of mixtures of maternal and fetal DNA, aligning the sequence reads to a reference genome, dividing the reference genome into bins, determining the coverage of the sequence reads in each bin, obtaining an overall profile comprising the expected coverage in each bin, obtained from a reference set with unaffected samples (diploids), adjusting the coverage using the expected coverage in each bin, obtaining coverages corrected by the overall profile, adjusting the corrected coverages based on a correction by the guanine and cytosine content (GC content) thus obtaining coverages corrected by the GC content and evaluating fetal aneuploidies based on the coverages corrected by the GC content. Said document discloses the use of this method in determination of the presence of the Y chromosome and in determination of the fetal sex.

Document WO2013109981A1 discloses a method to detect the presence or absence of a fetal aneuploidy which includes obtaining sequence reads of mixtures of maternal and fetal DNA, mapping the sequence reads to genomic sections of reference, counting the number of sequence reads that map to each genomic section of reference, normalizing the number of reads for a genomic section based on an expected number of reads for a group which includes samples and references, exposed to one or more experimental conditions and provides an indicative value of the presence or absence of fetal aneuploidy from the normalized reads value. The expected number of reads is a statistical value, such as the median or average. Said document discloses that the value of normalized reads is adjusted by the GC content of the reads. Document US 2018/089364 A1 discloses a method for determining the percentage of fetal DNA present in a maternal plasma sample containing maternal DNA and fetal DNA, comprising identifying genomic regions enriched in fetal DNA, extracting the DNA present in maternal plasma control samples, containing maternal DNA and fetal DNA, on which the percentage of fetal DNA has previously been determined, synthesizing libraries of the DNA present in said control samples to be sequenced, by massive sequencing, amplifying and PCR labelling said DNA libraries and combining said DNA libraries, obtaining sequence reads, by massive sequencing, of said DNA libraries, aligning said sequence reads against a reference genome, dividing each chromosome into bins with a base size from 50 to 200 Kb, and calculating the number of sequence reads of each bin, normalizing the number of sequence reads of each bin based on the total number of sequence reads of all the bins, performing a linear regression for each bin, wherein the independent variable is the normalized number of sequence reads of each bin, the dependent variable is the percentage of fetal DNA of said control samples and the co-variables are: gestational week, mother's height, mother's weight, aligning sequencing reads to bins, using a trained machine-learning model, e.g. linear regression, to identify the fetal fraction based on the counts in each or the bins.

## SUMMARY OF THE INVENTION

The problem of the prior art consists of providing an improvement in the analysis precision of the fetal DNA present in a maternal plasma sample containing maternal DNA and fetal DNA, by massive sequencing, wherein said improvement in the precision is determined as: (i) a reduction in the error associated with the value of the percentage of DNA determined with the method of the invention, in comparison with the percentage of DNA determined with other methods, such as the chrY method and digital PCR; (ii) a greater sensitivity in the alterations in the number of chromosomes or chromosome fragments determined with the method of the invention; (iii) a reduction in the number of false positives in the alterations in the number of chromosomes or chromosome fragments determined with the method of the invention; (iv) a reduction in the number of false positives in the absence or presence of the Y chromosome determined with the method of the invention.

The method for determining the percentage of fetal DNA of the invention, based on the identification of genomic regions enriched in fetal DNA, obtaining a first nucleosome profile comprising determining a distribution of the number of sequence reads of each bin that map to said genomic regions enriched in fetal DNA against the size of the fragments corresponding to each read obtained for said sample, determination of a nucleosome profile prediction in said genomic regions enriched in fetal DNA based on a Random Forest model which includes at least the variables: gestational week, mother's height and weight; and comparison of said nucleosome profile prediction with said first nucleosome profile, provides a solution to this technical problem.

The improvement in the precision, determined as a value of the error associated with the value of the percentage of DNA determined with the method of the invention, in comparison with the percentage of DNA determined with other methods, such as the chrY method and digital PCR, provided by the method for determining the percentage of fetal DNA of the invention is based on the aforementioned features. The present patent application shows that the method for determining the percentage of fetal DNA of the invention reduces the error in the determination of the percentage of fetal DNA, in comparison with the methods for determining the percentage of fetal DNA of the prior art.

The method for determining the percentage of fetal DNA of the invention uses a Random Forest prediction model which includes at least the variables: gestational week, mother's height, mother's weight and mother's intake of heparin. These physiological data are associated with the alteration in the quantity of fetal DNA present in the maternal plasma and the method and computer program product of the invention improves the precision as it incorporates said physiological data. The method of the invention considers predictor variables defined as informative by the Random Forest model. The gestational weeks is one of the predictor variables and is especially important for early weeks, such as week 9 or 10, or for late weeks, after week 16. The mother's weight is another of the predictor variables. Mother's intake of heparin is another of the predictor variables. The method of the invention considers these variables and is thus more precise in early weeks, such as week 9 or 10 and in late weeks, after week 16, in extreme cases, such as obese women and wherein the woman has taken heparin prior to the extraction of the plasma sample.

The method of the invention determines the percentage of fetal DNA in samples of women pregnant with male and female fetuses, in contrast to the chrY method, wherein it is only possible for determining the percentage of fetal DNA in samples of women pregnant with male fetuses.

The method for determining whether a fetus having fetal DNA is at risk of having alterations in the number of at least one chromosome and/or at least one chromosome fragment of the invention, based on the determination of the percentage of fetal DNA, according to the method for determining the percentage of fetal DNA of the invention and the adjustment of the number of copies of at least one chromosome or the number of copies of at least one chromosome fragment based on the value of the percentage of fetal DNA obtained with the method for determining the percentage of fetal DNA, provides a solution to this technical problem.

The method for determining the percentage of DNA of the present invention provides a greater precision in the determination of the percentage of fetal DNA and, in turn, a greater precision in the determination of alterations in the number of at least one chromosome and/or at least one chromosome fragment, based on the previously described features.

The method for determining absence or presence of the Y chromosome, based on the selection of bins with statistically significant differences between DNA control samples obtained from female subjects and control samples obtained from male subjects, provides a solution to this technical problem.

The embodiments of the method and computer program product of analysis of fetal DNA present in a maternal plasma sample containing maternal DNA and fetal DNA, by massive sequencing, of the invention are related to one another by a common inventive element, the method and computer program product for determining the percentage of fetal DNA of the invention, which provides an improvement in the precision of the analysis of the fetal DNA, wherein said precision is determined as: (i) a reduction in the error associated with the value of the percentage of DNA determined with the method of the invention, in comparison with the percentage of DNA determined with the chrY method; (ii) a greater sensitivity in the alterations in the number of chromosomes or chromosome fragments determined with the method of the invention; (iii) a reduction in the number of false positives in the alterations in the number of chromosomes or chromosome fragments determined with the method of the invention; (iv) a reduction in the number of false positives in the absence or presence of the Y chromosome determined with the method of the invention.

## DETAILED DESCRIPTION OF THE INVENTION

The present invention provides a method for determining the percentage of fetal DNA in a maternal plasma test sample containing maternal DNA and fetal DNA, comprising:

(a) calculating a reference random forest regression model for predicting percentage of fetal DNA, comprising the steps of:

(i) extracting DNA present in reference samples and repairing said DNA, wherein said reference samples are maternal plasma reference samples containing maternal DNA and fetal DNA from fetuses with a set of euploid chromosomes and wherein the percentage of fetal DNA in said reference samples has previously been determined,

(ii) synthesising libraries of the DNA present in said reference samples, amplifying and labelling said libraries by PCR,

(iii) obtaining sequence reads, by massive sequencing, of said amplified and labelled libraries,

(iv) aligning said sequence reads against a reference genome,

(v) dividing the sequence of each chromosome into first bins with a base size from 50 to 200 Kb, with a first sliding bin with a base size from 5 to 15 Kb and obtaining bin counts by counting the number of sequence reads in each said first bin, wherein each first bin base size is the same, wherein each first sliding bin base size is the same and wherein each first sliding bin base size is less than or equal to the first bin base size,

(vi) obtaining normalised bin counts by normalising said bin counts obtained in step (a)(v) based on the total bin counts in all first bins in said reference samples,

(vii) calculating a regression for each first bin using all said reference samples using the normalised bin count as the independent variable, using percentage of fetal DNA of said reference samples as the dependent variable, and using gestation week, mother's height, mother's weight and mother's intake of heparin as co-variables,

(viii) identifying genomic regions enriched in fetal DNA, wherein said genomic regions enriched in fetal DNA are those for which the statistical significance in the regression of the previous step, as determined by the p value, is greater than $10^{-10}$ and the Spearman correlation coefficient between percentage of fetal DNA predicted by said regression and the percentage of fetal DNA previously determined is greater than or equal to 0.5 and wherein said regions are not regions of the X chromosome,

(ix) obtaining a density of normalised bin counts of reads for each said reference sample which map to said genomic regions enriched in fetal DNA against insert size of reads for each said reference sample which map to said genomic regions enriched in fetal DNA and obtaining a density curve from said values obtained, wherein said density curve is a reference nucleosome profile,

(x) dividing said reference nucleosome profile into second bins of insert sizes with a base size from 2 to 100 nucleotides, with a second sliding bin with a base size from 1 to 20 nucleotides, wherein each second bin base size is the same, wherein each second sliding bin base size is the same and wherein each second sliding bin base size is less than or equal to the second bin base size and calculating the value of first variables selected from the group consisting of mean, minimum, maximum and the accumulative area under the curve for each second bin,

(xi) determining the variable importance of said first variables in said references samples by a random forest classification and determining a set of first predictive variables in said reference samples, wherein said predictive variables in said set are the n-most important variables as classified by said variable importance, wherein n is in a range from 100 to 300,

(xii) generating a first matrix containing the values in said reference samples of second predictive variables, wherein said second predictive variables comprise said first predictive variables and further comprise gestation week, mother's height, mother's weight and mother's intake of heparin,

(xiii) calculating a reference random forest regression model for predicting percentage of fetal DNA by growing random forest regression trees for said reference samples using part of said reference samples as a training set and part of said reference samples as a test set and using said first matrix containing the values of said second predictive variables as input to said reference random forest regression model, and by obtaining an average based on the trees obtained; and

4

(b) determining the percentage of fetal DNA in said test sample, comprising:

(i) extracting DNA from said test sample and repairing said DNA,
(ii) synthesizing libraries of the DNA present in said test sample, amplifying and labelling said libraries by PCR,
(iii) obtaining sequence reads, by massive sequencing, of said amplified and labelled libraries,
(iv) aligning said sequence reads against a reference genome,
(v) dividing the sequence of each chromosome into first bins as per step (a)(v) with a base size from 50 to 200 Kb, with a first sliding bin as per step (a)(v) with a base size from 5 to 15 Kb and obtaining bin counts by counting the number of sequence reads in each bin in said test sample,
(vi) obtaining normalised bin counts by normalising said bin counts obtained in step (b)(v) based on the total bin counts in all first bins in said test sample,
(vii) obtaining a density of normalised bin counts of reads for said test sample which map to said genomic regions enriched in fetal DNA against insert size of reads in said test sample which map to said genomic regions enriched in fetal DNA and obtaining a density curve from said values obtained, wherein said density curve is a test nucleosome profile,
(viii) dividing said test nucleosome profile into second bins as per step (a)(x) with a base size from 2 to 100 nucleotides, with a second sliding bin as per step (a)(x) with a base size from 1 to 20 nucleotides and calculating the value of said first variables selected from the group consisting of mean, minimum, maximum and the accumulative area under the curve for each second bin,
(ix) generating a second matrix containing the values in said test sample of said second predictive variables obtained in step (a)(xii) and
(x) determining the value of the percentage of fetal DNA in the test sample by growing random forest regression trees using said reference random forest model for predicting percentage of fetal DNA calculated in step (a)(xiii), using said second matrix containing the values in said test sample of second predictive variables as input to said reference random forest model and obtaining estimated values of the percentage of fetal DNA for each tree in the test sample, the average of said estimated values being the percentage of fetal DNA in said test sample.

Said method of the invention consists of a method partially implemented by computer. In said method of the invention, steps (a)(iv)-(a)(xiii) and (b)(iv)-(b)(x) are computer implemented steps. Steps (a)(iii) and (b)(iii) are steps partially implemented by computer.

Said method of the invention is based on bidirectional whole genome massive sequencing techniques.

According to the hypothesis most widely-accepted by the scientific community, the fetal DNA comes from the placenta apoptosis process. The size of the fetal DNA is statistically smaller than the size of the maternal DNA and this difference is caused by the nucleosomes. This is due to the fact that the fetus has a nucleosome profile statistically different to the mother since the sizes of maternal DNA are approximately the same as the sizes corresponding to the nucleosome with a fragment of bound link DNA, whilst the fetal DNA is biased towards smaller sizes than those of the maternal DNA and of same or smaller size than the nucleosome.

Some methods are based on the size of the sequence reads to estimate the proportion of fetal DNA (Lam et al. 2018). However, it is a statistical enrichment process and it cannot be indicated, a priori, as a clearly differentiating element.

Mother's intake of heparin variable relates to whether the mother has or has not taken heparin prior to the extraction of the plasma sample.

In an embodiment of the method of the invention, the regression analysis of step (a)(vii) includes the "presence of twins" variable as co-variable and the determination of the nucleosome profile prediction in the genomic regions enriched in fetal DNA based on a Random Forest model also includes said variable, together with the other variables (gestational week, mother's height and weight, mother's intake of heparin).

In an embodiment of the method of the invention, said first bins have a base size of 50 Kb and said first sliding bin has a base size of 5 Kb.

In an embodiment of the method of the invention, each chromosome is divided into bins with a base size of 100 Kb, with a sliding bin with a base size of 10 Kb.

The technical effect associated with the determination of the percentage of fetal DNA based on the nucleosome profile in genomic regions enriched in fetal DNA consists of the elimination of noisy and unreproducible genomic areas, centring the method on only those areas with greater quality and reproducibility. This is translated into a greater precision of the estimation method of fetal DNA against technical and/or biological fluctuations described in the prior art.

The Random Forest model used in step (b)(x) includes, at least, the variables: gestation week, mother's height, mother's weight and mother's intake of heparin. The incorporation of said variables increases the precision of the method as they are variables of a high importance and it enables being more precise to fluctuations associated with changes in the value of these variables.

In comparison with other methods of the prior art, the method of the invention provides more robust, and statistically significant results and greater stability in the analysis.

The method of the invention may be applied both to male and female fetuses.

In an embodiment of the method of the invention, said second bins have a base size of 5 nucleotides and said second sliding bin has a base size of 1 nucleotide.

In an embodiment of the method of the invention, said regression is selected from the group consisting of linear regression, exponential regression and logarithmical regression.

In an embodiment of the method of the invention, n is in a range selected from the group consisting of 120 to 280, 140 to 260, 160 to 240 and 180 to 220.

In a preferred embodiment, n is 200.

The present invention further provides a computer program product comprising a computer-readable medium, wherein instructions are coded to control a computer system comprising means for determining the percentage of fetal DNA in a maternal plasma test sample containing maternal DNA and fetal DNA according to the method of the invention, said computer system comprising the following means for calculating a reference random forest regression model for predicting percentage of fetal DNA:

(a) means for recording DNA sequence reads obtained from reference samples, wherein said reference samples are maternal plasma reference samples containing maternal DNA and fetal DNA from fetuses with a set of euploid chromosomes and wherein the percentage of fetal DNA in said reference samples has previously been determined,

(b) means for aligning said sequence reads against a reference genome,

(c) means for dividing the sequence of each chromosome into first bins with a base size from 50 to 200 Kb, with a first sliding bin with a base size from 5 to 15 Kb and obtaining bin counts by counting the number of sequence reads in each said first bin, wherein each first bin base size is the same, wherein each first sliding bin base size is the same and wherein each first sliding bin base size is less than or equal to the first bin base size,

(d) means for obtaining normalised bin counts by normalising said bin counts based on the total bin counts of all first bins in said reference samples,

(e) means for calculating a regression for each first bin in said reference samples using the normalised bin count as the independent variable, using percentage of fetal DNA of said reference samples as the dependent variable, and using gestation week, mother's height, mother's weight and mother's intake of heparin as co-variables,

(f) means for identifying genomic regions enriched in fetal DNA, wherein said genomic regions enriched in fetal DNA are those for which the statistical significance in the previous regression, as determined by the p value, is greater than $10^{-10}$ and the Spearman correlation coefficient between percentage of fetal DNA predicted by said regression and the percentage of fetal DNA previously determined is greater than or equal to 0.5 and wherein said regions are not regions of the X chromosome,

(g) means for obtaining a density of normalised bin counts of reads for each said reference sample which map to said genomic regions enriched in fetal DNA against insert size of reads for each said reference sample which map to said genomic regions enriched in fetal DNA and obtaining a density curve from saidvalues obtained, wherein said density curve is a reference nucleosome profile,

(h) means for dividing said reference nucleosome profile into second bins of insert sizes with a base size from 2 to 100 nucleotides, with a second sliding bin with a base size from 1 to 20 nucleotides, wherein each second bin base size is the same, wherein each second sliding bin base size is the same and wherein each second sliding bin base size is less than or equal to the second bin base size and calculating the value of first variables selected from the group consisting of mean, minimum, maximum and the accumulative area under the curve for each second bin,

(i) means for determining the variable importance of said first variables in said references samples by a random forest classification and determining a set of first predictive variables in said reference samples, wherein said predictive variables in said set are the n-most important variables as classified by said variable importance, wherein n is in a range from 100 to 300,

(j) means for generating a first matrix containing the values in said reference samples of second predictive variables, wherein said second predictive variables comprise said first predictive variables and further comprise gestation week, mother's height, mother's weight and mother's intake of heparin,

(k) means for calculating a reference random forest regression model for predicting percentage of fetal DNA by growing random forest regression trees for said reference samples using part of said reference samples as a training set and part of said reference samples as a test set and using said first matrix containing the values of said second predictive variables as input to said reference random forest regression model, and by obtaining an average based on the trees obtained; and,

wherein said computer system additionally comprises the following means for determining the percentage of fetal DNA in said test sample:

(l) means for recording DNA sequence reads obtained from said test sample,

(m) means for aligning said sequence reads against a reference genome,

(n) means for dividing the sequence of each chromosome into first bins with a base size from 50 to 200 Kb, with a first sliding bin with a base size from 5 to 15 Kb and obtaining bin counts by counting the number of sequence reads in each bin in said test sample,

(o) means for obtaining normalised bin counts by normalising said bin counts based on the total bin counts in all first bins in said test sample,

(p) means for obtaining a density of normalised bin counts of reads for said test sample which map to said genomic regions enriched in fetal DNA against insert size of reads in said test sample which map to said genomic regions enriched in fetal DNA and obtaining a density curve from said values obtained, wherein said density curve is a test nucleosome profile,

(q) means for dividing said test nucleosome profile into second bins with a base size from 2 to 100 nucleotides, with a second sliding bin with a base size from 1 to 20 nucleotides and calculating the value of said first variables selected from the group consisting of mean, minimum, maximum and the accumulative area under the curve for each second bin,

(r) means for generating a second matrix containing the values in said test sample of said second predictive variables obtained with means (j), and

(s) means for determining the value of the percentage of fetal DNA in the test sample by growing random forest regression trees using said reference random forest model for predicting percentage of fetal DNA calculated withe means (k), using said second matrix containing the values in said test sample of second predictive variables as input to said reference random forest model and obtaining an average based on the trees obtained.

In a preferred embodiment of the computer program product, each chromosome is divided into bins with a base size of 100 Kb, with a sliding bin with a base size of 10 Kb.

In an embodiment of the computer program product of the invention, said first bins have a base size of 50 Kb and said sliding bin has a base size of 5 Kb.

In an embodiment of the computer program product of the invention, said second bins have a base size of 5 nucleotides and said second sliding bin has a base size of 1 nucleotide.

The present invention further provides a method for determining whether a fetus having fetal DNA is at risk of having alterations in the number of at least one chromosome and/or at least one chromosome fragment, wherein said alterations in the number of chromosomes are complete chromosomal fetal aneuploidies and said alterations in the number of chromosome fragments are partial fetal aneuploidies, in a maternal plasma test sample containing maternal DNA and said fetal DNA, said method comprising:

(a) extracting DNA from said test sample and repairing said DNA,

(b) synthesising libraries of the DNA present in said test sample, amplifying and labelling said libraries by PCR,

(c) obtaining sequence reads, by massive sequencing, of said amplified and labelled libraries,

(d) aligning said sequence reads against a reference genome,

(e) determining, according to the method for determining the percentage of fetal DNA of the invention, the percentage of fetal DNA in said test sample,

(f) dividing the sequence of each chromosome into third bins with a base size from 100 Kb to 1 Mb and obtaining bin counts by counting the number of sequence reads in each third bin in said test sample,

(g) recovering bin counts in each third bin in second reference samples, wherein said bin counts in said second reference samples have been previously obtained according to steps (a)-(f) and wherein said second reference samples are maternal plasma reference samples containing maternal DNA and fetal DNA from fetuses with a set of euploid chromosomes,

(h) followed by the following steps:

(i) determining all autosomal third bins as normal bins in said test sample,

(ii) obtaining coverage normalisation coefficients in said test sample and said second reference samples by dividing the bin counts in each said normal bins by the mean of the total counts in said test sample and said second reference samples for all said normal bins,

(iii) obtaining normalised bin counts in said test sample and said second reference samples by multiplying said bin counts of all bins in said test sample and second reference samples by said coverage normalisation coefficients,

(iv) calculating a GC-content model for said test sample and a GC-content model for each said second reference sample by regression, using the GC-content of said normal bins as the independent variable and using said normalised bin counts as the dependent variable and obtaining predicted bin counts of all bins in said test sample and said second reference samples with said GC-content models,

(v) obtaining residuals by calculating differences between the normalised bin counts of all third bins and said predicted bin counts of all third bins in said test sample and said second reference samples,

(vi) obtaining standardised residuals of all third bins in said test sample by standardising said residuals of all third bins in said test sample by means of a Z-score obtained for each bin by comparing said residual of each third bin in said test sample to said residual of the corresponding third bin in said second reference samples,

(vii) obtaining first-scaled standardised residuals in said test sample by multiplying said standardised residuals in said test sample by a first scaling coefficient, said first scaling coefficient being the inverse of said percentage of fetal DNA determined in step (e),

(viii) determining bin copy numbers for each third bin by multiplying said first-scaled standardised residuals by a second scaling coefficient, thereby obtaining second-scaled standardised residuals and by adding a shifting coefficient to said second-scaled standardised residuals, wherein said second scaling coefficient has been previously determined from first-scaled standardised residuals and bin copy numbers for each third bin in third reference samples, wherein said third reference samples are maternal plasma reference samples containing maternal DNA and fetal DNA from fetuses with a set of aneuploid chromosomes, wherein said shifting coefficient has been previously determined from first-scaled standardised residuals and bin copy numbers for each third bin in said second reference samples,

(ix) eliminating third bins having a copy number different determining aneuploidy from the group of said normal bins in said test sample,

(x) repeating steps (h)(ii) to (h)(ix),

(xi) repeating step (h)(x) if said coverage normalisation coefficients differ more than a value in the range 0.1-5% with respect to previous said coverage normalisation coefficients,

(i) obtaining groups of contiguous bins by segmentation, thereby obtaining segments having the same copy number, whereby when any given segment of an autosomal chromosome has a copy number greater than 2.5 or less than 1.5, said fetus is classified as being at risk of having alterations in the number of at least one chromosome and/or at least one chromosome fragment.

Said preferred embodiment of the method of the invention consists of a method partially implemented by computer. In said preferred embodiment of the method of the invention, steps (d)-(i) are computer implemented steps.

Said preferred embodiment of the method of the invention is a Non-invasive Prenatal Diagnosis (NIPD) test based on bidirectional whole genome massive sequencing techniques.

Second scaling and shifting coefficients are determined from reference samples in a previously performed validation stage of the method for determining the copy numbers of chromosomes and chromosome fragments of the invention. Said coefficients are not determined on every test sample analysis.

In said reference samples, first-scaled standardised residuals and bin copy numbers for each bin have been previously determined and from these previously determined values, second scaling coefficient and shifting coefficients are determined.

The second scaling coefficient is determined from reference samples from fetuses having aneuploidy by calculating the coefficient by which the first-scaled residuals of the aneuploid chromosome (e.g. 21 trisomy) need to be multiplied for obtaining the affected number of copies (i.e. 3 copies in the considered 21 trisomy).

The shifting coefficient is determined from reference samples from fetus having an euploid set of chromosomes. Since residuals are zero-centered, as an example, a shifting coefficient of 2 will transform residuals to 2-centered (euploid).

Male fetus residuals from X chromosome show smaller values (more negative) than residuals from autosomes, due to the fact that GC-models are obtained from bins whose copy number is supposed to be 2 in the fetus. For this reason, shifting of X chromosome of male fetuses needs to be performed with a specific shifting parameter different from the shifting parameter of autosomes. In the algorithm, all standardized residuals of male fetuses regardless of the chromosome they come from, are scaled and shifted, and after this, X chromosome residuals are again shifted for considering this specific shifting parameter.

In an embodiment, the method of the invention rounds off to 3 segment copy numbers greater than 2.5; rounds off to 2 segment copy numbers from 1.5 to 2.5; rounds off to 1 segment copy numbers from 0.5 to 1.5 and rounds off to 0 segment copy numbers less than 0.5.

In an embodiment, the method of the invention determines, from the copy number of a segment, a risk value for each chromosome or part of a chromosome corresponding to said segment.

Some copy numbers are compatible with mosaicism. For example, a copy number of 2.4 is compatible with mosaicism (e.g. a mosaicism with 60% euploid cells and 40% aneuploid cells having a trisomy in chromosome 18).

In said preferred embodiment of the method of the invention, for determining whether a fetus having fetal DNA is at risk of having alterations in the number of at least one chromosome and/or at least one chromosome fragment, it is preferable that the percentage of fetal DNA is at least 3.5% in the maternal plasma sample.

The percentage of fetal DNA in maternal plasma is usually between 2 and 20%. A low percentage of fetal DNA in the maternal plasma sample affects the method's reliability as it hinders the identification of the variations in the number of DNA sequences associated with each one of the chromosomes.

Said preferred embodiment of the method of the invention is based on the comparison of the numbers of sequence reads obtained in sequencing the whole genome per bin of the samples to study with a series of control samples of circulating DNA from maternal plasma of mothers with healthy fetuses.

The samples of circulating DNA with affected foetuses have differences with respect to the control samples. The DNA present in circulating blood largely belongs to the mother, having a reduced concentration (from 2 to 20%) of fetal DNA. As a consequence of this fact, the magnitude of said differences between samples to study and control samples in circulating DNA is especially reduced. The precise numerical methods of the preferred embodiment of the method of the invention increase the reduced difference against the controls, increasing the precision in determining the number of copies of chromosomes and number of chromosome fragments with respect to the numerical methods of the prior art.

A strong relationship has been observed between the guanine and cytosine content (GC content) of the bins and the value of their reads. In said preferred embodiment of the method of the invention, the bias is corrected in the number of reads derived from the GC content and the harmful effects are eliminated in determination of the number of copies caused by said bias.

In an embodiment of the method of the invention, said third bins have a base size of 500 Kb.

In an embodiment of the method of the invention, in step (h)(iv), said regression is a LOESS regression or a polynomial regression.

In an embodiment of the method of the invention, in step (h)(v), said residuals are obtained by subtracting said predicted bin counts of all third bins from said normalised bin counts of all third bins in said test sample and said reference samples.

In an embodiment of the method of the invention, said segmentation is circular binary segmentation (CBS) or segmentation based on a hidden Markov model (HMM).

Said preferred embodiment of the method of the invention detects complete and partial chromosome anomalies from the circulating fetal DNA present in a low concentration in the mother's blood, having the great advantage of being a non-invasive test, without any risk for the fetus or for the mother. Said preferred embodiment of the method of the invention analyses the 24 chromosomes that define the human karyotype.

Said preferred embodiment of the method of the invention is typically performed in the first trimester of pregnancy in pregnant women with the aim of determining, at an early step of her pregnancy, possible whole and partial chromosome anomalies.

Complete chromosomal fetal aneuploidies are associated with fetal syndromes and diseases or to complications such as greater miscarriage frequency. In particular, trisomy 21 is associated with Down syndrome, trisomy 18 is associated with Edwards syndrome, trisomy 13 is associated with Patau syndrome and trisomies 16 and 22 are associated with a greater frequency of miscarriage.

The sexual chromosome aneuploidies are selected from the group consisting of the presence of a single X sexual chromosome, XXY, triple X, XYY and X polysomies.

The presence of a single X sexual chromosome is associated with Turner syndrome and XXY is associated with Klinefelter syndrome.

In an embodiment of the method of the invention, said complete chromosomal fetal aneuploidies are found in a chromosome selected from the group consisting of 9, 13, 15, 16, 18, 21, 22, X sexual chromosome and Y sexual chromosome.

In an embodiment of the method of the invention, said partial fetal aneuploidies are associated with the syndromes selected from the group consisting of Angelman syndrome, Prader-Willi syndrome, Cri du Chat syndrome, Wolf-Hirschhorn syndrome, Jacobsen syndrome, Langer-Giedion syndrome, DiGeorge syndrome, DiGeorge II syndrome and Phelan-McDermid syndrome.

These syndromes are associated with microdeletions in a defined chromosome location.

The chromosome location for the aforementioned syndromes is: 15q11 for Angelman syndrome and Prader-Willi syndrome, 5p for Cri du Chat syndrome, 4p16.3 for Wolf-Hirschhorn syndrome, 11q23 for Jacobsen syndrome, 8q24.1 for Langer-Giedion syndrome, 22q11 for DiGeorge syndrome, 10p14-13 for DiGeorge syndrome II and 22q13.3 for Phelan-McDermid syndrome.

In another preferred embodiment of the method of the invention, said partial fetal aneuploidy consists of a 1p36 deletion or a 16p11.2-p12.1.2 deletion.

In another preferred embodiment of the method of the invention, said value in step (h)(xi) is in a range selected from the group consisting of 0.2-4%, 0.3-3%, 0.4-2%, 0.5-1.5%, and 0.8-1.2%.

The present invention further provides a computer program product comprising a computer-readable medium, wherein instructions are coded to control a computer system comprising means for determining whether a fetus having fetal DNA is at risk of having alterations in the number of at least one chromosome and/or at least one chromosome fragment according to the method of the invention, wherein said alterations in the number of chromosomes are complete chromosomal fetal aneuploidies and said alterations in the number of chromosome fragments are partial fetal aneuploidies, in a maternal plasma test sample containing maternal DNA and said fetal DNA, said computer system comprising the following means:

(a) means for recording DNA sequence reads obtained from said test sample,
(b) means for aligning said sequence reads against a reference genome,
(c) means for determining, with the computer program product for determining the percentage of fetal DNA, the percentage of fetal DNA in said test sample,
(d) means for dividing the sequence of each chromosome into third bins with a base size from 100 Kb to 1 Mb and obtaining bin counts by counting the number of sequence reads in each third bin in said test sample,
(e) means for recovering bin counts in each third bin in second reference samples, wherein said bin counts in said second reference samples have been previously obtained with means (a)-(d) and wherein said second reference samples are maternal plasma reference samples containing maternal DNA and fetal DNA from fetuses with a set of euploid chromosomes,
(f) means for performing the following operations:

(i) means for determining all autosomal third bins as normal bins in said test sample,
(ii) means for obtaining coverage normalisation coefficients in said test sample and said second reference samples by dividing the bin counts in each said normal bins by the mean of the total counts in said test sample and said reference samples for all said normal bins,
(iii) means for obtaining normalised bin counts in said test sample and said second reference samples by multiplying said bin counts of all bins in said test sample and reference samples by said coverage normalisation coefficients,
(iv) means for calculating a GC-content model for said test sample and a GC-content model for each said second reference sample by regression, using the GC-content of said normal bins as the independent variable and using said normalised bin counts as the dependent variable and obtaining predicted bin counts of all bins in said test sample and said second reference samples with said GC-content models,
(v) means for obtaining residuals by calculating differences between the normalised bin counts of all third bins and said predicted bin counts of all third bins in said test sample and said second reference samples,
(vi) means for obtaining standardised residuals of all third bins in said test sample by standardising said residuals of all third bins in said test sample by means of a Z-score obtained for each bin by comparing said residual of each third bin in said test sample to said residual of the corresponding third bin in said second reference samples,
(vii) means for obtaining first-scaled standardised residuals in said test sample by multiplying said standardised residuals in said test sample by a first scaling coefficient, said first scaling coefficient being the inverse of said percentage of fetal DNA determined with means (c),
(viii) means for determining bin copy numbers for each third bin by multiplying said first-scaled standardised residuals by a second scaling coefficient, thereby obtaining second-scaled standardised residuals and by adding a shifting coefficient to said second-scaled standardised residuals, wherein said second scaling coefficient has been previously determined from first-scaled standardised residuals and bin copy numbers for each third bin in third reference samples, wherein said third reference samples are maternal plasma reference samples containing maternal DNA and fetal DNA from fetuses with a set of aneuploid chromosomes, wherein said shifting coefficient has been previously determined from first-scaled standardised residuals and bin copy numbers for each third bin in said second reference samples,
(ix) means for eliminating third bins having a copy number different determining aneuploidy from the group of said normal bins in said test sample,
(x) means for repeating operations (f)(ii) to (f)(ix),
(xi) means for repeating operation (f)(x) if said coverage normalisation coefficients differ more than a value in the range 0.1-5% with respect to previous said coverage normalisation coefficients,

(g) means for obtaining groups of contiguous bins by segmentation, thereby obtaining segments having the same copy number, whereby when any given segment of an autosomal chromosome has a copy number greater than 2.5 or less than 1.5, said fetus is classified as being at risk of having alterations in the number of at least one chromosome and/or at least one chromosome fragment.

In an embodiment of the computer program product of the invention, said third bins have a base size of 500 Kb.

The present invention further provides a method for determining the absence or presence of Y-chromosome in a maternal plasma test sample containing maternal DNA and fetal DNA, said method comprising the following steps:

(a) extracting DNA from said test sample and repairing said DNA,

(b) synthesising libraries of the DNA present in said test sample, amplifying and labelling said libraries by PCR and combining said libraries,

(c) obtaining sequence reads of the Y-chromosome, by massive sequencing, of the amplified, labelled and combined libraries,

(d) aligning said sequence reads of the Y-chromosome against a sequence of the Y-chromosome of a reference genome,

(e) dividing said sequence of the Y-chromosome into fourth bins with a base size from 100 Kb to 1 Mb and obtaining bin counts by counting the number of sequence reads in each fourth bin of said chromosome in said test sample,

(f) recovering bin counts in each fourth bin in second reference samples, wherein said bin counts in said second reference samples have been previously obtained according to steps (a)-(e) and wherein said second reference samples comprise male fetus reference samples and female fetus reference samples,

(g) obtaining normalised bin counts in said test sample and said second reference samples by normalising said bin counts of all bins based on the mean of the total counts of said test sample and said second reference samples for all normal bins in said test sample, wherein said normal bins are said normal bins determined in step (h)(xi) of the method for determining whether a fetus having fetal DNA is at risk of having alterations in the number of at least one chromosome and/or at least one chromosome fragment of the invention,

(h) determining bins of said sequence of the Y-chromosome that are statistically different by a first Wilcoxon statistical hypothesis test comparing said normalised bin counts in said female fetus reference samples to normalised bin counts in said male fetus reference samples, wherein the null hypothesis of said first Wilcoxon test is that the mean of said normalised bin counts in each fourth bin in said male fetus reference samples is significantly greater than the mean of said normalised bin counts in each fourth bin in said female fetus reference samples and wherein bins are determined statistically different when said null hypothesis of said first Wilcoxon test is verified as true,

(i) for said bins of said sequence of the Y-chromosome that are statistically different, comparing said normalised bin counts in said test sample to said normalised counts in said female fetus reference samples by a second Wilcoxon test, wherein the null hypothesis of said second Wilcoxon test is that the mean of said normalised bin counts in each said bin that is statistically different in said test sample is significantly greater than the mean of said normalised bin counts in each said bin that is statistically different in said female fetus reference samples,

(j) for said bins of said sequence of the Y-chromosome that are statistically different, comparing said normalised bin counts in said test sample to said normalised counts in said male fetus reference samples by a third Wilcoxon test, wherein the null hypothesis of said third Wilcoxon test is that the mean of said normalised bin counts in each said bin that is statistically different in said test sample is significantly less than the mean of said normalised bin counts in each said bin that is statistically different in said male fetus reference samples,

(k) determining male bins when both said null hypotheses of said second and said third Wilcoxon tests are verified as true and determining female bins when at least one of said null hypotheses of said second and said third Wilcoxon tests are not verified as true,

(l) determining the number of said male and female bins and determining the presence of the Y-chromosome wherein the number of said male bins is higher than the number of said female bins and determined the absence of the Y-chromosome otherwise, wherein said presence or absence of the Y chromosome is indicative of the fetal sex.

In an embodiment of the method of the invention, said fourth bins have a base size of 500 Kb.

The present invention further provides a computer program product comprising a computer-readable medium, wherein instructions are coded to control a computer system comprising means for determining the absence or presence of Y-chromosome in a maternal plasma test sample containing maternal DNA and fetal DNA according to the method of the invention, said computer system comprising the following means:

(a) means for recording DNA sequence reads obtained from said test sample,

(b) means for aligning said sequence reads of the Y chromosome against a sequence of the Y-chromosome of a reference genome,

(c) means for dividing said sequence of the Y-chromosome into fourth bins with a base size from 100 Kb to 1 Mb and obtaining bin counts by counting the number of sequence reads in each fourth bin of said chromosome in said test sample,

(d) means for recovering bin counts in each fourth bin in second reference samples, wherein said bin counts in said second reference samples have been previously obtained and wherein said second reference samples comprise male fetus reference samples and female fetus reference samples,

(e) means for obtaining normalised bin counts in said test sample and said second reference samples by normalising said bin counts of all bins based on the mean of the total counts of said test sample and said second reference samples for all normal bins in said test sample, wherein said normal bins are said normal bins determined with operation (f)(xi) of the computer program product for determining whether a fetus having fetal DNA is at risk of having alterations in the number of at least one chromosome and/or at least one chromosome fragment according of the invention,

(f) means for determining bins of said sequence of the Y-chromosome that are statistically different by a first Wilcoxon statistical hypothesis test comparing said normalised bin counts in said female fetus reference samples to normalised bin counts in said male fetus reference samples, wherein the null hypothesis of said first Wilcoxon test is that the mean of said normalised bin counts in each fourth bin in said male fetus reference samples is significantly greater than the mean of said normalised bin counts in each fourth bin in said female fetus reference samples and wherein bins are determined statistically different when said null hypothesis of said first Wilcoxon test is verified as true,

(g) means for comparing said normalised bin counts in said test sample to said normalised counts in said female fetus reference samples by a second Wilcoxon test for said bins of said sequence of the Y-chromosome that are statistically different, wherein the null hypothesis of said second Wilcoxon test is that the mean of said normalised bin counts in each said bin that is statistically different in said test sample is significantly greater than the mean of said normalised bin counts in each said bin that is statistically different in said female fetus reference samples,

(h) means for comparing said normalised bin counts in said test sample to said normalised counts in said male fetus reference samples by a third Wilcoxon test for said bins of said sequence of the Y-chromosome that are statistically different, wherein the null hypothesis of said third Wilcoxon test is that the mean of said normalised bin counts in each said bin that is statistically different in said test sample is significantly less than the mean of said normalised bin counts in each said bin that is statistically different in said male fetus reference samples,

(i) means for determining male bins when both said null hypotheses of said second and said third Wilcoxon tests are verified as true and determining female bins when at least one of said null hypotheses of said second and said third Wilcoxon tests are not verified as true,

(j) means for determining the number of said male and female bins and determining the presence of the Y-chromosome wherein the number of said male bins is higher than the number of said female bins and determined the absence of the Y-chromosome otherwise, wherein said presence or absence of the Y chromosome is indicative of the fetal sex.

In an embodiment of the computer program product of the invention, said fourth bins have a base size of 500 Kb.

In the present invention a computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may include, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and kits according to embodiments and/or steps of the invention. It will be understood that each square or diamond-shaped block of the flowchart illustrations and/or block diagrams, and combinations of blocks in the flowchart illustrations and/or block diagrams, can be implemented by biological techniques or computer readable program instructions, or combinations thereof.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

**Massive sequencing**

It is possible to use any suitable sequencing method to perform the methods described in this document. In some embodiments, a high-throughput sequencing method is used. In some high-throughput sequencing methods, the DNA sequences are generally massively parallel sequenced within a flow cell. In some sequencing platforms, the lane is the basic physical unit in a massive sequencing platform, and it relates to the basic independent execution in said massive sequencing platform. High-throughput sequencing technologies include sequencing by synthesis.

The systems used for high-throughput sequencing methods are commercially available and include, for example, the MiniSeq®, MiSeq®, NextSeq® 550, HiSeq® 2000, HiSeq® 2500, HiSeq® 3000, HiSeq® 4000 and NovaSeq® platforms from Illumina, Inc and the ION Proton and ION PGM sequencing platforms from ThermoFisher, among others.

In some embodiments, paired-end sequencing is used. Paired-end sequencing is based on the sequencing of both ends of a fragment and generates high quality sequence data, facilitating the detection of genomic reorganization and repetitive sequence elements, and novel gene fusions and transcriptions.

In sequencing by synthesis, millions of fragments of nucleic acid (e.g. DNA) can be parallel sequenced. In one example of this type of sequencing technology, a flow cell is used containing an optically transparent slide with 2 individual lanes on the surfaces of which oligonucleotides are bound (e.g. adapter oligonucleotides). A flow cell is often a solid support which can be configured to retain and/or allow the organized passing of the reagent solutions on the bound analytes. Flow cells frequently have a flat shape, optically transparent, generally in millimetric or submillimetric scale, and they often have channels or lanes wherein the analyte/reagent interaction occurs. In certain sequencing by synthesis procedures, for example, the template DNA is fragmented in lengths of several hundred base pairs in preparation for generation of the library. In certain embodiments, the isolation of the sample and the generation of the library is performed using automated methods and apparatus.

In certain sequencing by synthesis procedures, the adapter oligonucleotides are complementary to oligonucleotides present in the flow cells, and sometimes they are used to associate the DNA with a solid support, the internal surface of a flow cell, for example. In some embodiments, the adapter oligonucleotide includes indexing oligonucleotides (indexes) (e.g. a single series of nucleotides that can be used as indexes to allow the unequivocal identification of a sample), one or more hybridization sites of sequencing primers (e.g. universal sequencing primers, single end sequencing primers, paired-end sequencing primers, multiplexed sequence primers, and similar), or combinations thereof (for example, adapter/sequencing, adapter/index, adapter/index/sequencing). The indexing primers often have six or more base-length index tag. In certain embodiments, the indexes are associated with a sample, but they are sequenced in a separate sequencing reaction to avoid compromising the quality of the sequence reads. Subsequently, the reads of the index sequence and the sample sequence are interlinked, and the reads are demultiplexed.

In certain sequencing by synthesis procedures, the use of adapter oligonucleotides allows multiplexing of sequence reactions in a flow cell lane, which allows the analysis of multiple samples per lane of the flow cell. The number of samples that can be analysed in a given flow cell lane often depends on the number of single indexes used during preparation of the library. The number of indexes used is not limited in the methods described in this document and said methods can be performed using any number of indexes (e.g. 4, 8, 12, 20, 24, 48 or 96). The greater the number of indexes, the greater the number of samples that can be multiplexed in a single flow cell lane. In some sequencing platforms, multiplexing using 12 indexes I7 and 8 indexes I5 allows analysing 96 samples (e.g. equal to the quantity of wells in a 96-well plate) in a 2-lane flow cell.

In certain sequencing by synthesis procedures, template DNA modified with adapters is added to the flow cells and it is immobilized by means of hybridization with the anchoring oligonucleotides present in the flow cell in limiting dilution conditions. Multiple amplification cycles convert the DNA template of a single molecule into a clonally amplified cluster. The groups are denatured for the sequencing. The sequencing starts by hybridizing a primer complementary to the adapter sequences, followed by the polymerase addition and a mixture of four reversible fluorescent probe terminators of different colours. The terminators are incorporated according to the complementary nature of sequence in each chain in a clonal cluster. After the incorporation, the excess reagents are eliminated by washing, the groups are optically analysed, and the fluorescence signal is registered. With successive chemical steps, the reversible probe terminators are unblocked, the fluorescent labels are split and eliminated by washing, and the sequencing cycle is performed.

### Definitions

In the present specification, the technical term "massive sequencing" is synonymous and is indistinctly used with respect to the term "Next Generation Sequencing (NGS)".

In the present specification, the terms "fraction of fetal DNA" and "percentage of fetal DNA" are related by a factor of 100. The quantity of fetal DNA with respect to the total DNA (maternal DNA + fetal DNA) in a maternal plasma sample can be expressed as a "fraction of fetal DNA" or as a "percentage of fetal DNA".

In the present specification, "nucleosome profile" relates to a distribution of the number of sequence reads of one or more bins, wherein each chromosome has been divided, obtained by massive sequencing of a maternal plasma sample containing maternal DNA and fetal DNA against the insert size between each pair of reads. "Nucleosomal" relates to the fact that the size of the fetal DNA is statistically smaller than the size of the maternal DNA and this difference is caused by the different nucleosome profiles that the fetus and mother have. This is due to the sizes of the maternal DNA being approximately equal to the sizes corresponding to the nucleosome with a bound link DNA fragment, whilst the fetal DNA is biased towards smaller sizes than the maternal DNA and of equal or smaller size than the nucleosome.

In the present specification, "genomic regions enriched in fetal DNA" relates to the fact that the percentage of fetal DNA in a maternal plasma sample containing maternal DNA and fetal DNA mapping to said genomic regions is greater with respect to the average percentage of fetal DNA of said sample in all the genomic regions. Said genomic regions enriched in fetal DNA are identified when a model is obtained with a correlative concordance of at least 40% using a linear regression with the variables: gestational weeks, mother's height, weight and heparin intake as co-variables.

In the present specification, "sliding bin" relates to the fact that the calculation of the number of reads is performed in a bin with a base size (e.g. between 50 and 200 Kb, preferably 100 Kb) and said bin slides along the chromosome a number of bases equal to the size of the sliding bin (e.g. between 5 and 15 Kb, preferably 10 Kb), in this way generating overlapping bins.

In the present specification, "repairing the DNA" relates to the conversion of the cohesive ends of the DNA in blunt ends, a process wherein polymerase enzymes intervene.

In the present specification, "partial fetal aneuploidies" relate to a partial gain or loss of a variable size in a fetal chromosome in comparison with a reference genome.

In the present specification, "complete chromosomal fetal aneuploidies" relate to variations in the number of fetal chromosomes. The term aneuploidy makes reference to the change in the number of chromosomes. Said change in the number of chromosomes may give rise to genetic diseases. An aneuploid is a subject whose number of chromosomes differs from the wild type or euploid in part of its set of chromosomes, due to an extra or absent chromosome. Generally, the aneuploid set of chromosomes only differs from the wild type in one or a few chromosomes. Monosomies and trisomies are the most common aneuploidies.

In the present specification, "insert size" relates to the distance in nucleotides between the paired-end reads obtained from the sequencing. It is an estimator of the size of the sequenced DNA.

In the present specification, "Spearman correlation coefficient" relates to a measurement of the correlation (the association or interdependence) between two random variables (both continuous and discrete).

In the present specification, "chrY method" relates to a calculation method of the percentage of fetal DNA from the proportion of sequences of the Y chromosome, as described in Hudecova I et al. 2014. In women pregnant with a male fetus, the percentage of fetal DNA of the mother in a maternal plasma sample can be determined from the proportion of reads mapping to the Y chromosome (% chrY).

In the present specification, "residuals" relates to differences between the number of sequence reads of a bin and the number of reads of said bin obtained with a prediction of a guanine and cytosine content model.

In the present specification, "electropherogram" relates to a distribution of sizes of DNA fragments.

In the present specification "Indexes I7 and I5" relate to nucleotides sequences included in the amplification oligonucleotides that allow the unequivocal identification of a sample when several samples are sequenced at the same time.

In the present specification, "Random Forest" relates to a combination of predictor trees such that each tree depends on the values of an independently proven, random vector and with the same distribution for each one of these. It builds a long collection of uncorrelated trees and then averages them.

In the present specification, *"IncNodePurity"* relates to a method associated with a Random Forest model that evaluates the impact that elimination of a specific variable has on the estimation of the model using the quadratic sum of the residuals. The greater the value, the greater the impact it will have and the more important the predictor variable is.

In the present specification, "ARMA filter" relates to an *AutoRegressive Moving Average model* (abbreviated as ARMA) which is applied to temporary series of data. Given a temporary series of data, the ARMA model is a tool to understand and predict future values of the series. The model is formed by two parts, one autoregressive (AR) and another of moving average (MA).

In the present specification, "Loess model" relates to a locally weighted polynomial regression method. At each point of the range of the set of data, a low-grade polynomial is adjusted to a subset of the data, with variable explicative values close to the point whose response is estimated. The polynomial is adjusted using minimum weighted squares, which gives more weight to the points close to the point whose response is being estimated and less weight to the more distant points. The value of the regression function for the point is then obtained evaluating the local polynomial using the values of the explicative variable for that data point.

In the present specification, "scatterplot smoothing method" relates to a scatterplot smoothing method that adjusts a function through the points of a scatter plot to represent the ratio between the variables. The scatter plots can be smoothed by adjusting a line to the data points in a diagram. This line tries to show the non-random component of the association between the variables in a 2D scatter plot. The smoothing process tries to separate the non-random behaviour in the data from random fluctuations, eliminating or reducing these fluctuations, and it allows the prediction of the value based on the explicative variable response. The smoothing process is normally performed by means of the use of a straight line (simple linear regression), a quadratic or polynomial curve or local regression.

In the present specification, "Z-score" relates to a standard score number, which is the number of standard deviations for which the value of an observation or data point is above the average value of what is observed or measured. The values observed above the average have positive standard scores, whilst the values below the average have negative standard scores. The standard score is an adimensional quantity obtained by subtracting the populational average of an individual gross score and then dividing the differences between the standard deviation of the population. This conversion process is called standardization.

In the present invention, "Wilcoxon test" relates to a non-parametric test to compare the average range of two related samples and determine if there are differences between them. It is used as an alternative to the Student's t-test when the normality of said samples can be assumed. It is a non-parametric test to compare two related samples and therefore does not need a specific distribution. It is used to compare two related measurements and determine whether the difference between them is due to randomness or not (in this last case, that the difference is statistically significant). It is used when the underlying variable is continuous, but any type of particular distribution is not presumed.

## BRIEF DESCRIPTION OF THE DRAWINGS

Figure 1. Electropherogram showing the analysis of the sizes of the DNA fragments by means of analysis with the Agilent 2100 Bioanalyzer apparatus, using the Agilent High Sensitivity DNA assay kit.

Figure 2. Distribution of percentage of free circulating fetal DNA in accordance with the gestational week determined using the chrY method in maternal plasma control samples with male fetuses.

Figure 3. Flow diagram of the bioinformatics analysis for determining the percentage of fetal DNA by nucleosome profiling according to the invention.

Figure 4. Number of significant genomic regions enriched in fetal DNA per chromosome identified according to the invention. In its identification, the reference genome hg19 from the GRC consortium (Genome Reference Consortium Human Build 37 (GRCh37) was used, https://www.ncbi.nlm.nih.gov/assembly/GCF_000001405.13/).

Figure 5. Distribution of the DNA fragments size in base pairs (bp) mapping to a genomic region enriched in fetal DNA (curve 1) and the total of the sample (fetal + maternal, curve 2). Sample with 9% of fetal DNA.

Figure 6. Distribution of the DNA fragments size in base pairs (bp) mapping to a genomic region enriched in fetal DNA (curve 1) and the total of the sample (fetal + maternal, curve 2). Sample with < 3% of fetal DNA.

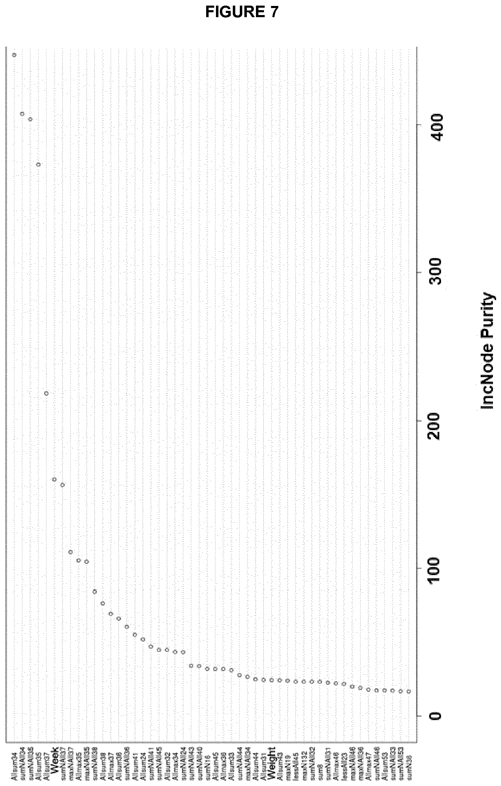

Figure 7. Weight of the 50 most important dependent variables using the IncNodePurity algorithm within a Random Forest model to establish predictive variables wherein more than 1000 possible dependent variables are used. Some are variables that describe the peaks observed in the DNA size distribution. The variables containing the term "sum" relate to a sum, the variables containing the term "max" relate to a maximum value, the variables containing the expression "less" relate to an accumulative value, the variables containing the expression "All" relate to the overall section with all the genomic regions and the variables that do not contain the expression "All" relate to the fetal section in genomic regions rich in fetal DNA. The number relates to the insert size in Kb. The Allsum54 variable relates to the sum of the peak area at 54 Kb in the total sample. The Max80 variable relates to the maximum value of the peak at 80 Kb in the enriched area.

Figure 8. Distribution of the difference between the percentage of fetal DNA predicted by a Random Forest model according to the invention and the percentage of fetal DNA determined by digital PCR and by the chrY method (Y-axis) represented against the gestational week (X-axis).

Figure 9. Flow diagram of the bioinformatics analysis for determining the number of copies of chromosomes and the number of copies of chromosome fragments according to the invention.

Figure 10. Graph of sequence read proportions with respect to the total of reads, both for the sample, and for the control, obtained in an analysis for determining the number of copies of chromosomes according to the invention.

Figure 11. Copy numbers per chromosome for a test sample subjected to analysis for determining the number of chromosomes according to the invention. The figure shows groups of contiguous bins and segments, represented as black or grey lines, obtained by segmentation. Segments for said groups of contiguous bins have the same copy number.

Figure 12. Graph of copy numbers in the chromosome region 22q11.21-22q12.1 of chromosome 22 for a test sample. This region is relevant for determining alterations in the number of chromosome fragments that would indicate that the fetus may suffer DiGeorge syndrome. The figure shows groups of contiguous bins and segments, represented as black or grey lines, obtained by segmentation. Segments for said groups of contiguous bins have the same copy number.

Figure 13. Flow diagram of the bioinformatics analysis for determining the absence or presence of the Y chromosome according to the invention.

Figure 14. Normalized number of reads of Y chromosome in male controls (curve 1) and female controls (curve 2).

**DESCRIPTION OF EMBODIMENTS**

**Example 1. Isolation, aliquoting and freezing of the plasma**

The blood from the mother was kept at ambient temperature (6-35 °C) before its processing. Each sample was processed in individualized manner in a different rack. No more than two samples were processed simultaneously.
All the processing steps, with the exception of the plasma centrifugations, were performed in a plasma culture chamber.
The process described below was started within 96 hours of the collection of the maternal blood sample. The maternal blood tubes were centrifuged for 15 minutes at 1600 g, at 4 °C. The plasma fraction was collected with the sterile 3 ml pipette, leaving approximately 5-8 mm of plasma before the white layer of leukocytes. The plasma was distributed in sterile 2 ml Eppendorf tubes and they were placed in ice. The Eppendorf tubes were centrifuged for 10 minutes at 16000 g, at 4 °C. The content of each Eppendorf tube was collected without rushing so as to not take the sediment, leaving approximately 50 µl of plasma and it was poured into other sterile 1.5 ml Eppendorf tubes. These tubes contain cell-free plasma. The new tubes were placed in ice and later frozen at -80 °C.

**Example 2. Extraction of fetal DNA from plasma**

The extraction process was performed in the laminar flow hood, which was previously cleaned with 70% ethanol and RNase away®. The material to be used (not the reagents) was exposed to UV light for approximately 5-10 minutes per side before starting the process.

The tubes obtained in Example 1 were incubated for 30 minutes at 60 °C (in bath). 7.2 ml of ACB buffer (guanidine thiocyanate and trometamol) were added to each tube. The tubes were incubated in ice for 5 minutes. The lysate was decanted in the extension tube of a QIAvac 24 plus® vacuum system, which contained a closed Vac-valve, a Vac-connector, an extraction column and a 20 ml extension tube in the open column. The vacuum pump was switched on, with the key closed. Vacuum was achieved (-800 to -900 mbar) and all the lysate passed through the column. The extension tube was eliminated. 600 µl of ACW1 buffer (guanidium chloride) was passed through the column. 750 µl of ACW2 buffer (chaotropic agent and salts) were passed through the column. 750 µl of ethanol were passed through the column. The column was placed in a 2 ml collector tube and it was centrifuged for 3 minutes at 14000 rpm (20000g). The column was placed in a new collector tube. It was incubated for 10 minutes at 56 °C in block, after cleaning with 70% ethanol and RNase away®, within the hood. After 10 minutes, the column was placed in a 1.5 ml elution tube. 50 µl of AVE elution buffer (ribonuclease-free water and 0.04% sodium azide) were added, in the bottom of the column, but without touching the filter with the tip of the pipette. It was incubated for 3 minutes at ambient temperature. It was centrifuged for 1 minute at 14000 rpm (20000g).

**Example 3. DNA repair**

DNA was resuspended in 10 mM Tris, 0.1 mM EDTA, pH 8.0 solution. The free circulating plasma DNA (5 ng in 10 µL) was dispensed in each PCR tube. The necessary volume of the reaction mixture to repair the DNA was prepared in ice according to the number of samples that are going to be processed, as described in Table 1.

Table 1. Reaction mixture to repair the DNA

| Reagents | Volume/reaction |
|---|---|
| GeneSGKit® Template Preparation Buffer | 2 µL |
| GeneSGKit® Template Preparation Enzyme | 1 µL |

To repair the DNA, 3 µl of the reaction mixture were added. It was carefully mixed. The tubes were placed in the thermocycler and the program of Table 2 was executed.

Table 2

| Temperature | Time |
|---|---|
| 22 °C | 25 min |
| 55 °C | 20 min |
| 4 °C | Hold maximum ≤ 2 h |

**Example 4. Library synthesis**

The necessary volume of library synthesis reaction mixture was prepared in ice according to the number of samples that are going to be processed, as described in Table 3. It was carefully mixed.

Table 3. Library synthesis reaction mixture

| Reagents | Volume/reaction |
|---|---|
| GeneSGKit® Library Synthesis Buffer | 1 µL |
| GeneSGKit® Library Synthesis Enzyme | 1 µL |

2 µL of the library synthesis reaction mixture were added to the product of the previous DNA repair reaction of Example 3. It was carefully mixed. The tubes were placed in the thermocycler and the program of Table 4 was executed.

Table 4

| Temperature | Time |
|---|---|
| 22 °C | 40 min |
| 4 °C | Hold for ≤ 30 min |

## Example 5. Amplification of the library

Indexing reagents were used which allow multiplexing multiple samples. The double indexes have 8 nucleotides in length. A combination of different indexes was used for each sample sequenced in the same lane. The sequences of the indexes are shown in Table 5.

Table 5

| Sequence of the double indexes | | | |
|---|---|---|---|
| Index I7 | Sequence | Index I5 | Sequence |
| D701 | ATTACTCG | D501 | TATAGCCT |
| D702 | TCCGGAGA | D502 | AT AGAGGC |
| D703 | CGCTCATT | D503 | CCTATCCT |
| D704 | GAGATTCC | D504 | GGCTCTGA |
| D705 | ATTCAGAA | D505 | AGGCGAAG |
| D706 | GAATTCGT | D506 | TAATCTTA |
| D707 | CTGAAGCT | D507 | CAGGACGT |
| D708 | TAATGCGC | D508 | GTACTGAC |
| D709 | CGGCTATG | | |
| D710 | TCCGCGAA | | |
| D711 | TCTCGCGC | | |
| D712 | AGCGATAG | | |

The necessary volume of library amplification reaction mixture was prepared in ice according to the number of samples that are going to be processed, as described in Table 6. It was carefully mixed.

Table 6. Library amplification reaction mixture

| Reagent | Volume/reaction |
|---|---|
| GeneSGKit® Library Amplification Buffer | 25.0 μL |
| GeneSGKit® Library Amplification Enzyme | 1.0 μL |
| Nuclease-free water | 4 μL |

30 μL of the reaction mixture for PCR amplification were added to each reaction. 5 μL of the selected indexes were added to each library. The reaction mixture was carefully mixed. The tubes were placed in the thermocycler and the program of Table 7 was executed.

Table 7

| Process | Step | Temperature | Time | No. cycles |
|---|---|---|---|---|
| Extension and cleavage | 1 | 72 °C | 3 min | 1 |
| | 2 | 85 °C | 2 min | 1 |

(continued)

| Process | Step | Temperature | Time | No. cycles |
|---|---|---|---|---|
| Denaturing | 3 | 98 °C | 2 min | 1 |
| Addition of indexes | 4 | 98 °C | 20 s | 4 |
| | | 67 °C | 20 s | |
| | | 72 °C | 40 s | |
| Amplification | 5 | 98 °C | 20 s | 7 |
| | | 72 °C | 50 s | |
| | 6 | 4 °C | Hold | 1 |

**Example 6. Purification of the library by means of AMPure XP beads@**

Ampure XP beads@ (Beckman Coulter) were maintained at ambient temperature for 30 minutes. 50 $\mu$L of library were mixed in a 1.5 mL tube, with 50 $\mu$L of nuclease-free water to obtain a final volume of 100 $\mu$L. 70 $\mu$l of Ampure XP beads@ solution were added to each tube containing the diluted library. They were mixed and incubated for 5 minutes at ambient temperature. Tubes were placed in a magnetic holder for 5 minutes. The supernatant was transferred to a new 1.5 mL tube. 187 $\mu$l of Ampure XP beads@ solution were added to each tube containing the sample. The soluction was mixed and incubated for 5 minutes at ambient temperature. The tubes were placed in the magnetic holder for 5 minutes. The tubes were kept in the magnetic holder whilst the supernatant was removed with a pipette, avoiding touching the beads. With the tubes in the magnetic holder, they were washed twice adding 200 $\mu$l of 80% ethanol to each tube. The tubes were left in the magnetic holder for 1 minute and the ethanol was removed with a pipette. The tubes were maintained in the magnetic holder and they were left to dry with the lid open at ambient temperature until all the residual ethanol was evaporated. 20 $\mu$l of 1 x Tris-EDTA (10 mM Tris-HCI, 1 mM EDTA, pH 8.0) buffer were added to each tube. They were mixed and incubated at ambient temperature for 2 minutes. The tubes were placed in the magnetic holder for 2 minutes, until the solution did not contain Ampure XP beads@. The supernatant was transferred ($\sim$19 $\mu$l) to a new 1.5 mL tube and the Ampure XP beads@ were removed.

**Example 7. Validation of the library**

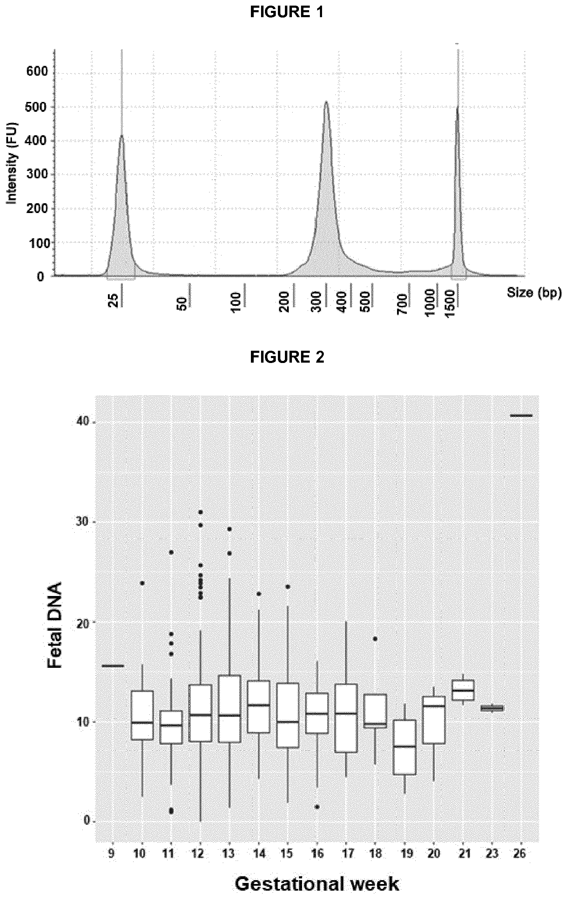

The obtained library quality was determined by analysing one aliquot in an Agilent 2100 Bioanalyzer Platform[®] (Agilent) apparatus, using the Agilent High Sensitivity DNA assay[®] kit (Agilent), following Agilent's instructions and protocols to perform this analysis. 1 $\mu$l or 2 $\mu$l of the library were used for the analysis. Figure 1 shows the electropherogram obtained, which shows a DNA peak of -300 bp (base pairs).

The libraries were quantified using the fluorescence method of the Qubit dsDNA HS Assay[®] kit, following the manufacturer's instructions.

**Example 8. Combination of libraries for sequencing**

The number of libraries to combine for the sequencing was determined. The concentration of each library was calculated. The quantity was determined of each one of the libraries that was used in the mixture using the following formula:

$$\text{Library volume} = (Vf \times Cf)/(nl \times Ci)$$

wherein

Vf is the final volume of the combination

Cf is the final concentration of library in the combination (4 nM)

nl is the number of libraries

Ci is the initial concentration of each library (nM)

The libraries were combined using the volume determined with the previous formula. The final volume of the library combination was adjusted to the desired concentration with 10 mM Tris-CI, pH 8.5, Tween 20 (0.1% v/v). If the final volume of the pool on combining the libraries is greater than the desired volume (Vf), it was lyophilized and resuspended in the desired volume.

**Example 9. Sequencing of the libraries by massive sequencing**

Pools of libraries with concentrations 1.5 pM and 10 pM were sequenced. Library sequencing was performed by massive sequencing in the MiSeq®, NextSeq® 550 and HiSeq® 2500 Illumina platforms.

**Example 10. Determination of the percentage of fetal DNA by nucleosome profiling**

The percentage of fetal DNA was determined in 600 control samples of plasma from women pregnant with a male and female fetus. The percentage of fetal DNA for male fetuses was determined by the chrY method and for female fetuses by Digital PCR. The 600 samples had a broad spectrum of different biological variables of interest, among them, the more important is the "gestational weeks" variable. The method is based on the fact that according to the gestational week the quantity of fetal DNA and its genetic origin may change and adapt. The distribution of the percentage of fetal DNA according to the different gestational weeks is shown in Figure 2.

**Bioinformatic analysis**

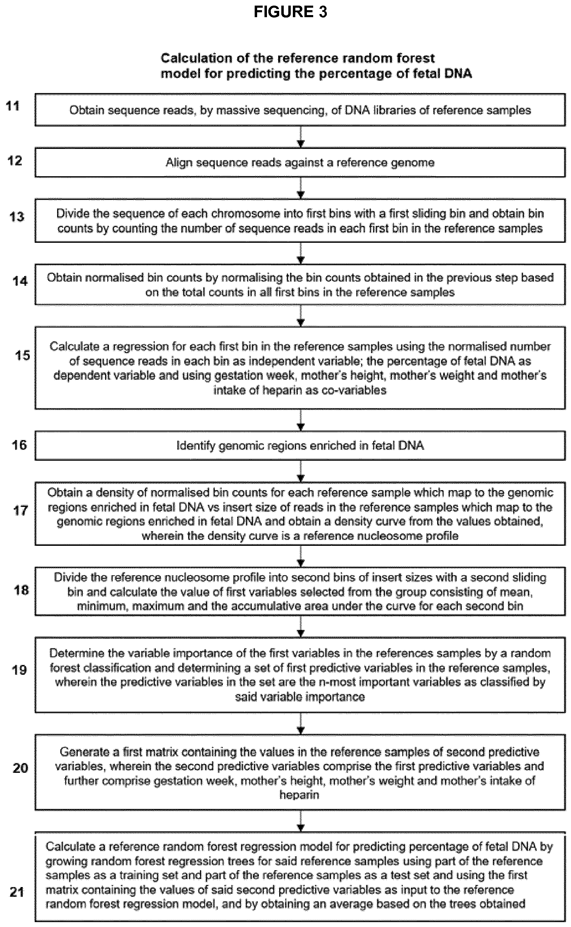

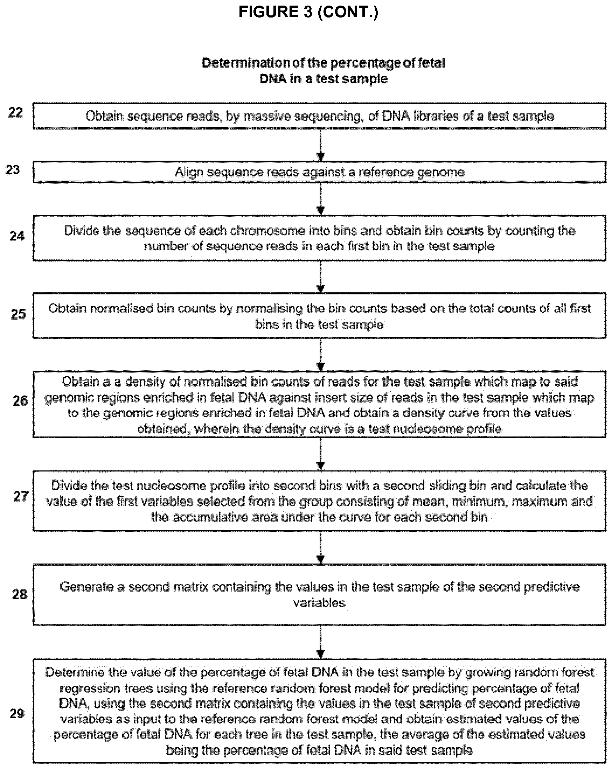

Figure 3 shows the flow diagram of the bioinformatics analysis of the method of the invention for determining fetal DNA by nucleosome profiling and shows steps performed in the bioinformatics analysis.
Alignment against the reference genome were performed with the BWA-MEM algorithm.
Division of the chromosome into bins were performed with the bedtools tool. The number of sequence reads (counts) was obtained by counting (adding up) the reads mapping to each bin.
In normalisation steps, for each region, the geometric mean was added using all the samples. Later, using those values, the median was calculated for each sample, providing a scaling factor per sample. Later, the initial values were divided by this scaling factor. Table 8 describes the substeps of the algorithm used in these steps:

Table 8

| Calculation of the geometric average by rows (chromosome regions) |
|---|
| Division of the initial values by this factor |
| Calculation of the median by columns (samples) and obtaining the normalization values by samples |
| Division of the initial matrix by the normalization values |

In the regression step, the mathematical formula used is a linear regression. Table 9 describes substeps of the algorithm used in this step, for each bin of the genome defined (called i).

Table 9

| Generation of a matrix with the co-variables and the number of reads (coverage) in a genomic region "i" |
|---|
| Linear regression with the percentage of fetal DNA as dependent variable |
| Generation of a matrix with the real and predicted values |
| Calculation of the correlation between both values |

In the step of obtaining nucleosome profiles, a density function was obtained with the normalized values and using the whole genome. Table 10 describes the substeps of the algorithm used in this step. The input data is a bam alignment file for each sample.

Table 10

| Bam file read |
|---|

(continued)

| |
| --- |
| Elimination of Y chromosome reads and mitochondrial DNA reads |
| Elimination of the pairs with insert size less than 60 bp |
| Elimination of the pairs with insert size greater than 250 bp |
| Calculation of the density function |

The density matrix was itemized in prevalence estimators (sum, average, minimum, etc) by sliding bins at different insert sizes. Table 11 describe the substeps of the algorithm used in this step, for each iteration t (t makes references to an insert or peak size value).

Table 11

| |
| --- |
| Assignment of a first value of 60 for t (minimum insert size of the algorithm) |
| Calculation of the maximum peak value |
| Calculation of the sum for that peak |
| Calculation of the average of the maximum value |
| Calculation of the average of the sum |
| Calculation of the area (sum) of the lower peaks up to the reached point |
| Another different bin of size 5 bp is defined |
| Calculation of the difference between the initial bin and the new bin (each bin is compared with all the bins of the same type of the density curve to measure dependencies) |
| Execution for the whole genome and for the genomic regions enriched in fetal DNA, thus obtaining two matrices with the same variables for the whole genome and for the genomic regions enriched in fetal DNA |
| Joining the two matrices in a single matrix x with an indicator that allows their separation |
| Calculation of the random Forest predictor |
| Calculation of the value of importance of each variable and generation of 10000 trees |

Step 21 started from the matrix x obtained in the previous step. An estimator using all the trees was performed in the prediction method.

Alignment of control sample sequences against the reference genome

Sequence reads of the control samples were obtained by massive sequencing. The sequence reads of the control samples were aligned against a reference genome.
The reference genome hg19 from the GRC consortium (Genome Reference Consortium Human Build 37 (GRCh37) was used,
https://www.ncbi.nlm.nih.gov/assembly/GCF_000001405.13/)

Division of the chromosome into bins and obtaining number of sequence reads of each bin from control samples

The human genome was divided into 100 Kb bins with a 10 Kb sliding bin and into bins having a base size of 50 Kb with a sliding bin of 5 Kb. The number of sequence reads of each bin was calculated for each sample.

Normalization of the number of sequence reads of each bin based on the total number of sequence reads

The number of sequence reads of each bin was normalized using the DESeq2 algorithm (Love et al. 2014).

Linear regression

A linear regression was performed in each bin using the number of sequence reads of each bin as independent variable, the percentage of fetal DNA as dependent variable and gestational week, mother's height and mother's weight as variants.

Obtaining genomic regions enriched in fetal DNA

Genomic regions enriched in fetal DNA were identified, correlated with the percentage of fetal DNA obtained using the chrY or Digital PCR methods (normalized by the variables: gestational weeks, height and weight). To do this, the genomic regions enriched in fetal DNA were identified when the linear regression was considered as statistically significant when the significance of the linear regression model was greater than $10^{-10}$ and the later correlation between the predicted and real values was greater than 50% (Spearman >= 0.50).

Said enriched genomic regions were identified as reproducible and enriched in fetal DNA considering the gestational weeks as a normalizing process.

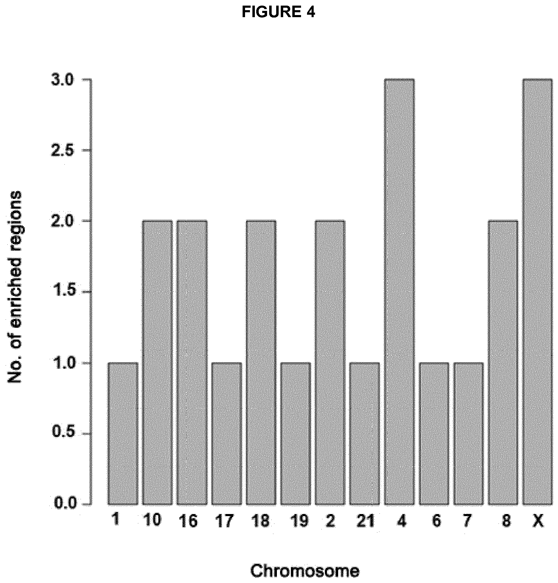

To avoid imbalances, the regions obtained for the X chromosome were eliminated. Female fetuses have two X chromosomes instead of one and may, therefore, have greater coverage. The elimination of regions obtained for the X chromosome aims to eliminate all possible bias for the fetal sex and aims to focus the determination of genomic regions enriched in fetal DNA in autosomal regions whose genomic load is the same in both sexes. Figure 4 shows the number of selected enriched regions per chromosome.

In the obtained genomic regions enriched in fetal DNA, there is a high proportion of fetal DNA based on its correlation with objective data associated with the fetus, such as the chrY method.

Obtaining the nucleosome profile in regions enriched in fetal DNA

The size of the fetal DNA was determined from the size of the paired-end sequence reads from the massive sequencing.

The distributions of the number of sequence reads of each bin mapping to the genomic regions enriched in fetal DNA were determined against the size of the fragments corresponding to each read obtained for the samples, thus determining the nucleosome profile in genomic regions enriched in fetal DNA.

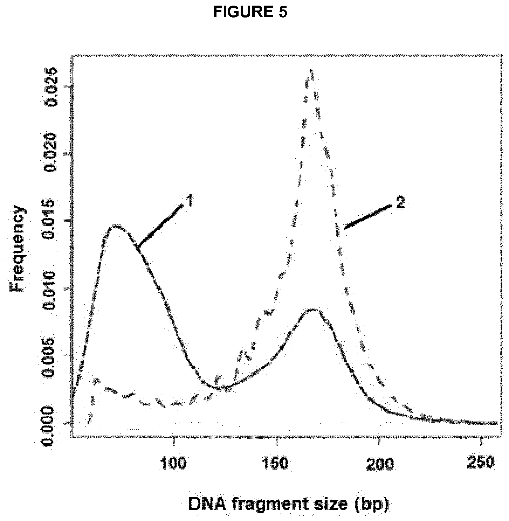

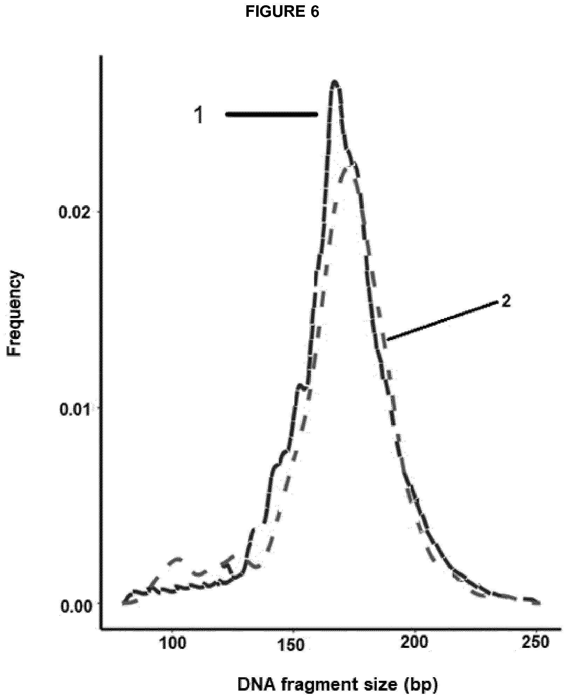

The nucleosome profile in genomic regions enriched in fetal DNA was different to the overall nucleosome profile in all the regions (which indicated the overall profile of fetal and maternal DNA) and the insert size or fragment had a distribution shifted to smaller sizes, although not total. Figure 5 shows distributions for different samples and also shows said shift to smaller sizes (below 100 bp), although a small proportion is observed at values higher than 150 bp (base pairs). In a sample with less than 3% of fetal DNA, both distributions tend to be the same. This effect is observed in Figure 6.

Random Forest study to establish predictor variables and fetal DNA estimation model

The distributions of the number of sequence reads of each bin against the size of the fragments corresponding to each read, both for the section in genomic regions rich in fetal DNA (fetal section), and for all the genomic regions (overall section: maternal + fetal), for each sample, provides information on the fetal DNA.

More than 1000 possible dependent variables were added for each sample. Additionally, the gestational week, mother's height and mother's weight were added to the model.

Figure 7 shows the weight of the first 50 variables and most important dependent variables of the model using the IncNodePurity algorithm (Kuhn S et al. 2008) to weigh said variables. In said figure, it is observed that the gestational week and mother's weight are variables of high importance for the correct prediction of the percentage of fetal DNA. Within this group, there are variables both of the overall section (contain the term "All") as of the exclusive fetal region (they do not contain the term "All").

To be able to evaluate the predictor capacity of the model, 50% of the samples were used as training set and the other 50 as test set. A Random Forest was performed (Liaw A and Wiener M 2002) and a linear regression was subsequently performed to adjust the prediction.

During processing of the training group and obtaining the models using the "Random Forest" methodology, it was verified that the gestational week is one of the most informative variables.

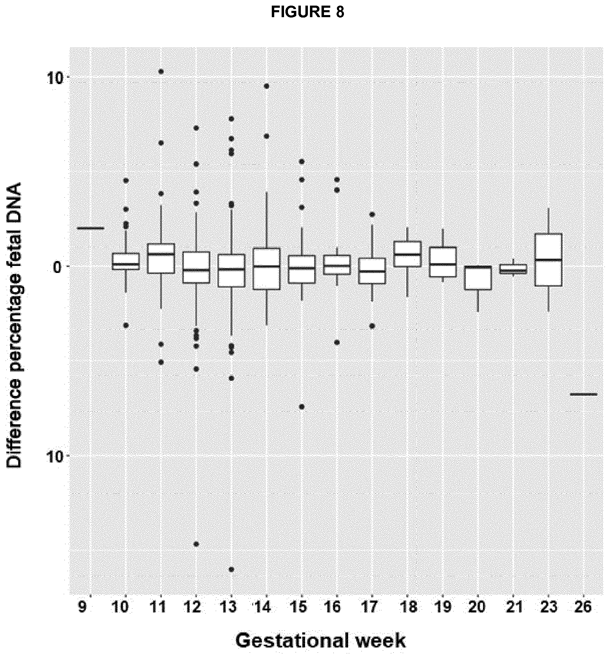

Figure 8 shows the difference between the percentage of fetal DNA predicted by the Random Forest model according to the invention and the percentage of fetal DNA determined by the chrY method.

**Analysis of the results**

To measure the effect of the selection of the genomic regions enriched in fetal DNA on the method precision, the same method for fetal DNA estimation and selection of the best prediction model was performed using all the unfiltered sequence reads per genomic region. Table 12 shows the fetal DNA estimation errors using the genomic regions enriched in fetal DNA and using all the unfiltered sequence reads per genomic region, in comparison with percentages of fetal DNA determined by the chrY method in samples of women pregnant with male fetuses and by digital PCR in samples of mothers pregnant with female fetuses. In Table 12, it can be observed that the strategy wherein all the unfiltered sequence reads per genomic region are used has a greater error level than the strategy of selection of genomic regions enriched in fetal DNA.

Table 12. Percentiles of percentage estimation errors of fetal DNA using all the regions, all the unfiltered sequence reads per genomic region, and using the genomic regions enriched in fetal DNA.

Table 12

|  | 1% | 5% | 25% | 50% | 90% | 95% |
|---|---|---|---|---|---|---|
| All the unfiltered sequence reads per genomic region (all the regions) | 0.03 | 0.16 | 0.67 | 1.48 | 5.25 | 7.06 |
| Genomic regions enriched in fetal DNA | 0.02 | 0.08 | 0.33 | 0.67 | 2.91 | 4.13 |

**Example 11. Determination of the number of copies of chromosomes and chromosome fragments**

The analysis was based on the comparison of the numbers of sequence reads obtained in whole genome sequencing per bin of the samples to be studied with a series of circulating DNA control samples from maternal plasma of mothers with healthy fetuses.

**Bioinformatics analysis**

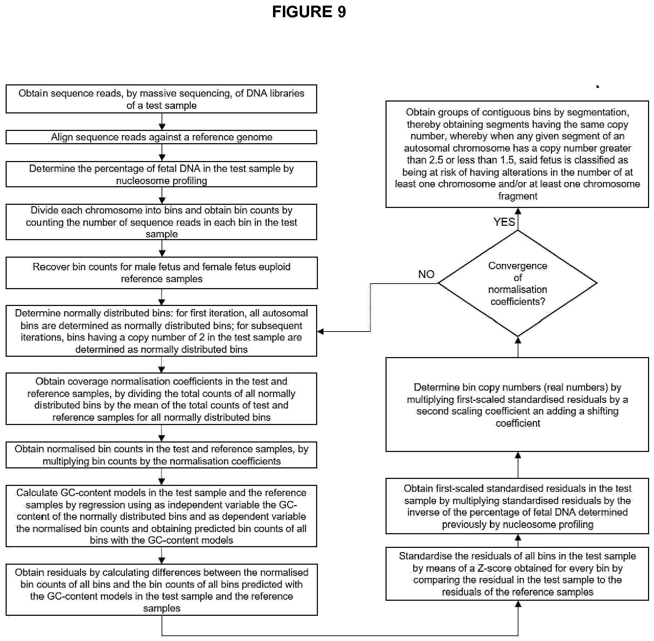

Figure 9 shows the flow diagram of the bioinformatics analysis of the method of the invention for determining the number of copies of chromosomes and the number of copies of chromosome fragments and shows the steps performed in the bioinformatics analysis.

Alignment step was performed with the BWA-MEM algorithm.

Division of the chromosome into bins was performed with the bedtools tool. The number of sequence reads (counts) was obtained by counting (adding up) the reads that map to each bin.

The number of reads i in each genomic region was divided into non-sexual chromosomes against the average for that region obtained from cases and controls, thus obtaining a normalization factor per number of reads (coverage). Then, the normalization factor was multiplied by the number of reads for each bin.

Residuals were calculated using the formula:

$$Residuals_{i,j} = NormalizedCounts_{i,j} - GCCountPredictions_{i,j} \forall i \in samples, \forall j \in bins$$

Z-score was calculated using the formula:

$$StandardResiduals_j = \frac{Residuals_{case,j} - mean(Residuals_{i,j} \forall i \in controls)}{sd(Residuals_{i,j} \forall i \in controls)} \forall j \in bins$$

Residuals were transformed by a scaling factor. The scaling factor is an empirical factor and is adjusted to the sex and fetal nucleosome profile.

$$TransformedResiduals_j = StandardResiduals_j \frac{ScalingFactor}{fetalFraction_{case}} + ShiftingCoefficient \forall j \in bins$$

Segmentation was performed using the formula:

$$Segmentation_k = segmentation(TransformedResiduals_j \forall j \in chr_k) \forall k \in chrs$$

The definitions of the terms used in the mathematical expressions described above are provided below.

*case:* case sample.
*controls:* Control samples.
*samples:* All the samples:

$$samples \supset case \cup controls$$

*bins:* Total bins into which the genome is divided.

*ScalingFactor.* Scaling factor to transform into number of copies; they are factors determined empirically in the initial validation phase.

*ShiftingCoefficient:* "shifting" coefficient to transform into number of copies. *chrs:* Set of chromosomes (autosomes and sex chromosomes), which would be 1, 2, ..., 22, X, Y.

*chr_k:* Set of bins of the chromosome *k,* wherein

$$k \in chrs$$

*fetalFraction_{case}:* Fetal fraction determined for the case sample, using the fetal fraction estimation method by nucleosome profiling of the method of the invention.

Alignment of sequences of control samples against the reference genome. Elimination of reads with uncertainty

Alignment of the paired-end sequence reads from the sequencing was performed against a reference genome. Reads wherein only the sense or antisense sequence maps against the reference genome (singletons) and the reads with a too large size (unproperly paired) were removed.

Division of the chromosome into bins and obtaining number of sequence reads of each bin

Each chromosome was divided into 500 Kb bins. The guanine and cytosine content was obtained (GC content) in each bin and the number of sequence reads aligned in each bin (vertical coverage).

Normalization of the number of sequence reads of each bin

Due to the fact that the total number of reads per sample is variable, a normalization was performed of the number of sequence reads of each bin based on the total number of reads of each sample. To confer a greater robustness to the method, only those reads from somatic and stable chromosomes were considered for the normalization (those wherein alterations in the number of copies have not been detected).

Filtering

Since the average coverage was very low, a prior filtering step was applied with an ARMA (autorregressive moving average) filter, aimed at smoothing the sequence read number curve in each bin.

Predictions of the GC content model of each bin. Obtaining residuals. Standardization of the residuals by means of Z-score with averages and deviations between the sample and the control samples

There is a close relationship between the GC content of each bin and the value of the number of its sequence reads. Hence, local Loess regression models were obtained for each sample based on the GC content, by means of the scatterplot smoothing method. Loess models thus obtained were used for obtaining predictions per bin based on the GC content.

The differences (residuals) were obtained between number of sequence reads of each bin and predictions of number of sequence reads, a guanine and cytosine content model, of each bin, of the sample and of control samples.

The residuals were calculated by means of Z-score with averages and deviations between the sample and the control samples.

Grouping of individual estimates of number of copies of chromosomes in each bin bv segmentation

A segmentation process was performed, aimed at grouping the individual estimates of numbers of copies obtained for each bin.

The segmentation process uses the spatial ratio, i.e. the coordinates of the points and if they are adjacent or not (similar to the Markov chain), which is necessary when there is a lot of noise (both technical and biological) in the points, individually. Therefore, the spatial ratio between points was used (and which must be affected or healthy in an overall or partial alteration), the average value was taken, and the dispersion was decreased, since in a certain area there must be similar values and the dispersions are due to irrelevant specific fluctuations in this process.

Thus, general estimates of the number of copies of each bin were obtained, and additionally, of the numbers of copies of each chromosome.

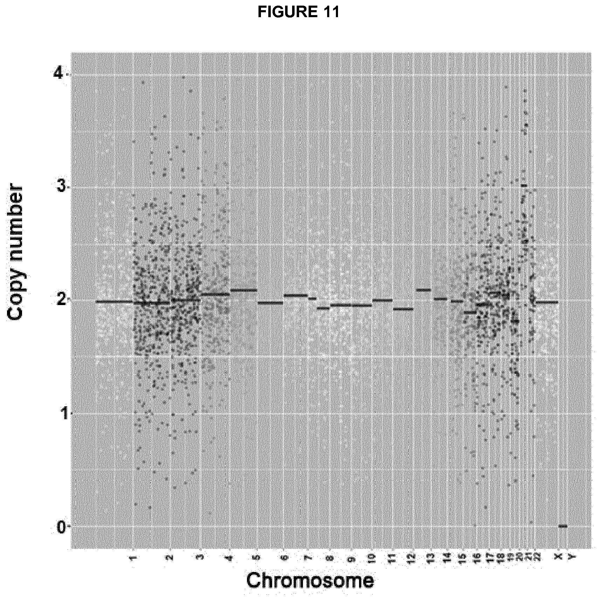

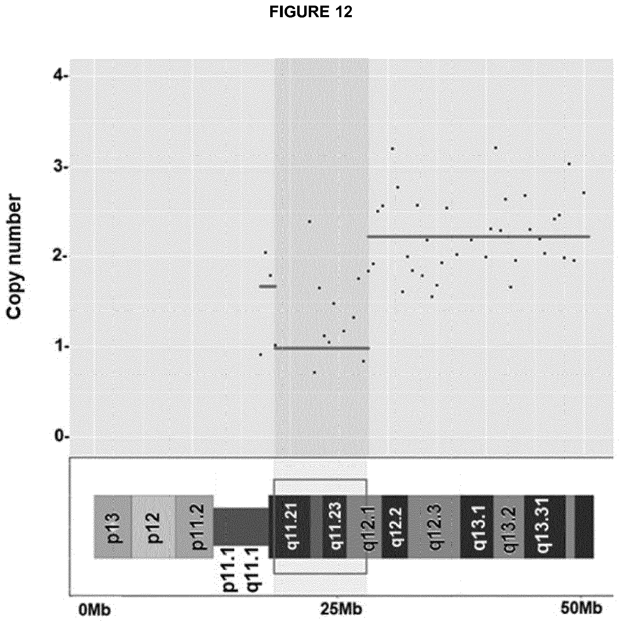

Figure 11 and 12 shows groups of contiguous bins and segments obtained by segmentation. Segments for said groups of contiguous bins have the same copy number.

Segments don't have a predefined or fixed size. The segmentation process determines if all bins from a chromosome apparently have the same copy number (only one segment is created) or assigns the bins of the chromosome to groups of adjacent/contiguous bins having the same copy number (several segments are created). The segmentation process determines copy numbers for every segment.

Adjustment of the number of copies of chromosomes and chromosome fragments by the value of the percentage of fetal DNA determined by nucleosome profiling

A factor of special influence on the sensitivity of the method of the invention for determining alterations in the number of chromosomes or chromosome fragments is the percentage of fetal DNA. The percentage of fetal DNA determines the differences between chromosomes with different number of copies in the fetus, both in the number of reads in each bin and in the rest of the values determined in the present method (normalized reads, residuals, standardized residuals, etc.). Thus, the residuals are transformed (scaled) based on the value of the previously obtained percentage of fetal DNA to guarantee that the obtained values represent the real number of copies of the bin.

The adjustment process consists of a scaling process based on the previously obtained value of the percentage of fetal DNA based on empirical scaling and shifting factors.

General estimates of the number of copies of at least one chromosome and/or at least one chromosome fragment were obtained based on the value of the previously obtained percentage of fetal DNA, thus obtaining an adjusted number of copies of at least one chromosome and/or an adjusted number of copies of at least one chromosome fragment.

**Analysis of the results**

The results of the bioinformatics analysis in a sample are shown below.

The details of the sample appear in Table 13.

Table 13. Details of the sample

| Gestation days | Mother's weight (Kg) | Mother's height (cm) |
|---|---|---|
| 70 | 57 | 167 |

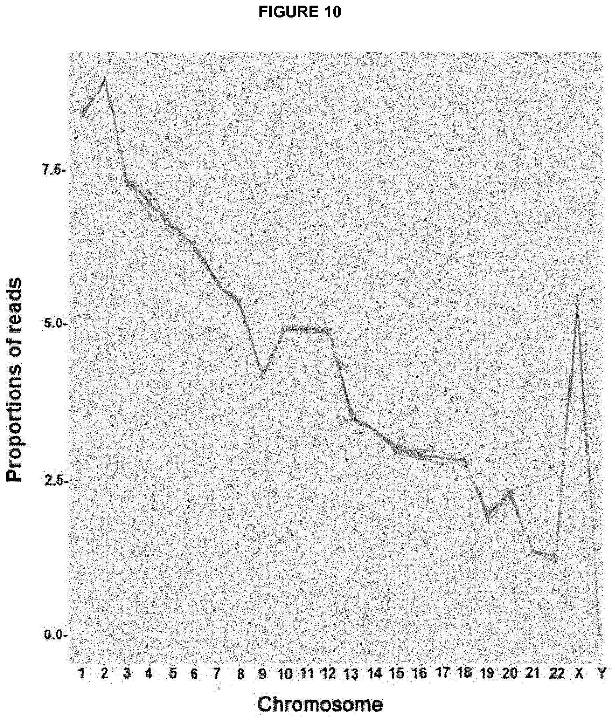

Figure 10 shows the graph of proportions of sequence reads per chromosome with respect to the total of reads, both for the sample and for the control samples.

Table 14 shows the statistics of the mapping performed after the sequencing.

Table 14. Mapping statistics

| Total number of reads | Mapped reads | HQ reads | PP reads | Unduplicated reads | Useful sequencing coverage | % useful reads |
|---|---|---|---|---|---|---|
| 10161841 | 10129627 | 9574019 | 9249595 | 9167225 | 0.2961283 | 90.21225 |

"HQ reads" relate to reads mapping with a high-quality value (not repetitive reads, nor mapping in areas of high homology or complexity, etc). "HQ" relates to "High Quality".
"PP reads" relate to paired-end reads with insert size within the expected range. "PP" relates to "properly paired".

Figure 11 shows the copy numbers per chromosome. This figure shows that the fetus may be affected by a trisomy in chromosome 21. The sample had a percentage of fetal DNA of 11%. The details of the results for this sample appear in Table 15.

Table 15. Results for the sample

|  | chrom. 1 | chrom. 2 | chrom. 3 | chrom. 4 |
|---|---|---|---|---|
| confidence of healthy chromosome | 0.979 | 0.959 | 0.997 | 0.906 |
| confidence of affected chromosome | 0.028 | 0.03 | 0.027 | 0.035 |
| risk of aneuploidy | low risk | low risk | low risk | low risk |
|  | chrom. 5 | chrom. 6 | chrom. 7 | chrom. 8 |
| confidence of healthy chromosome | 0.84 | 0.961 | 0.923 | 0.936 |
| confidence of affected chromosome | 0.043 | 0.03 | 0.034 | 0.032 |
| risk of aneuploidy | low risk | low risk | low risk | low risk |
|  | chrom. 9 | chrom. 10 | chrom. 11 | chrom. 12 |
| confidence of healthy chromosome | 0.925 | 092 | 1 | 0.862 |
| confidence of affected chromosome | 0.033 | 0.034 | 0.026 | 0.04 |
| risk of aneuploidy | low risk | low risk | low risk | low risk |
|  | chrom. 13 | chrom. 14 | chrom. 15 | chrom. 16 |
| confidence of healthy chromosome | 0.837 | 0.979 | 0.985 | 0.811 |
| confidence of affected chromosome | 0.044 | 0.028 | 0.028 | 0.047 |
| risk of aneuploidy | low risk | low risk | low risk | low risk |
|  | chrom. 17 | chrom. 18 | chrom. 19 | chrom. 20 |
| confidence of healthy chromosome | 0.936 | 0.88 | 0.915 | 0.681 |
| confidence of affected chromosome | 0.032 | 0.038 | 0.034 | 0.07 |
| risk of aneuploidy | low risk | low risk | low risk | low risk |
|  | chrom. 21 | chrom. 22 | chrom. X | chrom. Y |
| confidence of healthy chromosome | 0.024 | 0.994 | 0.97 | 0.579 |
| confidence of affected chromosome | 0.968 | 0.027 | 0.029 | 0.026 |
| risk of aneuploidy | high risk | low risk | low risk | low risk |

Table 16 shows the estimated results with confidence intervals.

Table 16

|  | Confidence of healthy chromosome | Confidence of affected chromosome | Estimated risk | Result |
|---|---|---|---|---|
| chrom. 21 (Down Syndrome) | 0.024 | 0.968 | high risk |  |
| chrom. 18 (Edwards syndrome) | > 0.5 | < 0.35 | low risk |  |
| chrom. 13 (Patau Syndrome) | > 0.5 | < 0.35 | low risk |  |
| Rest autosomes | > 0.5 | < 0.35 | low risk |  |
| X chrom. | > 0.5 | < 0.35 | low risk |  |
| Y chrom. | > 0.5 | < 0.35 | low risk |  |
| Fetal sex |  |  |  | Female |

The fetus of the analysed sample had a high risk for trisomy of chromosome 21, indicative of the presence of Down syndrome. In the sample, the absence or presence of the Y chromosome in the fetus was also analysed, according to Example 12 below. The fetus of the analysed sample had absence of the Y chromosome, indicative that the sex of the fetus is female.

A sample was analysed for determining fetal alterations in the number of chromosome fragments in the chromosome region 22q11.21-22q12.1, alterations that would indicate that the fetus may suffer DiGeorge syndrome. The results are shown in Figure 12. The fetus of the sample analysed had a high risk of a deletion in the chromosome region 22q11.21-22q12.1, a deletion indicative of DiGeorge syndrome.

## Example 12. Determination of fetal sex

In this example, a normalization by the GC content was not performed. The GC content models do not adapt to the Y chromosome counts as said models are prepared based on the number of reads in all the bins throughout the genome, bins which in their majority have a high fraction of maternal DNA in addition to fetal DNA, something which does not happen in the case of the Y chromosome.

### Bioinformatics analysis

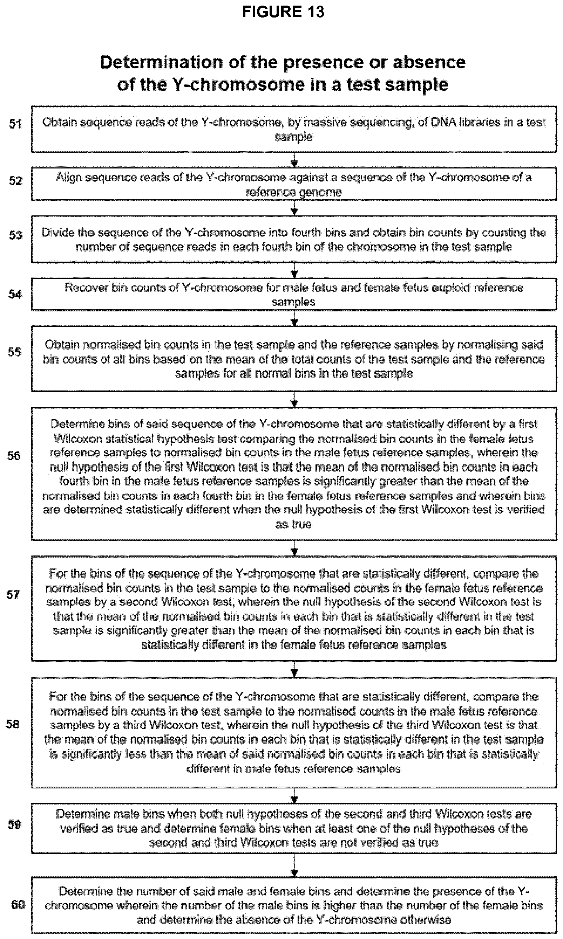

Figure 13 shows the flow diagram of the bioinformatics analysis of the method of the invention for determining the absence or presence of the Y chromosome, and it also shows the steps performed in the bioinformatics analysis. the percentage of fetal DNA in the sample by nucleosome profiling was determined as previously indicated.

Alignment was performed with the BWA-MEM algorithm.

Division of the chromosome into bins was performed with the bedtools tool. The number of sequence reads (counts) was obtained by counting (adding up) the reads mapping to each bin.

Table 17 shows the substeps of the algorithm used in steps 54-56.

Table 17

| Use of the normalized number of reads for Y chromosome |
|---|
| Determination of the "stable" bins for determination of presence of Y chromosome (those wherein there are no reads in the female controls) |
| Calculation of the p-values of the Wilcoxon test assuming that the sample has more numbers of reads than the female controls |

Alignment. Division of the Y chromosome into bins

Sequence reads of the Y chromosome from the massive sequencing were aligned against a reference genome.

The Y chromosome was divided into bins with a base size of 500 Kb.

Bin selection

Only those bins wherein a clear difference was manifested between male and female controls were selected. For the selection of these bins, a Wilcoxon test was performed. The selection of bins by means of the Wilcoxon test avoided alignment artefacts, such as those, for example, due to the pseudoautosomal regions (PAR regions).

Normalization

The number of sequence reads of each bin selected previously were normalized based on the total number of sequence reads.

Determination of the presence or absence of the Y chromosome

The ratio between number of sequence reads of each bin selected from sample and number of sequence reads of each bin selected from control samples was determined.

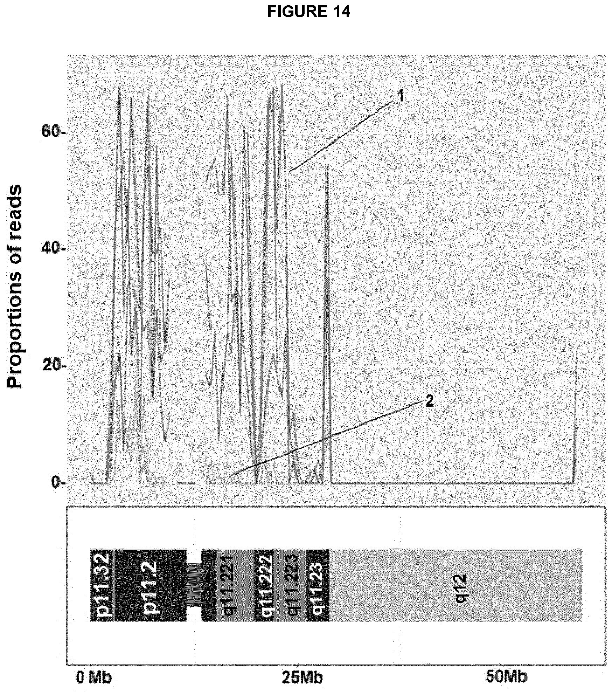

Figure 14 shows a comparison of normalized number of reads of Y chromosome between male controls and female controls. In Figure 14, great differences are observed in the normalized number of reads between male controls and female controls.

The presence or absence of the Y chromosome was determined based on the statistical significance obtained for said previously obtained ratio, wherein said presence or absence of the Y chromosome is indicative of the fetal sex. It was determined that there was statistical significance when the p-value for the Wilcoxon test performed per bin was greater than a threshold value.

The threshold value for determining that a bin has more reads than those expected for a woman based on female controls is 0.7.

The threshold value for determining that a bin has less reads than those expected for a male based on male controls is 0.15.

The determination of the fetal sex was performed on the sample analysed in Example 12 and the result obtained is shown in Table 16. Said sample showed an absence of Y chromosome, indicative that the sex of the fetus is female.

## List of References

Lam WKJ et al. (2018). Sequencing-based counting and size profiling of plasma Epstein-Barr virus DNA enhance population screening of nasopharyngeal carcinoma. Proc. Natl. Acad. Sci. USA., 115(22):E5115-E5124.

Love MI et al. (2014). Moderated estimation of fold change and dispersion for RNA-seq data with DESeq2. Genome Biology, 15, 550.

Kuhn S et al. (2008). Building blocks for automated elucidation of metabolites: machine learning methods for NMR prediction. BMC Bioinformatics, 9:400.

Liaw A and Wiener M (2002). Classification and Regression by randomForest. R News, 2(3), 18-22.

Hudecova I et al. (2014). Maternal plasma fetal DNA fractions in pregnancies with low and high risks for fetal chromosomal aneuploidies. PLoS One, 9(2).

## Claims

1. A method for determining the percentage of fetal DNA in a maternal plasma test sample containing maternal DNA and fetal DNA, comprising:

    (a) calculating a reference random forest regression model for predicting percentage of fetal DNA, comprising the steps of:

    (i) extracting DNA present in reference samples and repairing said DNA, wherein said reference samples are maternal plasma reference samples containing maternal DNA and fetal DNA from fetuses with a set of euploid chromosomes and wherein the percentage of fetal DNA in said reference samples has previously been determined,
    (ii) synthesising libraries of the DNA present in said reference samples, amplifying and labelling said libraries by PCR,
    (iii) obtaining sequence reads, by massive sequencing, of said amplified and labelled libraries,
    (iv) aligning said sequence reads against a reference genome,
    (v) dividing the sequence of each chromosome into first bins with a base size from 50 to 200 Kb, with a first sliding bin with a base size from 5 to 15 Kb and obtaining bin counts by counting the number of sequence reads in each said first bin, wherein each first bin base size is the same, wherein each first sliding bin base size is the same and wherein each first sliding bin base size is less than or equal to the first bin base size,
    (vi) obtaining normalised bin counts by normalising said bin counts obtained in step (a)(v) based on the total bin counts in all first bins in said reference samples,
    (vii) calculating a regression for each first bin using all said reference samples using the normalised bin count as the independent variable, using percentage of fetal DNA of said reference samples as the dependent variable, and using gestation week, mother's height, mother's weight and mother's intake of heparin as co-variables,
    (viii) identifying genomic regions enriched in fetal DNA, wherein said genomic regions enriched in fetal DNA are those for which the statistical significance in the regression of the previous step, as determined by the p value, is greater than $10^{-10}$ and the Spearman correlation coefficient between percentage of fetal DNA predicted by said regression and the percentage of fetal DNA previously determined is greater than or equal to 0.5 and wherein said regions are not regions of the X chromosome,
    (ix) obtaining a density of normalised bin counts of reads for each said reference sample which map to said genomic regions enriched in fetal DNA against insert size of reads for each said reference sample which map to said genomic regions enriched in fetal DNA and obtaining a density curve from said values obtained, wherein said density curve is a reference nucleosome profile,

(x) dividing said reference nucleosome profile into second bins of insert sizes with a base size from 2 to 100 nucleotides, with a second sliding bin with a base size from 1 to 20 nucleotides, wherein each second bin base size is the same, wherein each second sliding bin base size is the same and wherein each second sliding bin base size is less than or equal to the second bin base size and calculating the value of first variables selected from the group consisting of mean, minimum, maximum and the accumulative area under the curve for each second bin,

(xi) determining the variable importance of said first variables in said references samples by a random forest classification and determining a set of first predictive variables in said reference samples, wherein said predictive variables in said set are the n-most important variables as classified by said variable importance, wherein n is in a range from 100 to 300,

(xii) generating a first matrix containing the values in said reference samples of second predictive variables, wherein said second predictive variables comprise said first predictive variables and further comprise gestation week, mother's height, mother's weight and mother's intake of heparin,

(xiii) calculating a reference random forest regression model for predicting percentage of fetal DNA by growing random forest regression trees for said reference samples using part of said reference samples as a training set and part of said reference samples as a test set and using said first matrix containing the values of said second predictive variables as input to said reference random forest regression model, and by obtaining an average based on the trees obtained; and

(b) determining the percentage of fetal DNA in said test sample, comprising:

(i) extracting DNA from said test sample and repairing said DNA,

(ii) synthesizing libraries of the DNA present in said test sample, amplifying and labelling said libraries by PCR,

(iii) obtaining sequence reads, by massive sequencing, of said amplified and labelled libraries,

(iv) aligning said sequence reads against a reference genome,

(v) dividing the sequence of each chromosome into first bins as per step (a)(v) with a base size from 50 to 200 Kb, with a first sliding bin as per step (a)(v) with a base size from 5 to 15 Kb and obtaining bin counts by counting the number of sequence reads in each first bin in said test sample,

(vi) obtaining normalised bin counts by normalising said bin counts obtained in step (b)(v) based on the total bin counts in all first bins in said test sample,

(vii) obtaining a density of normalised bin counts of reads for said test sample which map to said genomic regions enriched in fetal DNA against insert size of reads in said test sample which map to said genomic regions enriched in fetal DNA and obtaining a density curve from said values obtained, wherein said density curve is a test nucleosome profile,

(viii) dividing said test nucleosome profile into second bins as per step (a)(x) with a base size from 2 to 100 nucleotides, with a second sliding bin as per step (a)(x) with a base size from 1 to 20 nucleotides and calculating the value of said first variables selected from the group consisting of mean, minimum, maximum and the accumulative area under the curve for each said second bin,

(ix) generating a second matrix containing the values in said test sample of said second predictive variables obtained in step (a)(xii) and

(x) determining the value of the percentage of fetal DNA in the test sample by growing random forest regression trees using said reference random forest model for predicting percentage of fetal DNA calculated in step (a)(xiii), using said second matrix containing the values in said test sample of second predictive variables as input to said reference random forest model and obtaining estimated values of the percentage of fetal DNA for each tree in the test sample, the average of said estimated values being the percentage of fetal DNA in said test sample.

2. The method according to claim 1, wherein said regression is selected from the group consisting of linear regression, exponential regression and logarithmical regression.

3. The method according to claim 1 or 2, wherein n is in a range selected from the group consisting of 120 to 280, 140 to 260, 160 to 240 and 180 to 220.

4. A computer program product comprising a computer-readable medium, wherein instructions are coded to control a computer system comprising means for determining the percentage of fetal DNA in a maternal plasma test sample containing maternal DNA and fetal DNA according to the method of any one of claims 1-3, said computer system comprising the following means for calculating a reference random forest regression model for predicting percentage of fetal DNA:

(a) means for recording DNA sequence reads obtained from reference samples, wherein said reference samples are maternal plasma reference samples containing maternal DNA and fetal DNA from fetuses with a set of euploid chromosomes and wherein the percentage of fetal DNA in said reference samples has previously been determined,

(b) means for aligning said sequence reads against a reference genome,

(c) means for dividing the sequence of each chromosome into first bins with a base size from 50 to 200 Kb, with a first sliding bin with a base size from 5 to 15 Kb and obtaining bin counts by counting the number of sequence reads in each said first bin, wherein each first bin base size is the same, wherein each first sliding bin base size is the same and wherein each first sliding bin base size is less than or equal to the first bin base size,

(d) means for obtaining normalised bin counts by normalising said bin counts based on the total bin counts of all first bins in said reference samples,

(e) means for calculating a regression for each first bin in said reference samples using the normalised bin count as the independent variable, using percentage of fetal DNA of said reference samples as the dependent variable, and using gestation week, mother's height, mother's weight and mother's intake of heparin as co-variables,

(f) means for identifying genomic regions enriched in fetal DNA, wherein said genomic regions enriched in fetal DNA are those for which the statistical significance in the previous regression, as determined by the p value, is greater than $10^{-10}$ and the Spearman correlation coefficient between percentage of fetal DNA predicted by said regression and the percentage of fetal DNA previously determined is greater than or equal to 0.5 and wherein said regions are not regions of the X chromosome,

(g) means for obtaining a density of normalised bin counts of reads for each said reference sample which map to said genomic regions enriched in fetal DNA against insert size of reads for each said reference sample which map to said genomic regions enriched in fetal DNA and obtaining a density curve from said values obtained, wherein said density curve is a reference nucleosome profile,

(h) means for dividing said reference nucleosome profile into second bins of insert sizes with a base size from 2 to 100 nucleotides, with a second sliding bin with a base size from 1 to 20 nucleotides, wherein each second bin base size is the same, wherein each second sliding bin base size is the same and wherein each second sliding bin base size is less than or equal to the second bin base size and calculating the value of first variables selected from the group consisting of mean, minimum, maximum and the accumulative area under the curve for each second bin,

(i) means for determining the variable importance of said first variables in said references samples by a random forest classification and determining a set of first predictive variables in said reference samples, wherein said predictive variables in said set are the n-most important variables as classified by said variable importance, wherein n is in a range from 100 to 300,

(j) means for generating a first matrix containing the values in said reference samples of second predictive variables, wherein said second predictive variables comprise said first predictive variables and further comprise gestation week, mother's height, mother's weight and mother's intake of heparin,

(k) means for calculating a reference random forest regression model for predicting percentage of fetal DNA by growing random forest regression trees for said reference samples using part of said reference samples as a training set and part of said reference samples as a test set and using said first matrix containing the values of said second predictive variables as input to said reference random forest regression model, and by obtaining an average based on the trees obtained; and,

wherein said computer system additionally comprises the following means for determining the percentage of fetal DNA in said test sample:

(l) means for recording DNA sequence reads obtained from said test sample,

(m) means for aligning said sequence reads against a reference genome,

(n) means for dividing the sequence of each chromosome into first bins with a base size from 50 to 200 Kb, with a first sliding bin with a base size from 5 to 15 Kb and obtaining bin counts by counting the number of sequence reads in each bin in said test sample,

(o) means for obtaining normalised bin counts by normalising said bin counts based on the total bin counts in all first bins in said test sample,

(p) means for obtaining a density of normalised bin counts of reads for said test sample which map to said genomic regions enriched in fetal DNA against insert size of reads in said test sample which map to said genomic regions enriched in fetal DNA and obtaining a density curve from said values obtained, wherein said density curve is a test nucleosome profile,

(q) means for dividing said test nucleosome profile into second bins with a base size from 2 to 100 nucleotides, with a second sliding bin with a base size from 1 to 20 nucleotides and calculating the value of said first variables selected from the group consisting of mean, minimum, maximum and the accumulative area under the curve for each second bin,

(r) means for generating a second matrix containing the values in said test sample of said second predictive variables obtained with means (j), and

(s) means for determining the value of the percentage of fetal DNA in the test sample by growing random forest regression trees using said reference random forest model for predicting percentage of fetal DNA calculated with means (k), using said second matrix containing the values in said test sample of second predictive variables as input to said reference random forest model and obtaining an average based on the trees obtained.

5. A method for determining whether a fetus having fetal DNA is at risk of having alterations in the number of at least one chromosome and/or at least one chromosome fragment, wherein said alterations in the number of chromosomes are complete chromosomal fetal aneuploidies and said alterations in the number of chromosome fragments are partial fetal aneuploidies, in a maternal plasma test sample containing maternal DNA and said fetal DNA, said method comprising:

   (a) extracting DNA from said test sample and repairing said DNA,
   (b) synthesising libraries of the DNA present in said test sample, amplifying and labelling said libraries by PCR,
   (c) obtaining sequence reads, by massive sequencing, of said amplified and labelled libraries,
   (d) aligning said sequence reads against a reference genome,
   (e) determining, according to any one of claims 1-3, the percentage of fetal DNA in said test sample,
   (f) dividing the sequence of each chromosome into third bins with a base size from 100 Kb to 1 Mb and obtaining bin counts by counting the number of sequence reads in each third bin in said test sample,
   (g) recovering bin counts in each third bin in second reference samples, wherein said bin counts in said second reference samples have been previously obtained according to steps (a)-(f) and wherein said second reference samples are maternal plasma reference samples containing maternal DNA and fetal DNA from fetuses with a set of euploid chromosomes,
   (h) followed by the following steps:

      (i) determining all autosomal third bins as normal bins in said test sample,
      (ii) obtaining coverage normalisation coefficients in said test sample and said second reference samples by dividing the bin counts in each said normal bins by the mean of the total counts in said test sample and said second reference samples for all said normal bins,
      (iii) obtaining normalised bin counts in said test sample and said second reference samples by multiplying said bin counts of all bins in said test sample and second reference samples by said coverage normalisation coefficients,
      (iv) calculating a GC-content model for said test sample and a GC-content model for each said second reference sample by regression, using the GC-content of said normal bins as the independent variable and using said normalised bin counts as the dependent variable and obtaining predicted bin counts of all bins in said test sample and said second reference samples with said GC-content models,
      (v) obtaining residuals by calculating differences between the normalised bin counts of all third bins and said predicted bin counts of all third bins in said test sample and said second reference samples,
      (vi) obtaining standardised residuals of all third bins in said test sample by standardising said residuals of all third bins in said test sample by means of a Z-score obtained for each bin by comparing said residual of each third bin in said test sample to said residual of the corresponding third bin in said second reference samples,
      (vii) obtaining first-scaled standardised residuals in said test sample by multiplying said standardised residuals in said test sample by a first scaling coefficient, said first scaling coefficient being the inverse of said percentage of fetal DNA determined in step (e),
      (viii) determining bin copy numbers for each third bin by multiplying said first-scaled standardised residuals by a second scaling coefficient, thereby obtaining second-scaled standardised residuals and by adding a shifting coefficient to said second-scaled standardised residuals, wherein said second scaling coefficient has been previously determined from first-scaled standardised residuals and bin copy numbers for each third bin in third reference samples, wherein said third reference samples are maternal plasma reference samples containing maternal DNA and fetal DNA from fetuses with a set of aneuploid chromosomes, wherein said shifting coefficient has been previously determined from first-scaled standardised residuals and bin copy numbers for each third bin in said second reference samples,

(ix) eliminating third bins having a copy number different determining aneuploidy from the group of said normal bins in said test sample,

(x) repeating steps (h)(ii) to (h)(ix),

(xi) repeating step (h)(x) if said coverage normalisation coefficients differ more than a value in the range 0.1-5% with respect to previous said coverage normalisation coefficients,

(i) obtaining groups of contiguous bins by segmentation, thereby obtaining segments having the same copy number, whereby when any given segment of an autosomal chromosome has a copy number greater than 2.5 or less than 1.5, said fetus is classified as being at risk of having alterations in the number of at least one chromosome and/or at least one chromosome fragment.

6. The method according to claim 5, wherein in step (h)(iv) said regression is a LOESS regression or a polynomial regression.

7. The method according to claim 5 or 6, wherein in step (h)(v) said residuals are obtained by subtracting said predicted bin counts of all third bins from said normalised bin counts of all third bins in said test sample and said reference samples.

8. The method according to any one of claims 5-7, wherein said segmentation is circular binary segmentation (CBS) or segmentation based on a hidden Markov model (HMM).

9. The method according to any one of claims 5-8, wherein said complete chromosomal fetal aneuploidies are found in a chromosome selected from the group consisting of 9, 13, 15, 16, 18, 21, 22, X sexual chromosome and Y sexual chromosome.

10. The method according to any one of claims 5-9, wherein said partial fetal aneuploidies are associated with the syndromes selected from the group consisting of Angelman syndrome, Prader-Willi syndrome, Cri du Chat syndrome, Wolf-Hirschhorn syndrome, Jacobsen syndrome, Langer-Giedion syndrome, DiGeorge syndrome, DiGeorge II syndrome and Phelan-McDermid syndrome.

11. The method according to any one of claims 5-10, wherein said partial fetal aneuploidy consists of a 1p36 deletion or a 16p11.2-p12.1.2 deletion.

12. The method according to any one of claims 5-11, wherein said value in step (h)(xi) is in a range selected from the group consisting of 0.2-4%, 0.3-3%, 0.4-2%, 0.5-1.5%, and 0.8-1.2%.

13. A computer program product comprising a computer-readable medium, wherein instructions are coded to control a computer system comprising means for determining whether a fetus having fetal DNA is at risk of having alterations in the number of at least one chromosome and/or at least one chromosome fragment according to the method of any one claims 5-12, wherein said alterations in the number of chromosomes are complete chromosomal fetal aneuploidies and said alterations in the number of chromosome fragments are partial fetal aneuploidies, in a maternal plasma test sample containing maternal DNA and said fetal DNA, said computer system comprising the following means:

(a) means for recording DNA sequence reads obtained from said test sample,

(b) means for aligning said sequence reads against a reference genome,

(c) means for determining, with the computer program product according to claim 6, the percentage of fetal DNA in said test sample,

(d) means for dividing the sequence of each chromosome into third bins with a base size from 100 Kb to 1 Mb and obtaining bin counts by counting the number of sequence reads in each third bin in said test sample,

(e) means for recovering bin counts in each third bin in second reference samples, wherein said bin counts in said second reference samples have been previously obtained with means (a)-(d) and wherein said second reference samples are maternal plasma reference samples containing maternal DNA and fetal DNA from fetuses with a set of euploid chromosomes,

(f) means for performing the following operations:

(i) means for determining all autosomal third bins as normal bins in said test sample,

(ii) means for obtaining coverage normalisation coefficients in said test sample and said second reference samples by dividing the bin counts in each said normal bins by the mean of the total counts in said test sample and said reference samples for all said normal bins,

(iii) means for obtaining normalised bin counts in said test sample and said second reference samples by multiplying said bin counts of all bins in said test sample and reference samples by said coverage normalisation coefficients,

(iv) means for calculating a GC-content model for said test sample and a GC-content model for each said second reference sample by regression, using the GC-content of said normal bins as the independent variable and using said normalised bin counts as the dependent variable and obtaining predicted bin counts of all bins in said test sample and said second reference samples with said GC-content models,

(v) means for obtaining residuals by calculating differences between the normalised bin counts of all third bins and said predicted bin counts of all third bins in said test sample and said second reference samples,

(vi) means for obtaining standardised residuals of all third bins in said test sample by standardising said residuals of all third bins in said test sample by means of a Z-score obtained for each bin by comparing said residual of each third bin in said test sample to said residual of the corresponding third bin in said second reference samples,

(vii) means for obtaining first-scaled standardised residuals in said test sample by multiplying said standardised residuals in said test sample by a first scaling coefficient, said first scaling coefficient being the inverse of said percentage of fetal DNA determined with means (c),

(viii) means for determining bin copy numbers for each third bin by multiplying said first-scaled standardised residuals by a second scaling coefficient, thereby obtaining second-scaled standardised residuals and by adding a shifting coefficient to said second-scaled standardised residuals, wherein said second scaling coefficient has been previously determined from first-scaled standardised residuals and bin copy numbers for each third bin in third reference samples, wherein said third reference samples are maternal plasma reference samples containing maternal DNA and fetal DNA from fetuses with a set of aneuploid chromosomes, wherein said shifting coefficient has been previously determined from first-scaled standardised residuals and bin copy numbers for each third bin in said second reference samples,

(ix) means for eliminating third bins having a copy number different determining aneuploidy from the group of said normal bins in said test sample,

(x) means for repeating operations (f)(ii) to (f)(ix),

(xi) means for repeating operation (f)(x) if said coverage normalisation coefficients differ more than a value in the range 0.1-5% with respect to previous said coverage normalisation coefficients,

(g) means for obtaining groups of contiguous bins by segmentation, thereby obtaining segments having the same copy number, whereby when any given segment of an autosomal chromosome has a copy number greater than 2.5 or less than 1.5, said fetus is classified as being at risk of having alterations in the number of at least one chromosome and/or at least one chromosome fragment.

**14.** A method for determining the absence or presence of Y-chromosome in a maternal plasma test sample containing maternal DNA and fetal DNA, comprising the following steps:

(a) extracting DNA from said test sample and repairing said DNA,

(b) synthesising libraries of the DNA present in said test sample, amplifying and labelling said libraries by PCR and combining said libraries,

(c) obtaining sequence reads of the Y-chromosome, by massive sequencing, of the amplified, labelled and combined libraries,

(d) aligning said sequence reads of the Y-chromosome against a sequence of the Y-chromosome of a reference genome,

(e) dividing said sequence of the Y-chromosome into fourth bins with a base size from 100 Kb to 1 Mb and obtaining bin counts by counting the number of sequence reads in each fourth bin of said chromosome in said test sample,

(f) recovering bin counts in each fourth bin in second reference samples, wherein said bin counts in said second reference samples have been previously obtained according to steps (a)-(e) and wherein said second reference samples comprise male fetus reference samples and female fetus reference samples,

(g) obtaining normalised bin counts in said test sample and said second reference samples by normalising said bin counts of all bins based on the mean of the total counts of said test sample and said second reference samples for all normal bins in said test sample, wherein said normal bins are said normal bins determined in step (h)(xi) of the method according to claim 5,

(h) determining bins of said sequence of the Y-chromosome that are statistically different by a first Wilcoxon statistical hypothesis test comparing said normalised bin counts in said female fetus reference samples to normalised bin counts in said male fetus reference samples, wherein the null hypothesis of said first Wilcoxon test is that the mean of said normalised bin counts in each fourth bin in said male fetus reference samples is significantly greater than the mean of said normalised bin counts in each fourth bin in said female fetus reference samples and wherein bins are determined statistically different when said null hypothesis of said first Wilcoxon test is verified as true,

(i) for said bins of said sequence of the Y-chromosome that are statistically different, comparing said normalised bin counts in said test sample to said normalised counts in said female fetus reference samples by a second Wilcoxon test, wherein the null hypothesis of said second Wilcoxon test is that the mean of said normalised bin counts in each said bin that is statistically different in said test sample is significantly greater than the mean of said normalised bin counts in each said bin that is statistically different in said female fetus reference samples,

(j) for said bins of said sequence of the Y-chromosome that are statistically different, comparing said normalised bin counts in said test sample to said normalised counts in said male fetus reference samples by a third Wilcoxon test, wherein the null hypothesis of said third Wilcoxon test is that the mean of said normalised bin counts in each said bin that is statistically different in said test sample is significantly less than the mean of said normalised bin counts in each said bin that is statistically different in said male fetus reference samples,

(k) determining male bins when both said null hypotheses of said second and said third Wilcoxon tests are verified as true and determining female bins when at least one of said null hypotheses of said second and said third Wilcoxon tests are not verified as true,

(l) determining the number of said male and female bins and determining the presence of the Y-chromosome wherein the number of said male bins is higher than the number of said female bins and determine the absence of the Y-chromosome otherwise, wherein said presence or absence of the Y-chromosome is indicative of the fetal sex.

15. A computer program product comprising a computer-readable medium, wherein instructions are coded to control a computer system comprising means for determining the absence or presence of Y-chromosome in a maternal plasma test sample containing maternal DNA and fetal DNA according to the method of claim 14, said computer system comprising the following means:

(a) means for recording DNA sequence reads obtained from said test sample,

(b) means for aligning said sequence reads of the Y-chromosome against a sequence of the Y-chromosome of a reference genome,

(c) means for dividing said sequence of the Y-chromosome into fourth bins with a base size from 100 Kb to 1 Mb and obtaining bin counts by counting the number of sequence reads in each fourth bin of said chromosome in said test sample,

(d) means for recovering bin counts in each fourth bin in second reference samples, wherein said bin counts in said second reference samples have been previously obtained and wherein said second reference samples comprise male fetus reference samples and female fetus reference samples,

(e) means for obtaining normalised bin counts in said test sample and said second reference samples by normalising said bin counts of all bins based on the mean of the total counts of said test sample and said second reference samples for all normal bins in said test sample, wherein said normal bins are said normal bins determined with operation (f)(xi) of the computer program product according to claim 13,

(f) means for determining bins of said sequence of the Y-chromosome that are statistically different by a first Wilcoxon statistical hypothesis test comparing said normalised bin counts in said female fetus reference samples to normalised bin counts in said male fetus reference samples, wherein the null hypothesis of said first Wilcoxon test is that the mean of said normalised bin counts in each fourth bin in said male fetus reference samples is significantly greater than the mean of said normalised bin counts in each fourth bin in said female fetus reference samples and wherein bins are determined statistically different when said null hypothesis of said first Wilcoxon test is verified as true,

(g) means for comparing said normalised bin counts in said test sample to said normalised counts in said female fetus reference samples by a second Wilcoxon test for said bins of said sequence of the Y-chromosome that are statistically different, wherein the null hypothesis of said second Wilcoxon test is that the mean of said normalised bin counts in each said bin that is statistically different in said test sample is significantly greater than the mean of said normalised bin counts in each said bin that is statistically different in said female fetus reference samples,

(h) means for comparing said normalised bin counts in said test sample to said normalised counts in said male fetus reference samples by a third Wilcoxon test for said bins of said sequence of the Y-chromosome that are statistically different, wherein the null hypothesis of said third Wilcoxon test is that the mean of said normalised bin counts in each said bin that is statistically different in said test sample is significantly less than the mean of said normalised bin counts in each said bin that is statistically different in said male fetus reference samples,

(i) means for determining male bins when both said null hypotheses of said second and said third Wilcoxon tests are verified as true and determining female bins when at least one of said null hypotheses of said second and said third Wilcoxon tests are not verified as true,

(j) means for determining the number of said male and female bins and determining the presence of the Y-chromosome wherein the number of said male bins is higher than the number of said female bins and determined the absence of the Y-chromosome otherwise, wherein said presence or absence of the Y-chromosome is indicative of the fetal sex.

**Patentansprüche**

1. Eine Methode zur Bestimmung des Prozentsatzes an fetaler DNA in einer maternalen Plasmaprobe, die maternale DNA und fetale DNA enthält, umfassend:

(a) Berechnen eines zufälligen Random Forest Referenz-Regressionsmodells zum Vorhersagen von 5 Prozent der fetalen DNA, umfassend die Schritte:

(i) Extrahieren von DNA, die in Referenzproben vorhanden ist, und Reparieren der DNA, wobei die Referenzproben maternale Plasma-Referenzproben sind, die maternale DNA und fetale DNA aus Feten mit einem Satz von euploiden Chromosomen enthalten, und wobei der Prozentsatz der fetalen DNA in den Referenzproben zuvor bestimmt wurde,

(ii) Synthetisieren von Bibliotheken der in den Referenzproben vorhandenen DNA, Amplifizieren und Markieren der Bibliotheken durch PCR,

(iii) Erhalten von Sequenzlesungen ("sequence reads") durch massive Sequenzierung der amplifizierten und markierten Bibliotheken,

(iv) Alignieren der Sequenzlesungen mit einem Referenzgenom,

(v) Unterteilen der Sequenz jedes Chromosoms in erste Bins ("bins") mit einer Basengröße ("base size") von 50 bis 200 kbp, mit einer ersten Gleit-Bin ("sliding bin") mit einer Basengröße von 5 bis 15 kbp und Erhalten von Bin-Anzahlen ("bin counts") durch Zählen der Anzahl der Sequenzlesungen in jeder ersten Bin, wobei jede erste Bin-Basengröße ("bin base size") die gleiche ist, wobei jede erste Gleit-Bin-Basengröße ("sliding bin base size") die gleiche ist und wobei jede erste Gleit-Bin-Basengröße kleiner oder gleich der ersten Bin-Basengröße ist,

(vi) Erhalten normalisierter Bin-Anzahlen durch Normalisieren der in Schritt (a)(v) erhaltenen Bin-Anzahlen auf der Grundlage der gesamten Bin-Anzahlen in allen ersten Bins in den Referenzproben,

(vii) Berechnen einer Regression für jeden ersten Bin unter Verwendung aller Referenzproben unter Verwendung der normalisierten Bin-Anzahlen als unabhängige Variable, unter Verwendung des Prozentsatzes der fetalen DNA der Referenzproben als abhängige Variable und unter Verwendung der Schwangerschaftswoche, der Körpergröße der Mutter, des Körpergewichts der Mutter und der Aufnahme von Heparin durch die Mutter als Kovariablen,

(viii) Identifizieren genomischer Regionen, die mit fetaler DNA angereichert sind, wobei die genomischen Regionen, die mit fetaler DNA angereichert sind, diejenigen sind, für die die statistische Signifikanz in der Regression des vorherigen Schritts, wie durch den p-Wert bestimmt, größer als $10^{-10}$ ist und der Spearman-Korrelationskoeffizient zwischen dem Prozentsatz der fetalen DNA, die durch die Regression vorhergesagt wird, und dem Prozentsatz der fetalen DNA, der zuvor bestimmt wurde, größer als oder gleich 0,5 ist, und wobei die Regionen keine Regionen des X-Chromosoms sind,

(ix) Erhalten einer Dichte normalisierter Bin-Anzahlen von Sequenzlesungen für jede Referenzprobe, der die genomischen Regionen zugeordnet sind, die mit fetaler DNA angereichert sind, gegen eine Einfügungsgröße ("insert size") von Sequenzlesungen für jede Referenzprobe, die den genomische Regionen, die mit fetaler DNA angereichert sind, zugeordnet sind, und Erhalten einer Dichtekurve aus den erhaltenen Werten, wobei die Dichtekurve ein Referenznukleosomprofil ist,

(x) Unterteilen des Referenznukleosomprofils in zweite Bins mit Einfügungsgrößen mit einer Basengröße von 2 bis 100 Nukleotiden, mit einer zweiten Gleit-Bin mit einer Basengröße von 1 bis 20 Nukleotiden, wobei jede zweite Bin-Basengröße die gleiche ist, wobei jede zweite Gleit-Bin-Basengröße die gleiche ist und wobei jede zweite Gleit-Bin-Basengröße kleiner oder gleich der zweiten Bin-Basengröße ist, und Berechnen des Werts von ersten Variablen, die aus der Gruppe ausgewählt sind, die aus Mittelwert, Minimum, Maximum und der akkumulativen Fläche unter der Kurve für jeden zweiten Bin besteht,

(xi) Bestimmen der variablen Bedeutung der ersten Variablen in den Referenzproben durch eine zufällige Random Forest Klassifizierung und Bestimmen eines Satzes erster prädiktiver Variablen in den Referenzproben, wobei die prädiktiven Variablen in dem Satz die n-wichtigsten Variablen sind, wie sie durch die variable Bedeutung klassifiziert sind, wobei n in einem Bereich von 100 bis 300 liegt,

(xii) Erzeugen einer ersten Matrix, die die Werte in den Referenzproben von zweiten prädiktiven Variablen enthält, wobei die zweiten prädiktiven Variablen die ersten prädiktiven Variablen umfassen und ferner die Schwangerschaftswoche, die Körpergröße der Mutter, das Körpergewicht der Mutter und die Aufnahme von Heparin durch die Mutter umfassen,

(xiii) Berechnen eines Random Forest Referenz-Regressionsmodells zum Vorhersagen des Prozentsatzes der fetalen DNA durch das Anlegen von Random Forest Regressionsbäumen für die Referenzproben unter Verwendung eines Teils der Referenzproben als Trainingssatz und eines Teils der Referenzproben als Testsatz und unter Verwendung der ersten Matrix, die die Werte der zweiten Vorhersagevariablen enthält, als Input in das Random Forest Referenz-Regressionsmodells und durch Erhalten eines Durchschnitts auf der Grundlage der erhaltenen Bäume; und

(b) Bestimmen des Prozentsatzes an fetaler DNA in der Testprobe, umfassend:

(i) Extrahieren von DNA aus der Testprobe und Reparieren der DNA,

(ii) Synthetisieren von Bibliotheken der in der Testprobe vorhandenen DNA, Amplifizieren und Markieren der Bibliotheken durch PCR,

(iii) Erhalten von Sequenzlesungen durch massive Sequenzierung der amplifizierten und markierten Bibliotheken,

(iv) Alignieren der Sequenzlesungen mit einem Referenzgenom,

(v) Unterteilen der Sequenz jedes Chromosoms in erste Bins gemäß Schritt (a)(v) mit einer Basengröße von 50 bis 200 kbp, mit einem ersten Gleit-Bin gemäß Schritt (a)(v) mit einer Basengröße von 5 bis 15 kbp und Erhalten von Bin-Anzahlen durch Zählen der Anzahl der Sequenzlesungen in jedem ersten Bin in der Testprobe,

(vi) Erhalten normalisierter Bin-Anzahlen durch Normalisieren der in Schritt (b)(v) erhaltenen Bin-Anzahlen auf Grundlage der Bin-Gesamtanzahl in allen ersten Bins in der Testprobe,

(vii) Erhalten einer Dichte von normalisierter Bin-Anzahlen von Lesungen für die Testprobe, der die genomischen Regionen zugeordnet sind, die mit fetaler DNA angereichert sind, gegen die Einfügungsgröße von Sequenzlesungen in der Testprobe, die den genomische Regionen, die mit fetaler DNA angereichert sind, zugeordnet sind, und Erhalten einer Dichtekurve aus den erhaltenen Werten, wobei die Dichtekurve ein Testnukleosomprofil ist,

(viii) Aufteilen des Testnukleosomprofils in zweite Bins gemäß Schritt (a) (x) mit einer Basengröße von 2 bis 100 Nukleotiden, mit einer zweiten Gleit-Bin gemäß Schritt (a)(x) mit einer Basengröße von 1 bis 20 Nukleotiden und Berechnen des Werts der ersten Variablen, die aus der Gruppe ausgewählt sind, die aus dem Mittelwert, dem Minimum, dem Maximum und der akkumulativen Fläche unter der Kurve für jeden zweiten Bin besteht,

(ix) Erzeugen einer zweiten Matrix, die die Werte in der Testprobe der in Schritt (a) (xii) erhaltenen zweiten prädiktiven Variablen enthält, und

(x) Bestimmen des Werts des Prozentsatzes der fetalen DNA in der Testprobe durch das Anlegen von Random Forest Regressionsbäumen unter Verwendung des Random Forest Referenzmodells zum Vorhersagen des Prozentsatzes der fetalen DNA, der in Schritt (a)(xiii) berechnet wurde, wobei die zweite Matrix die Werte in der Testprobe von zweiten Vorhersagevariablen als Input in das Random Forest Referenzmodell enthält und geschätzte Werte des Prozentsatzes der fetalen DNA für jeden Baum in der Testprobe erhalten werden, wobei der Durchschnitt der geschätzten Werte der Prozentsatz der fetalen DNA in der Testprobe ist.

2. Die Methode nach Anspruch 1, wobei die Regression aus der Gruppe ausgewählt ist, die aus linearer Regression, exponentieller Regression und logarithmischer Regression besteht.

3. Die Methode nach Anspruch 1 oder 2, wobei n in einem Bereich ist, der aus der Gruppe ausgewählt ist, die aus 120 bis 280, 140 bis 260, 160 bis 240 und 180 bis 220 besteht.

4. Ein Computerprogramm-Produkt, das ein computerlesbares Medium umfasst, wobei Anweisungen kodiert sind, um ein Computersystem zu steuern, das Mittel zum Bestimmen des Prozentsatzes an fetaler DNA in einer maternalen Plasmaprobe, die maternale DNA und fetale DNA enthält, gemäß der Methode nach einem der Ansprüche 1 bis 3 umfasst, wobei das Computersystem die folgenden Mittel zum Berechnen eines Random Forest Referenz-Regressionsmodells zum Vorhersagen des Prozentsatzes an fetaler DNA umfasst:

(a) Mittel zum Aufzeichnen von DNA-Sequenzlesungen, die von Referenzproben erhalten wurden, wobei die Referenzproben Referenzproben aus maternalem Plasma sind, die maternale DNA und fetale DNA von Feten mit einem Satz von euploiden Chromosomen enthalten, und wobei der Prozentsatz der fetalen DNA in den Referenzproben zuvor bestimmt wurde,

(b) Mittel zum Alignieren der Sequenzlesungen mit einem Referenzgenom,

(c) Mittel zum Unterteilen der Sequenz jedes Chromosoms in erste Bins mit einer Basengröße von 50 bis 200 kbp, mit einer ersten Gleit-Bin mit einer Basengröße von 5 bis 15 kbp und Erhalten von Bin-Anzahlen durch Zählen der Anzahl der Sequenzlesungen in jedem ersten Bin, wobei jede erste Bin-Basengröße die gleiche ist, wobei jede erste Gleit-Bin-Basengröße die gleiche ist und wobei jede erste Gleit-Bin-Basengröße kleiner oder gleich der ersten Bin-Basengröße ist,

(d) Mittel zum Erhalten normalisierter Bin-Anzahlen durch Normalisieren der Bin-Anzahlen auf der Grundlage der Bin-Gesamtanzahl aller ersten Bins in den Referenzproben,

(e) Mittel zur Berechnung einer Regression für jeden ersten Bin in den Referenzproben unter Verwendung der normalisierten Bin-Anzahl als unabhängige Variable, unter Verwendung des Prozentsatzes der fetalen DNA der Referenzproben als abhängige Variable und unter Verwendung der Schwangerschaftswoche, der Körpergröße der Mutter, des Körpergewichts der Mutter und der Aufnahme von Heparin durch die Mutter als Kovariablen,

(f) Mittel zum Identifizieren genomischer Regionen, die mit fetaler DNA angereichert sind, wobei die genomischen Regionen, die mit fetaler DNA angereichert sind, diejenigen sind, für die die statistische Signifikanz in der vorherigen Regression, wie durch den p-Wert bestimmt, größer als $10^{-10}$ ist und der Spearman-Korrelationskoeffizient zwischen dem Prozentsatz an fetaler DNA, der durch die Regression vorhergesagt wird, und dem Prozentsatz an fetaler DNA, der zuvor bestimmt wurde, größer als oder gleich 0,5 ist, und wobei die Regionen keine Regionen des X-Chromosoms sind,

(g) Mittel zum Erhalten einer Dichte normalisierter Bin-Anzahlen von Sequenzlesungen für jede Referenzprobe, der die genomischen Regionen zugeordnet sind, die mit fetaler DNA angereichert sind, gegen eine Einfügungsgröße von Sequenzlesungen für jede Referenzprobe, die den genomische Regionen, die mit fetaler DNA angereichert sind, zugeordnet sind, und zum Erhalten einer Dichtekurve aus den erhaltenen Werten, wobei die Dichtekurve ein Referenznukleosomprofil ist,

(h) Mittel zum Aufteilen des Referenznukleosomprofils in zweite Bins mit Einfügungsgrößen einer Basengröße von 2 bis 100 Nukleotiden, mit einer zweiten Gleit-Bin mit einer Basengröße von 1 bis 20 Nukleotiden, wobei jede zweite Bin-Basengröße die gleiche ist, wobei jede zweite Gleit-Bin-Basengröße die gleiche ist und wobei jede zweite Gleit-Bin-Basengröße kleiner oder gleich der zweiten Bin-Basengröße ist, und Berechnen des Werts von ersten Variablen, die aus der Gruppe ausgewählt sind, die aus Mittelwert, Minimum, Maximum und der akkumulativen Fläche unter der Kurve für jede zweite Bin besteht,

(i) Mittel zum Bestimmen der variablen Bedeutung der ersten Variablen in den Referenzproben durch eine Random Forest Klassifizierung und Bestimmen eines Satzes von ersten prädiktiven Variablen in den Referenzproben, wobei die prädiktiven Variablen in dem Satz die n-wichtigsten Variablen sind, wie sie durch die variable Bedeutung klassifiziert sind, wobei n in einem Bereich von 100 bis 300 liegt,

(j) Mittel zum Erzeugen einer ersten Matrix, die die Werte in den Referenzproben von zweiten prädiktiven Variablen enthält, wobei die zweiten prädiktiven Variablen die ersten prädiktiven Variablen umfassen und ferner die Schwangerschaftswoche, die Körpergröße der Mutter, das Körpergewicht der Mutter und die Aufnahme von Heparin durch die Mutter umfassen,

(k) Mittel zum Berechnen eines Random Forest Referenz-Regressionsmodells zum Vorhersagen des Prozentsatzes der fetalen DNA durch das Anlegen von Random Forest Regressionsbäumen für die Referenzproben unter Verwendung eines Teils der Referenzproben als Trainingssatz und eines Teils der Referenzproben als Testsatz und unter Verwendung der ersten Matrix, die die Werte der zweiten Vorhersagevariablen enthält, als Input in das Random Forest Referenz-Regressionsmodell und durch Erhalten eines Durchschnitts auf der Grundlage der erhaltenen Bäume; und

wobei das Computersystem zusätzlich die folgenden Mittel zum Bestimmen des Prozentsatzes an fetaler DNA in der Testprobe umfasst:

(l) Mittel zur Aufzeichnung von DNA-Sequenzmesswerten, die aus der Testprobe gewonnen wurden,

(m) Mittel zum Alignieren der Sequenzlesungen mit einem Referenzgenom,

(n) Mittel zum Unterteilen der Sequenz jedes Chromosoms in erste Bins mit einer Basengröße von 50 bis 200 kbp, mit einer ersten Gleit-Bin mit einer Basengröße von 5 bis 15 kbp und Erhalten von Bin-Anzahlen durch Zählen der Anzahl der Sequenzlesungen in jeder Bin in der Testprobe,

(o) Mittel zum Erhalten normalisierter Bin-Anzahlen durch Normalisieren der Bin-Anzahlen auf der Grundlage der gesamten Bin-Anzahlen in allen ersten Bins in der Testprobe,

(p) Mittel zum Erhalten einer Dichte von normalisierten Bin-Anzahlen von Lesungen für die Testprobe, der die genomischen Regionen zugeordnet sind, die mit fetaler DNA angereichert sind, gegen eine Einfügungsgröße von Sequenzlesungen für jede Testprobe, die den genomische Regionen, die mit fetaler DNA angereichert sind, zugeordnet sind, und zum Erhalten einer Dichtekurve aus den erhaltenen Werten, wobei die Dichtekurve ein Testnukleosomprofil ist,

(q) Mittel zum Aufteilen des Testnukleosomprofils in zweite Bins mit einer Basengröße von 2 bis 100 Nukleotiden, mit einer zweiten Gleit-Bin mit einer Basengröße von 1 bis 20 Nukleotiden und Berechnen des Werts der ersten Variablen, die aus der Gruppe ausgewählt sind, die aus dem Mittelwert, dem Minimum, dem Maximum und der akkumulativen Fläche unter der Kurve für jede zweite Bin besteht,

(r) Mittel zum Erzeugen einer zweiten Matrix, die die mit Mitteln (j) erhaltenen Werte der zweiten prädiktiven Variablen in der Testprobe enthält, und

(s) Mittel zum Bestimmen des Werts des Prozentsatzes der fetalen DNA in der Testprobe durch das Anlegen von Random Forest Regressionsbäumen unter Verwendung des Random Forest Referenzmodells zum Vorhersagen des Prozentsatzes der fetalen DNA, berechnet mit Mitteln (k), unter Verwendung der zweiten Matrix, die die Werte der zweiten Vorhersagevariablen in der Testprobe enthält, als Input in das Random Forest Referenzmodell und Erhalten eines Durchschnitts basierend auf den erhaltenen Bäume.

5. Eine Methode zum Bestimmen, ob ein Fetus mit fetaler DNA Gefahr läuft, Veränderungen in der Anzahl von mindestens einem Chromosom und/oder mindestens einem Chromosomenfragment aufzuweisen, wobei die Veränderungen in der Anzahl von Chromosomen vollständige chromosomale fetale Aneuploidien sind und die Veränderungen in der Anzahl von Chromosomenfragmentes partielle fetale Aneuploidien sind, in einer maternalen Plasmatestprobe, die maternale DNA und die fetale DNA enthält, wobei die Methode umfasst:

(a) Extrahieren von DNA aus der Testprobe und Reparieren der DNA,

(b) Synthetisieren von Bibliotheken der in der Testprobe vorhandenen DNA, Amplifizieren und Markieren der Bibliotheken durch PCR,

(c) Erhalten von Sequenzlesungen durch massive Sequenzierung der amplifizierten und markierten Bibliotheken,

(d) Alignieren der Sequenzlesungen mit einem Referenzgenom,

(e) Bestimmen, gemäß einem der Ansprüche 1 bis 3, den Prozentsatz der fetalen DNA in der Testprobe,

(f) Unterteilen der Sequenz jedes Chromosoms in dritte Bins mit einer Basengröße von 100 kbp bis 1 Mbp und Erhalten von Bin-Anzahlen durch Zählen der Anzahl der Sequenzlesungen in jeder dritten Bin in der Testprobe,

(g) Gewinnen von Bin-Anzahlen in jeder dritten Bin in zweiten Referenzproben, wobei die Bin-Anzahlen in den zweiten Referenzproben zuvor gemäß den Schritten (a)-(f) erhalten wurden und wobei die zweiten Referenzproben Referenzproben für maternales Plasma sind, die maternale DNA und fetale DNA aus Feten mit einem Satz von euploiden Chromosomen enthalten,

(h) gefolgt von folgenden Schritten:

(i) Bestimmung aller autosomalen dritten Bins als normale Bins in der Testprobe,

(ii) Erhalten von Deckungsnormalisierungskoeffizienten in der Testprobe und den zweiten Referenzproben durch Teilen der Bin-Anzahlen in jeder der normalen Bins durch den Mittelwert der Gesamtanzahl in der Testprobe und den zweiten Referenzproben für alle normalen Bins,

(iii) Erhalten normalisierter Bin-Anzahlen in der Testprobe und den zweiten Referenzproben durch Multiplizieren der Bin-Anzahlen aller Bins in der Testprobe und der zweiten Referenzproben mit den Deckungsnormalisierungskoeffizienten,

(iv) Berechnen eines GC-Gehaltsmodells für die Testprobe und eines GC-Gehaltsmodells für jede zweite Referenzprobe durch Regression, unter Verwendung des GC-Gehalts der normalen Bins als die unabhängige Variable und unter Verwendung der normalisierten Bin-Anzahlen als die abhängige Variable und Erhalten von vorhergesagten Bin-Anzahlen aller Bins in der Testprobe und den zweiten Referenzproben mit den GC-Gehaltsmodellen,

(v) Erhalten von Resten durch Berechnen von Differenzen zwischen den normalisierten Bin-Anzahlen aller dritten Bins und den vorhergesagten Bin-Anzahlen aller dritten Bins in der Testprobe und den zweiten Referenzproben,

(vi) Erhalten standardisierter Reste aller dritten Bins in der Testprobe durch Standardisieren der Reste aller dritten Bins in der Testprobe mittels eines Z-Scores, der für jede Bin erhalten wird, indem der Rest jeder dritten Bin in der Testprobe mit dem Rest der entsprechenden dritten Bin in den zweiten Referenzproben verglichen wird,

(vii) Erhalten erstskalierter standardisierter Reste in der Testprobe durch Multiplizieren der standardisierten Reste in der Testprobe mit einem ersten Skalierungskoeffizienten, wobei der erste Skalierungskoeffizient die Inverse des in Schritt (e) bestimmten Prozentsatzes an fetaler DNA ist,

(viii) Bestimmen von Bin-Kopienzahlen für jeden dritten Bin durch Multiplizieren der erstskalierten standardisierten Reste mit einem zweiten Skalierungskoeffizienten, wodurch zweitskalierte standardisierte Reste erhalten werden, und durch Addieren eines Verschiebungskoeffizienten zu den zweitskalierten standardisierten Resten, wobei der zweite Skalierungskoeffizient zuvor aus erstskalierten standardisierten Resten und Bin-Kopienzahlen für jeden dritten Bin in dritten Referenzproben bestimmt wurde, wobei die dritten Referenzproben maternale Plasma-Referenzproben sind, die maternale DNA und fetale DNA von Feten mit einem Satz aneuploider Chromosomen enthalten, wobei der Verschiebungskoeffizient zuvor aus erstskalierten standardisierten Resten und Bin-Kopienzahlen für jeden dritten Bin in den zweiten Referenzproben bestimmt wurde,

(ix) Beseitigen dritter Bins mit einer Kopienzahl, die sich zur Bestimmung der Aneuploidie von der Gruppe der normalen Bins in der Testprobe unterscheidet,

(x) Wiederholung der Schritte (h)(ii) bis (h)(ix),

(xi) Wiederholen von Schritt (h)(x), wenn sich die Deckungsnormalisierungskoeffizienten in Bezug auf die vorherigen Deckungsnormalisierungskoeffizienten um mehr als einen Wert im Bereich von 0,1 bis 5 % unterscheiden,

(i) Erhalten von Gruppen von aneinandergrenzenden Bins durch Segmentierung, wodurch Segmente mit der gleichen Kopienzahl erhalten werden, wobei, wenn ein gegebenes Segment eines autosomalen Chromosoms eine Kopienzahl von mehr als 2,5 oder weniger als 1,5 aufweist, der Fetus als gefährdet eingestuft wird, Veränderungen in der Anzahl von mindestens einem Chromosom und/oder mindestens einem Chromosomenfragment aufzuweisen.

6. Die Methode nach Anspruch 5, wobei in Schritt (h)(iv) die Regression eine LOESS Regression oder eine Polynomregression ist.

7. Die Methode nach Anspruch 5 oder 6, wobei in Schritt (h)(v) die Reste erhalten werden durch Subtrahieren der vorhergesagten Bin-Anzahlen aller dritten Bins von den normalisierten Bin-Anzahlen aller dritten Bins in der Testprobe und den Referenzproben.

8. Die Methode nach einem der Ansprüche 5 bis 7, wobei die Segmentierung eine kreisförmige binäre Segmentierung (CBS) oder Segmentierung auf der Grundlage eines versteckten Markov-Modells (HMM) ist.

9. Die Methode nach einem der Ansprüche 5 bis 8, wobei die kompletten chromosomalen fetalen Aneuploidien in einem Chromosom gefunden werden, das aus der Gruppe ausgewählt ist, die aus 9, 13, 15, 16, 18, 21, 22, X-Chromosomen und Y-Chromosomen besteht.

10. Die Methode nach einem der Ansprüche 5 bis 9, wobei die partiellen fetalen Aneuploidien mit den Syndromen assoziiert sind, die aus der Gruppe ausgewählt sind, die aus dem Angelman-Syndrom, dem Prader-Willi-Syndrom, dem Katzenschrei-Syndrom, dem Wolf-Hirschhorn-Syndrom, dem Jacobsen-Syndrom, dem Langer-Giedion-Syndrom, dem DiGeorge-Syndrom, dem DiGeorge II-Syndrom und dem Phelan-McDermid-Syndrom besteht.

11. Die Methode nach einem der Ansprüche 5 bis 10, wobei die partielle fetale Aneuploidie aus einer 1p36-Deletion oder einer 16p11.2-p12.1.2-Deletion besteht.

12. Die Methode nach einem der Ansprüche 5 bis 11, wobei der Wert in Schritt (h)(xi) in einem Bereich liegt, der aus der Gruppe ausgewählt ist, die aus 0,2 bis 4 %, 0,3 bis 3 %, 0,4 bis 2 %, 0,5 bis 1,5 % und 0,8 bis 1,2 % besteht.

13. Ein Computerprogramm-Produkt, umfassend ein computerlesbares Medium, worin Befehle kodiert sind, um ein Computersystem, umfassend Mittel zum Bestimmen, ob ein Fetus mit fetaler DNA Gefahr läuft, Änderungen in der Anzahl von mindestens einem Chromosom und/oder mindestens einem Chromosomenfragment zu haben, gemäß der Methode nach einem der Ansprüche 5 bis 12 zu steuern, wobei die Änderungen in der Anzahl von Chromosomen vollständige chromosomale fetale Aneuploidien sind und die Änderungen in der Anzahl von Chromosomenfragmenten partielle fetale Aneuploidien sind, in einer maternalen Plasmatestprobe, die maternale DNA und die fetale DNA enthält, wobei das Computersystem die folgenden Mittel umfasst:

a) Mittel zur Aufzeichnung von DNA-Sequenzmesswerten, die aus der Testprobe gewonnen wurden,
b) Mittel zum Alignieren der Sequenzlesungen mit einen Referenzgenom,
c) Mittel zur Bestimmung des Prozentsatzes der fetalen DNA in der Testprobe mit dem Computerprogramm-Produkt nach Anspruch 6,
d) Mittel zum Aufteilen der Sequenz jedes Chromosoms in dritte Bins mit einer Basengröße von 100 kbp bis 1 Mbp und zum Erhalten von Bin-Anzahlen durch Zählen der Anzahl der Sequenzlesungen in jeder dritten Bin in der Testprobe,
e) Mittel zum Wiedergewinnen von Bin-Anzahlen in jeder dritten Bin in zweiten Referenzproben, wobei die Bin-Anzahlen in den zweiten Referenzproben zuvor mit Mitteln (a)-(d) erhalten wurden und wobei die zweiten Referenzproben Referenzproben für maternales Plasma sind, die maternale DNA und fetale DNA aus Feten mit einem Satz von euploiden Chromosomen enthalten,
f) Mittel zur Durchführung folgender Vorgänge:

i) Mittel zur Bestimmung aller autosomalen dritten Bins als normale Bins in der Testprobe,
(ii) Mittel zum Erhalten von Deckungsnormalisierungskoeffizienten in der Testprobe und den zweiten Referenzproben durch Teilen der Bin-Anzahlen in jeder der normalen Bin durch den Mittelwert der Gesamtzahl in der Testprobe und den Referenzproben für alle normalen Bins,
(iii) Mittel zum Erhalten normalisierter Bin-Anzahlen in der Testprobe und den zweiten Referenzproben durch Multiplizieren der Bin-Anzahlen aller Bins in der Testprobe und den Referenzproben mit den Deckungsnormalisierungskoeffizienten,
(iv) Mittel zum Berechnen eines GC-Gehaltsmodells für die Testprobe und eines GC-Gehaltsmodells für jede zweite Referenzprobe durch Regression, unter Verwendung des GC-Gehalts der normalen Bins als die unabhängige Variable und unter Verwendung der normalisierten Bin-Anzahlen als die abhängige Variable und Erhalten von vorhergesagten Bin-Anzahlen aller Bins in der Testprobe und den zweiten Referenzproben mit den GC-Gehaltsmodellen,
(v) Mittel zum Erhalten von Resten durch Berechnen von Differenzen zwischen den normalisierten Bin-Anzahlen aller dritten Bins und den vorhergesagten Bin-Anzahlen aller dritten Bins in der Testprobe und den zweiten Referenzproben,
(vi) Mittel zum Erhalten standardisierter Reste aller dritten Bins in der Testprobe durch Standardisieren der Reste aller dritten Bins in der Testprobe mittels eines Z-Scores, der für jeden Bin erhalten wird, durch Vergleichen des Restes jeder dritten Bin in der Testprobe mit dem Rest der entsprechenden dritten Bin in den zweiten Referenzproben,
(vii) Mittel zum Erhalten von zuerst skalierten standardisierten Resten in der Testprobe durch Multiplizieren der standardisierten Reste in der Testprobe mit einem ersten Skalierungskoeffizienten, wobei der erste Skalierungskoeffizient die Inverse des Prozentsatzes an fetaler DNA ist, der mit Mitteln (c) bestimmt wurde,
(viii) Mittel zum Bestimmen von Bin-Kopienzahlen für jeden dritten Bin durch Multiplizieren der erstskalierten standardisierten Reste mit einem zweiten Skalierungskoeffizienten, wodurch zweitskalierte standardisierte Reste erhalten werden, und durch Addieren eines Verschiebungskoeffizienten zu den zweitskalierten standardisierten Resten, wobei der zweite Skalierungskoeffizient zuvor aus erstskalierten standardisierten Resten und Bin-Kopienzahlen für jeden dritten Bin in dritten Referenzproben bestimmt wurde, wobei die dritten Referenzproben maternale Plasma-Referenzproben sind, die maternale DNA und fetale DNA von Feten mit einem Satz aneuploider Chromosomen enthalten, wobei der Verschiebungskoeffizient zuvor aus erstskalierten standardisierten Resten und Bin-Kopienzahlen für jeden dritten Bin in den zweiten Referenzproben bestimmt wurde,
(ix) Mittel zum Beseitigen dritter Bins mit einer Kopienzahl, die sich zur Bestimmung der Aneuploidie von der Gruppe der normalen Bins in der Testprobe unterscheidet,
(x) Mittel zur Wiederholung der Operationen (f)(ii) bis (f)(ix);
(xi) Mittel zum Wiederholen der Operation (f)(x), wenn sich die Deckungsnormalisierungskoeffizienten in Bezug auf die vorherigen Deckungsnormalisierungskoeffizienten um mehr als einen Wert im Bereich von 0,1 bis 5 % unterscheiden,

g) Mittel zum Erhalten von Gruppen von aneinandergrenzenden Bins durch Segmentierung, wodurch Segmente mit der gleichen Kopienzahl erhalten werden, wobei, wenn ein gegebenes Segment eines autosomalen Chromosoms eine Kopienzahl von mehr als 2,5 oder weniger als 1,5 aufweist, der Fetus als gefährdet eingestuft wird, Veränderungen in der Anzahl von mindestens einem Chromosom und/oder mindestens einem Chromosomenfragment aufzuweisen.

14. Eine Methode zur Bestimmung der Abwesenheit oder Anwesenheit von Y-Chromosom in einer maternalen Plasmaprobe, die maternale DNA und fetale DNA enthält, umfassend die folgenden Schritte:

(a) Extrahieren von DNA aus der Testprobe und Reparieren der DNA,
(b) Synthetisieren von Bibliotheken der in der Testprobe vorhandenen DNA, Amplifizieren und Markieren der Bibliotheken durch PCR und Kombinieren der Bibliotheken,
(c) Erhalten von Sequenzlesungen des Y-Chromosoms durch massive Sequenzierung der amplifizierten, markierten und kombinierten Bibliotheken,
(d) Alignieren der Sequenzlesungen des Y-Chromosoms mit einer Sequenz des Y-Chromosoms eines Referenzgenoms,
(e) Unterteilen der Sequenz des Y-Chromosoms in vierte Bins mit einer Basengröße von 100 kbp bis 1 Mbp und Erhalten von Bin-Anzahlen durch Zählen der Anzahl der Sequenzlesungen in jedem vierten Bin des Chromosoms in der Testprobe,
(f) Gewinnen von Bin-Anzahlen in jedem vierten Bin in zweiten Referenzproben, wobei die Bin-Anzahlen in den zweiten Referenzproben zuvor gemäß der Schritte (a)-(e) erhalten wurden und wobei die zweiten Referenzproben männliche Fetusreferenzproben und weibliche Fetusreferenzproben umfassen,
(g) Erhalten normalisierter Bin-Anzahlen in der Testprobe und den zweiten Referenzproben durch Normalisieren der Bin-Anzahlen aller Bins basierend auf dem Mittelwert der Gesamtanzahl der Testprobe und der zweiten Referenzproben für alle normalen Bins in der Testprobe, wobei die normalen Bins die normalen Bins sind, die in Schritt (h)(xi) der Methode nach Anspruch 5 bestimmt werden,
(h) Bestimmen von Bins der Sequenz des Y-Chromosoms, die sich statistisch durch einen ersten statistischen Wilcoxon-Hypothesentest unterscheiden, Vergleichen der normalisierten Bin-Anzahlen in den weiblichen Fetusreferenzproben mit normalisierten Bin-Anzahlen in den männlichen Fetusreferenzproben, wobei die Nullhypothese des ersten Wilcoxon-Tests darin besteht, dass der Mittelwert der normalisierten Bin-Anzahlen in jedem vierten Bin in den männlichen Fetusreferenzproben signifikant größer ist, als der Mittelwert der normalisierten Bin-Anzahlen in jedem vierten Bin in den weiblichen Fetusreferenzproben, und wobei Bins statistisch unterschiedlich bestimmt werden, wenn die Nullhypothese des ersten Wilcoxon-Tests als wahr verifiziert wird,
(i) für die Bins der Sequenz des Y-Chromosoms, die statistisch unterschiedlich sind, Vergleichen der normalisierten Bin-Anzahlen in der Testprobe mit den normalisierten Anzahlen in den weiblichen Fetusreferenzproben durch einen zweiten Wilcoxon-Test, wobei die Nullhypothese des zweiten Wilcoxon-Tests darin besteht, dass der Mittelwert der normalisierten Bin-Anzahlen in jedem Bin, der statistisch unterschiedlich in der Testprobe ist, signifikant größer ist als der Mittelwert der normalisierten Bin-Anzahlen in jedem Bin, der statistisch unterschiedlich in den weiblichen Fetusreferenzproben ist,
(j) für die Bins der Sequenz des Y-Chromosoms, die statistisch unterschiedlich sind, Vergleichen der normalisierten Bin-Anzahlen in der Testprobe mit den normalisierten Anzahlen in den männlichen Fetusreferenzproben durch einen dritten Wilcoxon-Test, wobei die Nullhypothese des dritten Wilcoxon-Tests darin besteht, dass der Mittelwert der normalisierten Bin-Anzahlen in jedem Bin, der statistisch unterschiedlich in der Testprobe ist, signifikant geringer ist als der Mittelwert der normalisierten Bin-Anzahlen in jedem Bin, der statistisch unterschiedlich in den männlichen Fetusreferenzproben ist,
(k) Bestimmen von männlichen Bins, wenn sowohl die Nullhypothesen des zweiten als auch des dritten Wilcoxon-Tests als wahr verifiziert werden, und Bestimmen von weiblichen Bins, wenn mindestens eine der Nullhypothesen des zweiten und des dritten Wilcoxon-Tests nicht als wahr verifiziert wird,
(l) Bestimmen der Anzahl der männlichen und weiblichen Bins und Bestimmen des Vorhandenseins des Y-Chromosoms, wobei die Anzahl der männlichen Bins höher als die Anzahl der weiblichen Bins ist, und anderweitiges Bestimmen der Abwesenheit des Y-Chromosoms, wobei das Vorhandensein oder die Abwesenheit des Y-Chromosoms das fetale Geschlecht anzeigt.

15. Ein Computerprogramm-Produkt, umfassend ein computerlesbares Medium, worin Anweisungen kodiert sind, um ein Computersystem, umfassend Mittel zum Bestimmen der Abwesenheit oder Anwesenheit von Y-Chromosom in einer maternalen Plasmaprobe, die maternale DNA und fetale DNA enthält, gemäß der Methode nach Anspruch 14 zu steuern, wobei das Computersystem die folgenden Mittel umfasst:

(a) Mittel zum Aufzeichnen von DNA-Sequenzmesswerten, die aus der Testprobe gewonnen wurden,
(b) Mittel zum Alignieren der Sequenzlesungen des Y-Chromosoms gegen eine Sequenz des Y-Chromosoms eines Referenzgenoms,

(c) Mittel zum Aufteilen der Sequenz des Y-Chromosoms in vierte Bins mit einer Basengröße von 100 kbp bis 1 Mbp und Erhalten von Bin-Anzahlen durch Zählen der Anzahl der Sequenzlesungen in jedem vierten Bin des Chromosoms in der Testprobe,

(d) Mittel zum Wiedergewinnen von Bin-Anzahlen in jedem vierten Bin in zweiten Referenzproben, wobei die Bin-Anzahlen in den zweiten Referenzproben zuvor erhalten wurden und wobei die zweiten Referenzproben männliche Fetusreferenzproben und weibliche Fetusreferenzproben umfassen,

(e) Mittel zum Erhalten normalisierter Bin-Anzahlen in der Testprobe und den zweiten Referenzproben durch Normalisieren der Bin-Anzahlen aller Bins basierend auf dem Mittelwert der Gesamtanzahl der Testprobe und der zweiten Referenzproben für alle normalen Bins in der Testprobe, wobei die normalen Bins die normalen Bins sind, die mit der Operation (f)(xi) des Computerprogramm-Produkts nach Anspruch 13 bestimmt werden,

(f) Mittel zum Bestimmen von Bins der Sequenz des Y-Chromosoms, die sich statistisch durch einen ersten statistischen Wilcoxon-Hypothesentest unterscheiden, Vergleichen der normalisierten Bin-Anzahlen in den weiblichen Fetusreferenzproben mit normalisierten Bin-Anzahlen in den männlichen Fetusreferenzproben, wobei die Nullhypothese des ersten Wilcoxon-Tests darin besteht, dass der Mittelwert der normalisierten Bin-Anzahlen in jeder vierten Bin in den männlichen Fetusreferenzproben signifikant größer ist als der Mittelwert der normalisierten Bin-Anzahlen in jedem vierten Bin in den weiblichen Fetusreferenzproben, und wobei Bins statistisch unterschiedlich bestimmt werden, wenn die Nullhypothese des ersten Wilcoxon-Tests als wahr verifiziert wird,

(g) Mittel zum Vergleichen der normalisierten Bin-Anzahlen in der Testprobe mit den normalisierten Anzahlen in den weiblichen Fetusreferenzproben durch einen zweiten Wilcoxon-Test für die Bins der Sequenz des Y-Chromosoms, die statistisch unterschiedlich sind, wobei die Nullhypothese des zweiten Wilcoxon-Tests darin besteht, dass der Mittelwert der normalisierten Bin-Anzahlen in jedem Bin, der statistisch unterschiedlich in der Testprobe ist, signifikant größer ist, als der Mittelwert der normalisierten Bin-Anzahlen in jedem Bin, der statistisch unterschiedlich in den weiblichen Fetusreferenzproben ist,

(h) Mittel zum Vergleichen der normalisierten Bin-Anzahlen in der Testprobe mit den normalisierten Anzahlen in den männlichen Fetusreferenzproben durch einen dritten Wilcoxon-Test für die Bins der Sequenz des Y-Chromosoms, die statistisch unterschiedlich sind, wobei die Nullhypothese des dritten Wilcoxon-Tests darin besteht, dass der Mittelwert der normalisierten Bin-Anzahlen in jedem Bin, der statistisch unterschiedlich in der Testprobe ist, signifikant geringer ist, als der Mittelwert der normalisierten Bin-Anzahlen in jedem Bin, der statistisch unterschiedlich in den männlichen Fetusreferenzproben ist,

(i) Mittel zum Bestimmen von männlichen Bins, wenn sowohl die Nullhypothesen des zweiten als auch des dritten Wilcoxon-Tests als wahr verifiziert werden, und zum Bestimmen von weiblichen Bins, wenn mindestens eine der Nullhypothesen des zweiten und des dritten Wilcoxon-Tests nicht als wahr verifiziert wird,

(j) Mittel zum Bestimmen der Anzahl der männlichen und weiblichen Bins und zum Bestimmen des Vorhandenseins des Y-Chromosoms, wobei die Anzahl der männlichen Bins höher ist als die Anzahl der weiblichen Bins und das Fehlen des Y-Chromosoms anderweitig bestimmt wird, wobei das Vorhandensein oder Fehlen des Y-Chromosoms das fetale Geschlecht anzeigt.

## Revendications

1. Procédé pour déterminer le pourcentage d'ADN fœtal dans un échantillon d'essai de plasma maternel contenant de l'ADN maternel et de l'ADN fœtal, comprenant :

(a) le calcul d'un modèle de régression de forêt aléatoire de référence pour prédire 5 % d'ADN fœtal, comprenant les étapes consistant à :

(i) extraire l'ADN présent dans les échantillons de référence et réparer ledit ADN, lesdits échantillons de référence étant des échantillons de référence de plasma maternel contenant de l'ADN maternel et de l'ADN fœtal provenant de fœtus ayant un ensemble de chromosomes euploïdes et le pourcentage d'ADN fœtal dans lesdits échantillons de référence ayant été préalablement déterminé,

(ii) synthétiser des banques de l'ADN présent dans lesdits échantillons de référence, amplifier et étiqueter lesdites banques par PCR,

(iii) obtenir des lectures de séquences, par séquençage massif, desdites banques amplifiées et étiquetées,

(iv) aligner lesdites lectures de séquences par rapport à un génome de référence,

(v) diviser la séquence de chaque chromosome en des premiers bacs ayant une taille de base de 50 à 200 Kb, avec un premier bac coulissant ayant une taille de base de 5 à 15 Kb, et obtenir des comptes de bacs en comptant le nombre de lectures de séquences dans chacun desdits premiers bacs, chaque taille de base de premier bac étant la même, chaque taille de base de premier bac coulissant étant la même et chaque taille de base de premier bac coulissant étant inférieure ou égale à la taille de base de premier bac,

(vi) obtenir des comptes de bacs normalisés en normalisant lesdits comptes de bacs obtenus à l'étape (a)(v) sur la base des comptes de bacs totaux de tous les premiers bacs dans lesdits échantillons de référence,

(vii) calculer une régression pour chaque premier bac en utilisant l'ensemble desdits échantillons de référence, en utilisant le compte de bacs normalisé comme variable indépendante, en utilisant le pourcentage d'ADN fœtal desdits échantillons de référence comme variable dépendante, et en utilisant la semaine de gestation, la taille de la mère, le poids de la mère et l'absorption d'héparine par la mère comme covariables,

(viii) identifier des régions génomiques enrichies en ADN fœtal, lesdites régions génomiques enrichies en ADN fœtal étant celles pour lesquelles la signification statistique dans la régression de l'étape précédente, telle que déterminée par la valeur p, est supérieure à $10^{'10}$ et le coefficient de corrélation de Spearman entre le pourcentage d'ADN fœtal prédit par ladite régression et le pourcentage d'ADN fœtal précédemment déterminé est supérieur ou égal à 0,5 et lesdites régions n'étant pas des régions du chromosome X,

(ix) obtenir une densité de comptes de bacs normalisés de lectures pour chacun desdits échantillons de référence qui correspondent auxdites régions génomiques enrichies en ADN fœtal par rapport à la taille d'insert des lectures pour chacun desdits échantillons de référence qui correspondent auxdites régions génomiques enrichies en ADN fœtal et obtenir une courbe de densité à partir desdites valeurs obtenues, ladite courbe de densité étant un profil nucléosomique de référence,

(x) diviser ledit profil nucléosomique de référence en des deuxièmes bacs de tailles d'insert ayant une taille de base de 2 à 100 nucléotides, avec un deuxième bac coulissant ayant une taille de base de 1 à 20 nucléotides, chaque taille de base de deuxième bac étant la même, chaque taille de base de deuxième bac coulissant étant la même et chaque taille de base de deuxième bac coulissant étant inférieure ou égale à la taille de base de deuxième bac, et calculer la valeur des premières variables choisies dans le groupe constitué de moyenne, minimum, maximum et de la zone accumulée sous la courbe pour chaque deuxième bac,

(xi) déterminer l'importance variable desdites premières variables dans lesdits échantillons de référence par une classification de forêt aléatoire et déterminer un ensemble de premières variables prédictives dans lesdits échantillons de référence, lesdites variables prédictives dans ledit ensemble étant les n variables les plus importantes telles que classées par ladite importance variable, où n est compris entre 100 et 300,

(xii) générer une première matrice contenant les valeurs dans lesdits échantillons de référence de secondes variables prédictives, lesdites secondes variables prédictives comprenant lesdites premières variables prédictives et comprenant en outre la semaine de gestation, la taille de la mère, le poids de la mère et l'absorption d'héparine par la mère,

(xiii) calculer un modèle de régression de forêt aléatoire de référence pour prédire le pourcentage d'ADN fœtal en faisant croître des arbres de régression de forêt aléatoire pour lesdits échantillons de référence en utilisant une partie desdits échantillons de référence comme ensemble d'apprentissage et une partie desdits échantillons de référence comme ensemble d'essai et en utilisant ladite première matrice contenant les valeurs desdites secondes variables prédictives comme entrée dans ledit modèle de régression de forêt aléatoire de référence, et en obtenant une moyenne basée sur les arbres obtenus ; et

(b) déterminer le pourcentage d'ADN fœtal dans ledit échantillon d'essai, comprenant les étapes consistant à :

(i) extraire l'ADN dudit échantillon d'essai et réparer ledit ADN,

(ii) synthétiser des banques de l'ADN présent dans ledit échantillon d'essai, amplifier et étiqueter lesdites banques par PCR,

(iii) obtenir des lectures de séquences, par séquençage massif, desdites banques amplifiées et étiquetées,

(iv) aligner lesdites lectures de séquences par rapport à un génome de référence,

(v) diviser la séquence de chaque chromosome en des premiers bacs selon l'étape (a)(v) ayant une taille de base de 50 à 200 Kb, avec un premier bac coulissant selon l'étape (a)(v) ayant une taille de base de 5 à 15 Kb, et obtenir des comptes de bacs en comptant le nombre de lectures de séquences dans chaque premier bac dans ledit échantillon d'essai,

(vi) obtenir des comptes de bacs normalisés en normalisant lesdits comptes de bacs obtenus à l'étape (b)(v) sur la base des comptes de bacs totaux de tous les premiers bacs dans ledit échantillon d'essai,

(vii) obtenir une densité de comptes de bacs normalisés de lectures pour ledit échantillon d'essai qui correspondent auxdites régions génomiques enrichies en ADN fœtal par rapport à la taille d'insert des lectures dans ledit échantillon d'essai qui correspondent auxdites régions génomiques enrichies en ADN fœtal et obtenir une courbe de densité à partir desdites valeurs obtenues, ladite courbe de densité étant un profil nucléosomique d'essai,

(viii) diviser ledit profil nucléosomique d'essai en des deuxièmes bacs selon l'étape (a)(x) ayant une taille de base de 2 à 100 nucléotides, avec un deuxième bac coulissant selon l'étape (a)(x) ayant une taille de base de 1 à 20 nucléotides, et calculer la valeur desdites premières variables choisies dans le groupe constitué de moyenne, minimum, maximum et de la zone accumulée sous la courbe pour chacun desdits deuxièmes bacs,

(ix) générer une seconde matrice contenant les valeurs dans ledit échantillon d'essai desdites secondes variables prédictives obtenues à l'étape (a)(xii), et

(x) déterminer la valeur du pourcentage d'ADN fœtal dans l'échantillon d'essai en faisant croître des arbres de régression de forêt aléatoire en utilisant ledit modèle de forêt aléatoire de référence pour prédire le pourcentage d'ADN fœtal calculé à l'étape (a)(xiii), en utilisant ladite seconde matrice contenant les valeurs dans ledit échantillon d'essai de secondes variables prédictives comme entrée dans ledit modèle de forêt aléatoire de référence et en obtenant des valeurs estimées du pourcentage d'ADN fœtal pour chaque arbre dans l'échantillon d'essai, la moyenne desdites valeurs estimées étant le pourcentage d'ADN fœtal dans ledit échantillon d'essai.

2. Procédé selon la revendication 1, dans lequel ladite régression est choisie dans le groupe constitué d'une régression linéaire, d'une régression exponentielle et d'une régression logarithmique.

3. Procédé selon la revendication 1 ou 2, dans lequel n se situe dans une plage choisie dans le groupe constitué de 120 à 280, 140 à 260, 160 à 240 et 180 à 220.

4. Produit de programme informatique comprenant un support lisible par ordinateur, dans lequel des instructions sont codées pour commander un système informatique comprenant des moyens pour déterminer le pourcentage d'ADN fœtal dans un échantillon d'essai de plasma maternel contenant de l'ADN maternel et de l'ADN fœtal selon le procédé de l'une quelconque des revendications 1 à 3, ledit système informatique comprenant les moyens suivants pour calculer un modèle de régression de forêt aléatoire de référence pour prédire le pourcentage d'ADN foetal :

(a) des moyens pour enregistrer des lectures de séquences d'ADN obtenues à partir d'échantillons de référence, lesdits échantillons de référence étant des échantillons de référence de plasma maternel contenant de l'ADN maternel et de l'ADN fœtal provenant de fœtus ayant un ensemble de chromosomes euploïdes et le pourcentage d'ADN fœtal dans lesdits échantillons de référence ayant été préalablement déterminé,

(b) des moyens pour aligner lesdites lectures de séquences par rapport à un génome de référence,

(c) des moyens pour diviser la séquence de chaque chromosome en des premiers bacs ayant une taille de base de 50 à 200 Kb, avec un premier bac coulissant ayant une taille de base de 5 à 15 Kb, et obtenir des comptes de bacs en comptant le nombre de lectures de séquences dans chacun desdits premiers bacs, chaque taille de base de premier bac étant la même, chaque taille de base de premier bac coulissant étant la même et chaque taille de base de premier bac coulissant étant inférieure ou égale à la taille de base de premier bac,

(d) des moyens pour obtenir des comptes de bacs normalisés en normalisant lesdits comptes de bacs sur la base des comptes de bacs totaux de tous les premiers bacs dans lesdits échantillons de référence,

(e) des moyens pour calculer une régression pour chaque premier bac dans lesdits échantillons de référence en utilisant le compte de bacs normalisé comme variable indépendante, en utilisant le pourcentage d'ADN fœtal desdits échantillons de référence comme variable dépendante, et en utilisant la semaine de gestation, la taille de la mère, le poids de la mère et l'absorption d'héparine par la mère comme covariables,

(f) des moyens pour identifier des régions génomiques enrichies en ADN fœtal, lesdites régions génomiques enrichies en ADN fœtal étant celles pour lesquelles la signification statistique dans la régression précédente, telle que déterminée par la valeur p, est supérieure à $10^{'10}$ et le coefficient de corrélation de Spearman entre le pourcentage d'ADN fœtal prédit par ladite régression et le pourcentage d'ADN fœtal précédemment déterminé est supérieur ou égal à 0,5 et lesdites régions n'étant pas des régions du chromosome X,

(g) des moyens pour obtenir une densité de comptes de bacs normalisés de lectures pour chacun desdits échantillons de référence qui correspondent auxdites régions génomiques enrichies en ADN fœtal par rapport à la taille d'insert des lectures pour chacun desdits échantillons de référence qui correspondent auxdites régions génomiques enrichies en ADN fœtal et obtenir une courbe de densité à partir desdites valeurs obtenues, ladite courbe de densité étant un profil nucléosomique de référence,

(h) des moyens pour diviser ledit profil nucléosomique de référence en des deuxièmes bacs de tailles d'insert ayant une taille de base de 2 à 100 nucléotides, avec un deuxième bac coulissant ayant une taille de base de 1 à 20 nucléotides, chaque taille de base de deuxième bac étant la même, chaque taille de base de deuxième bac coulissant étant la même et chaque taille de base de deuxième bac coulissant étant inférieure ou égale à la taille de base de deuxième bac, et calculer la valeur des premières variables choisies dans le groupe constitué de moyenne, minimum, maximum et de la zone accumulée sous la courbe pour chaque deuxième bac,

(i) des moyens pour déterminer l'importance variable desdites premières variables dans lesdits échantillons de référence par une classification de forêt aléatoire et déterminer un ensemble de premières variables prédictives dans lesdits échantillons de référence, lesdites variables prédictives dans ledit ensemble étant les n variables les plus importantes telles que classées par ladite importance variable, où n est compris entre 100 et 300,

(j) des moyens pour générer une première matrice contenant les valeurs dans lesdits échantillons de référence de secondes variables prédictives, lesdites secondes variables prédictives comprenant lesdites premières variables prédictives et comprenant en outre la semaine de gestation, la taille de la mère, le poids de la mère et l'absorption d'héparine par la mère,

(k) des moyens pour calculer un modèle de régression de forêt aléatoire de référence pour prédire le pourcentage d'ADN fœtal en faisant croître des arbres de régression de forêt aléatoire pour lesdits échantillons de référence en utilisant une partie desdits échantillons de référence comme ensemble d'apprentissage et une partie desdits échantillons de référence comme ensemble d'essai et en utilisant ladite première matrice contenant les valeurs desdites secondes variables prédictives comme entrée dans ledit modèle de régression de forêt aléatoire de référence, et en obtenant une moyenne basée sur les arbres obtenus ; et,

ledit système informatique comprenant en outre les moyens suivants pour déterminer le pourcentage d'ADN fœtal dans ledit échantillon d'essai :

(l) des moyens pour enregistrer des lectures de séquences d'ADN obtenues à partir dudit échantillon d'essai,

(m) des moyens pour aligner lesdites lectures de séquences par rapport à un génome de référence,

(n) des moyens pour diviser la séquence de chaque chromosome en des premiers bacs ayant une taille de base de 50 à 200 Kb, avec un premier bac coulissant ayant une taille de base de 5 à 15 Kb, et obtenir des comptes de bacs en comptant le nombre de lectures de séquences dans chaque bac dans ledit échantillon d'essai,

(o) des moyens pour obtenir des comptes de bacs normalisés en normalisant lesdits comptes de bacs sur la base des comptes de bacs totaux de tous les premiers bacs dans ledit échantillon d'essai,

(p) des moyens pour obtenir une densité de comptes de bacs normalisés de lectures pour ledit échantillon d'essai qui correspondent auxdites régions génomiques enrichies en ADN fœtal par rapport à la taille d'insert des lectures dans ledit échantillon d'essai qui correspondent auxdites régions génomiques enrichies en ADN fœtal et obtenir une courbe de densité à partir desdites valeurs obtenues, ladite courbe de densité étant un profil nucléosomique d'essai,

(q) des moyens pour diviser ledit profil nucléosomique d'essai en des deuxièmes bacs ayant une taille de base de 2 à 100 nucléotides, avec un deuxième bac coulissant ayant une taille de base de 1 à 20 nucléotides, et calculer la valeur desdites premières variables choisies dans le groupe constitué de moyenne, minimum, maximum et de la zone accumulée sous la courbe pour chacun desdits deuxièmes bacs,

(r) des moyens pour générer une seconde matrice contenant les valeurs dans ledit échantillon d'essai desdites secondes variables prédictives obtenues avec les moyens (j), et

(s) des moyens pour déterminer la valeur du pourcentage d'ADN foetal dans l'échantillon d'essai en faisant croître des arbres de régression de forêt aléatoire en utilisant ledit modèle de forêt aléatoire de référence pour prédire le pourcentage d'ADN fœtal calculé avec les moyens (k), en utilisant ladite seconde matrice contenant les valeurs dans ledit échantillon d'essai de secondes variables prédictives comme entrée dans ledit modèle de forêt aléatoire de référence et en obtenant une moyenne basée sur les arbres obtenus.

5. Procédé pour déterminer si un fœtus ayant de l'ADN fœtal risque d'avoir des altérations du nombre d'au moins un chromosome et/ou d'au moins un fragment de chromosome, lesdites altérations du nombre de chromosomes étant des aneuploïdies fœtales chromosomiques complètes et lesdites altérations du nombre de fragments de chromosomes étant des aneuploïdies fœtales partielles, dans un échantillon d'essai de plasma maternel contenant de l'ADN maternel et ledit ADN fœtal, ledit procédé comprenant les étapes consistant à :

(a) extraire l'ADN dudit échantillon d'essai et réparer ledit ADN,

(b) synthétiser des banques de l'ADN présent dans ledit échantillon d'essai, amplifier et étiqueter lesdites banques par PCR,

(c) obtenir des lectures de séquences, par séquençage massif, desdites banques amplifiées et étiquetées,

(d) aligner lesdites lectures de séquences par rapport à un génome de référence,

(e) déterminer, selon l'une quelconque des revendications 1 à 3, le pourcentage d'ADN fœtal dans ledit échantillon d'essai,

(f) diviser la séquence de chaque chromosome en des troisièmes bacs ayant une taille de base de 100 Kb à 1 Mb et obtenir des comptes de bacs en comptant le nombre de lectures de séquences dans chaque troisième bac dans ledit échantillon d'essai,

(g) récupérer les comptes de bacs dans chaque troisième bac dans des seconds échantillons de référence, lesdits comptes de bacs dans lesdits seconds échantillons de référence ayant été préalablement obtenus selon les étapes (a)-(f) et lesdits seconds échantillons de référence étant des échantillons de référence de plasma maternel contenant de l'ADN maternel et de l'ADN fœtal provenant de fœtus ayant un ensemble de chromosomes euploïdes,

(h) suivies des étapes suivantes :

(i) déterminer tous les troisièmes bacs autosomiques en tant que bacs normaux dans ledit échantillon d'essai,

(ii) obtenir des coefficients de normalisation de couverture dans ledit échantillon d'essai et lesdits seconds échantillons de référence en divisant les comptes de bacs dans chacun desdits bacs normaux via les comptes totaux dans ledit échantillon d'essai et lesdits seconds échantillons de référence pour tous lesdits bacs normaux,

(iii) obtenir des comptes de bacs normalisés dans ledit échantillon d'essai et lesdits seconds échantillons de référence en multipliant lesdits comptes de bacs de tous les bacs dans ledit échantillon d'essai et lesdits seconds échantillons de référence par lesdits coefficients de normalisation de couverture,

(iv) calculer un modèle de contenu GC pour ledit échantillon d'essai et un modèle de contenu GC pour chacun desdits seconds échantillons de référence par régression, en utilisant le contenu GC desdits bacs normaux comme variable indépendante et en utilisant lesdits comptes de bacs normalisés comme variable dépendante et en obtenant des comptes de bacs prédits de tous les bacs dans ledit échantillon d'essai et lesdits seconds échantillons de référence avec lesdits modèles de contenu GC,

(v) obtenir des résidus en calculant les différences entre les comptes de bacs normalisés de tous les troisièmes bacs et lesdits comptes de bacs prédits de tous les troisièmes bacs dans ledit échantillon d'essai et lesdits seconds échantillons de référence,

(vi) obtenir des résidus standardisés de tous les troisièmes bacs dans ledit échantillon d'essai en standardisant lesdits résidus de tous les troisièmes bacs dans ledit échantillon d'essai au moyen d'un score Z obtenu pour chaque bac en comparant ledit résidu de chaque troisième bac dans ledit échantillon d'essai audit résidu du troisième bac correspondant dans lesdits seconds échantillons de référence,

(vii) obtenir des résidus standardisés mis à l'échelle en premier dans ledit échantillon d'essai en multipliant lesdits résidus standardisés dans ledit échantillon d'essai par un premier coefficient de mise à l'échelle, ledit premier coefficient de mise à l'échelle étant l'inverse dudit pourcentage d'ADN fœtal déterminé à l'étape (e),

(viii) déterminer des nombres de copies de bac pour chaque troisième bac en multipliant lesdits résidus standardisés mis à l'échelle en premier par un second coefficient de mise à l'échelle, obtenant ainsi des résidus standardisés mis à l'échelle en second, et en ajoutant un coefficient de décalage auxdits résidus standardisés mis à l'échelle en second, ledit second coefficient de mise à l'échelle ayant été préalablement déterminé à partir des résidus standardisés mis à l'échelle en premier et des nombres de copies de bac pour chaque troisième bac dans des troisièmes échantillons de référence, lesdits troisièmes échantillons de référence étant des échantillons de référence de plasma maternel contenant de l'ADN maternel et de l'ADN fœtal provenant de fœtus ayant un ensemble de chromosomes aneuploïdes, ledit coefficient de décalage ayant été préalablement déterminé à partir des résidus standardisés mis à l'échelle en premier et des nombres de copies de bac pour chaque troisième bac dans lesdits seconds échantillons de référence,

(ix) éliminer les troisièmes bacs ayant un nombre de copies différent déterminant l'aneuploïdie du groupe desdits bacs normaux dans ledit échantillon d'essai,

(x) répéter les étapes (h)(ii) à (h)(ix),

(xi) répéter l'étape (h)(x) si lesdits coefficients de normalisation de couverture diffèrent de plus d'une valeur comprise entre 0,1 et 5 % par rapport auxdits coefficients de normalisation de couverture précédents,

(i) obtenir des groupes de bacs contigus par segmentation, obtenant ainsi des segments ayant le même nombre de copies, de sorte que lorsqu'un segment donné d'un chromosome autosomique a un nombre de copies supérieur à 2,5 ou inférieur à 1,5, ledit fœtus est classé comme présentant un risque d'altération du nombre d'au moins un chromosome et/ou d'au moins un fragment de chromosome.

6. Procédé selon la revendication 5, dans lequel, dans l'étape (h)(iv), ladite régression est une régression LOESS ou une régression polynomiale.

7. Procédé selon la revendication 5 ou 6, dans lequel, dans l'étape (h)(v), lesdits résidus sont obtenus en soustrayant lesdits comptes de bacs prédits de tous les troisièmes bacs desdits comptes de bacs normalisés de tous les troisièmes bacs dans ledit échantillon d'essai et lesdits échantillons de référence.

8. Procédé selon l'une quelconque des revendications 5 à 7, dans lequel ladite segmentation est une segmentation binaire circulaire (CBS) ou une segmentation basée sur un modèle de Markov caché (HMM).

9. Procédé selon l'une quelconque des revendications 5 à 8, dans lequel lesdites aneuploïdies fœtales chromosomiques complètes sont trouvées dans un chromosome choisi dans le groupe constitué par le chromosome sexuel 9, 13, 15, 16, 18, 21, 22, X et le chromosome sexuel Y.

10. Procédé selon l'une quelconque des revendications 5 à 9, dans lequel lesdites aneuploïdies fœtales partielles sont associées aux syndromes choisis dans le groupe constitué par le syndrome d'Angelman, le syndrome de Prader-Willi, le syndrome du cri du chat, le syndrome de Wolf-Hirschhorn, le syndrome de Jacobsen, le syndrome de Langer-Giedion, le syndrome de DiGeorge, le syndrome de DiGeorge II et le syndrome de Phelan-McDermid.

11. Procédé selon l'une quelconque des revendications 5 à 10, dans lequel ladite aneuploïdie fœtale partielle consiste en une délétion 1p36 ou une délétion 16p11.2-p12.1.2.

12. Procédé selon l'une quelconque des revendications 5 à 11, dans lequel ladite valeur dans l'étape (h)(xi) se situe dans une plage choisie dans le groupe constitué de 0,2-4 %, 0,3-3 %, 0,4-2 %, 0,5-1,5 %, et 0,8-1,2 %.

13. Produit de programme informatique comprenant un support lisible par ordinateur, dans lequel des instructions sont codées pour commander un système informatique comprenant des moyens pour déterminer si un fœtus ayant de l'ADN fœtal risque d'avoir des altérations du nombre d'au moins un chromosome et/ou d'au moins un fragment de chromosome conformément au procédé selon l'une quelconque des revendications 5 à 12, lesdites altérations du nombre de chromosomes étant des aneuploïdies fœtales chromosomiques complètes et lesdites altérations du nombre de fragments de chromosomes étant des aneuploïdies fœtales partielles, dans un échantillon d'essai de plasma maternel contenant de l'ADN maternel et ledit ADN fœtal, ledit système informatique comprenant les moyens suivants :

(a) des moyens pour enregistrer des lectures de séquences d'ADN obtenues à partir dudit échantillon d'essai,
(b) des moyens pour aligner lesdites lectures de séquences par rapport à un génome de référence,
(c) des moyens pour déterminer, avec le produit de programme informatique selon la revendication 6, le pourcentage d'ADN fœtal dans ledit échantillon d'essai,
(d) des moyens pour diviser la séquence de chaque chromosome en des troisièmes bacs ayant une taille de base de 100 Kb à 1 Mb et obtenir des comptes de bacs en comptant le nombre de lectures de séquences dans chaque troisième bac dans ledit échantillon d'essai,
(e) des moyens pour récupérer les comptes de bacs dans chaque troisième bac dans des seconds échantillons de référence, lesdits comptes de bacs dans lesdits seconds échantillons de référence ayant été préalablement obtenus avec les moyens (a)-(d) et lesdits seconds échantillons de référence étant des échantillons de référence de plasma maternel contenant de l'ADN maternel et de l'ADN fœtal provenant de fœtus ayant un ensemble de chromosomes euploïdes,
(f) des moyens permettant d'effectuer les opérations suivantes :

(i) des moyens pour déterminer tous les troisièmes bacs autosomiques en tant que bacs normaux dans ledit échantillon d'essai,
(ii) des moyens pour obtenir des coefficients de normalisation de couverture dans ledit échantillon d'essai et lesdits seconds échantillons de référence en divisant les comptes de bacs dans chacun desdits bacs normaux via les comptes totaux dans ledit échantillon d'essai et lesdits échantillons de référence pour tous lesdits bacs normaux,
(iii) des moyens pour obtenir des comptes de bacs normalisés dans ledit échantillon d'essai et lesdits seconds échantillons de référence en multipliant lesdits comptes de bacs de tous les bacs dans ledit échantillon d'essai et lesdits échantillons de référence par lesdits coefficients de normalisation de couverture,
(iv) des moyens pour calculer un modèle de contenu GC pour ledit échantillon d'essai et un modèle de contenu GC pour chacun desdits seconds échantillons de référence par régression, en utilisant le contenu GC desdits bacs normaux comme variable indépendante et en utilisant lesdits comptes de bacs normalisés comme variable dépendante et en obtenant des comptes de bacs prédits de tous les bacs dans ledit échantillon d'essai et lesdits seconds échantillons de référence avec lesdits modèles de contenu GC,

(v) des moyens pour obtenir des résidus en calculant les différences entre les comptes de bacs normalisés de tous les troisièmes bacs et lesdits comptes de bacs prédits de tous les troisièmes bacs dans ledit échantillon d'essai et lesdits seconds échantillons de référence,

(vi) des moyens pour obtenir des résidus standardisés de tous les troisièmes bacs dans ledit échantillon d'essai en standardisant lesdits résidus de tous les troisièmes bacs dans ledit échantillon d'essai au moyen d'un score Z obtenu pour chaque bac en comparant ledit résidu de chaque troisième bac dans ledit échantillon d'essai audit résidu du troisième bac correspondant dans lesdits seconds échantillons de référence,

(vii) des moyens pour obtenir des résidus standardisés mis à l'échelle en premier dans ledit échantillon d'essai en multipliant lesdits résidus standardisés dans ledit échantillon d'essai par un premier coefficient de mise à l'échelle, ledit premier coefficient de mise à l'échelle étant l'inverse dudit pourcentage d'ADN fœtal déterminé avec les moyens (c),

(viii) des moyens pour déterminer des nombres de copies de bac pour chaque troisième bac en multipliant lesdits résidus standardisés mis à l'échelle en premier par un second coefficient de mise à l'échelle, obtenant ainsi des résidus standardisés mis à l'échelle en second, et en ajoutant un coefficient de décalage auxdits résidus standardisés mis à l'échelle en second, ledit second coefficient de mise à l'échelle ayant été préalablement déterminé à partir des résidus standardisés mis à l'échelle en premier et des nombres de copies de bac pour chaque troisième bac dans des troisièmes échantillons de référence, lesdits troisièmes échantillons de référence étant des échantillons de référence de plasma maternel contenant de l'ADN maternel et de l'ADN fœtal provenant de fœtus avec un ensemble de chromosomes aneuploïdes, ledit coefficient de décalage ayant été préalablement déterminé à partir des résidus standardisés mis à l'échelle en premier et des nombres de copies de bac pour chaque troisième bac dans lesdits seconds échantillons de référence,

(ix) des moyens pour éliminer les troisièmes bacs ayant un nombre de copies différent déterminant l'aneuploïdie du groupe desdits bacs normaux dans ledit échantillon d'essai,

(x) des moyens pour répéter les opérations visées aux points (f)(ii) à (f)(ix),

(xi) des moyens pour répéter l'opération visée au point (f)(x) si lesdits coefficients de normalisation de couverture diffèrent de plus d'une valeur comprise entre 0,1 et 5 % par rapport auxdits coefficients de normalisation de couverture précédents,

(g) des moyens pour obtenir des groupes de bacs contigus par segmentation, obtenant ainsi des segments ayant le même nombre de copies, de sorte que lorsqu'un segment donné d'un chromosome autosomique a un nombre de copies supérieur à 2,5 ou inférieur à 1,5, ledit fœtus est classé comme présentant un risque d'altération du nombre d'au moins un chromosome et/ou d'au moins un fragment de chromosome.

**14.** Procédé pour déterminer l'absence ou la présence de chromosome Y dans un échantillon d'essai de plasma maternel contenant de l'ADN maternel et de l'ADN fœtal, comprenant les étapes suivantes :

(a) extraire l'ADN dudit échantillon d'essai et réparer ledit ADN,

(b) synthétiser des banques de l'ADN présent dans ledit échantillon d'essai, amplifier et étiqueter lesdites banques par PCR et combiner lesdites banques,

(c) obtenir des lectures de séquences du chromosome Y, par séquençage massif, des banques amplifiées, étiquetées et combinées,

(d) aligner lesdites lectures de séquences du chromosome Y par rapport à une séquence du chromosome Y d'un génome de référence,

(e) diviser ladite séquence du chromosome Y en des quatrièmes bacs ayant une taille de base de 100 Kb à 1 Mb et obtenir des comptes de bacs en comptant le nombre de lectures de séquences dans chaque quatrième bac dudit chromosome dans ledit échantillon d'essai,

(f) récupérer les comptes de bacs dans chaque quatrième bac dans des seconds échantillons de référence, lesdits comptes de bacs dans lesdits seconds échantillons de référence ayant été préalablement obtenus selon les étapes (a)-(e) et lesdits seconds échantillons de référence comprenant des échantillons de référence de fœtus mâle et des échantillons de référence de fœtus femelle,

(g) obtenir des comptes de bacs normalisés dans ledit échantillon d'essai et lesdits seconds échantillons de référence en normalisant lesdits comptes de bacs de tous les bacs sur la base de la moyenne des comptes totaux dudit échantillon d'essai et desdits seconds échantillons de référence pour tous les bacs normaux dans ledit échantillon d'essai, lesdits bacs normaux étant lesdits bacs normaux déterminés à l'étape (h)(xi) du procédé selon la revendication 5,

(h) déterminer des bacs de ladite séquence du chromosome Y qui sont statistiquement différents par un premier test d'hypothèse statistique de Wilcoxon comparant lesdits comptes de bacs normalisés dans lesdits échantillons de référence de fœtus femelle aux comptes de bacs normalisés dans lesdits échantillons de référence de fœtus mâle, l'hypothèse nulle dudit premier test de Wilcoxon étant que la moyenne desdits comptes de bacs normalisés dans chaque quatrième bac dans lesdits échantillons de référence de fœtus mâle est significativement supérieure à la moyenne desdits comptes de bacs normalisés dans chaque quatrième bac dans lesdits échantillons de référence de fœtus femelle et les bacs étant déterminés comme étant statistiquement différents lorsque ladite hypothèse nulle dudit premier test de Wilcoxon est vérifiée,

(i) pour lesdits bacs de ladite séquence du chromosome Y qui sont statistiquement différents, comparer lesdits comptes de bacs normalisés dans ledit échantillon d'essai auxdits comptes normalisés dans lesdits échantillons de référence de fœtus femelle par un second test de Wilcoxon, l'hypothèse nulle dudit second test de Wilcoxon étant que la moyenne desdits comptes de bacs normalisés dans chacun desdits bacs qui est statistiquement différent dans ledit échantillon d'essai est significativement supérieure à la moyenne desdits comptes de bacs normalisés dans chacun desdits bacs qui est statistiquement différent dans lesdits échantillons de référence de fœtus femelle,

(j) pour lesdits bacs de ladite séquence du chromosome Y qui sont statistiquement différents, comparer lesdits comptes de bacs normalisés dans ledit échantillon d'essai auxdits comptes normalisés dans lesdits échantillons de référence de fœtus mâle par un troisième test de Wilcoxon, l'hypothèse nulle dudit troisième test de Wilcoxon étant que la moyenne desdits comptes de bacs normalisés dans chacun desdits bacs qui est statistiquement différent dans ledit échantillon d'essai est significativement inférieure à la moyenne desdits comptes de bacs normalisés dans chacun desdits bacs qui est statistiquement différent dans lesdits échantillons de référence de fœtus mâle,

(k) déterminer des bacs mâles lorsque lesdites deux hypothèses nulles desdits second et troisième tests de Wilcoxon sont vérifiées et déterminer des bacs femelles lorsque au moins une desdites hypothèses nulles desdits second et troisième tests de Wilcoxon n'est pas vérifiée,

(l) déterminer le nombre desdits bacs mâles et femelles et déterminer la présence du chromosome Y, le nombre desdits bacs mâles étant supérieur au nombre desdits bacs femelles, et déterminer l'absence du chromosome Y dans le cas contraire, ladite présence ou absence du chromosome Y indiquant le sexe fœtal.

15. Produit de programme informatique comprenant un support lisible par ordinateur, dans lequel des instructions sont codées pour commander un système informatique comprenant des moyens pour déterminer l'absence ou la présence du chromosome Y dans un échantillon d'essai de plasma maternel contenant de l'ADN maternel et de l'ADN fœtal selon le procédé de la revendication 14, ledit système informatique comprenant les moyens suivants :

(a) des moyens pour enregistrer des lectures de séquences d'ADN obtenues à partir dudit échantillon d'essai,

(b) des moyens pour aligner lesdites lectures de séquences du chromosome Y par rapport à une séquence du chromosome Y d'un génome de référence,

(c) des moyens pour diviser ladite séquence du chromosome Y en des quatrièmes bacs ayant une taille de base de 100 Kb à 1 Mb et obtenir des comptes de bacs en comptant le nombre de lectures de séquences dans chaque quatrième bac dudit chromosome dans ledit échantillon d'essai,

(d) des moyens pour récupérer les comptes de bacs dans chaque quatrième bac dans des seconds échantillons de référence, lesdits comptes de bacs dans lesdits seconds échantillons de référence ayant été préalablement obtenus et lesdits seconds échantillons de référence comprenant des échantillons de référence de fœtus mâle et des échantillons de référence de fœtus femelle,

(e) des moyens pour obtenir des comptes de bacs normalisés dans ledit échantillon d'essai et lesdits seconds échantillons de référence en normalisant lesdits comptes de bacs de tous les bacs sur la base de la moyenne des comptes totaux dudit échantillon d'essai et desdits seconds échantillons de référence pour tous les bacs normaux dans ledit échantillon d'essai, lesdits bacs normaux étant lesdits bacs normaux déterminés avec l'opération visée au point (f)(xi) du produit de programme informatique selon la revendication 13,

(f) des moyens pour déterminer des bacs de ladite séquence du chromosome Y qui sont statistiquement différents par un premier test d'hypothèse statistique de Wilcoxon comparant lesdits comptes de bacs normalisés dans lesdits échantillons de référence de fœtus femelle aux comptes de bacs normalisés dans lesdits échantillons de référence de fœtus mâle, l'hypothèse nulle dudit premier test de Wilcoxon étant que la moyenne desdits comptes de bacs normalisés dans chaque quatrième bac dans lesdits échantillons de référence de fœtus mâle est significativement supérieure à la moyenne desdits comptes de bacs normalisés dans chaque quatrième bac dans lesdits échantillons de référence de fœtus femelle et les bacs étant déterminés comme étant statistiquement différents lorsque ladite hypothèse nulle dudit premier test de Wilcoxon est vérifiée,

(g) des moyens pour comparer lesdits comptes de bacs normalisés dans ledit échantillon d'essai auxdits comptes normalisés dans lesdits échantillons de référence de fœtus femelle par un second test de Wilcoxon pour lesdits bacs de ladite séquence du chromosome Y qui sont statistiquement différents, l'hypothèse nulle dudit second test de Wilcoxon étant que la moyenne desdits comptes de bacs normalisés dans chacun desdits bacs qui est statistiquement différent dans ledit échantillon d'essai est significativement supérieure à la moyenne desdits comptes de bacs normalisés dans chacun desdits bacs qui est statistiquement différent dans lesdits échantillons de référence de fœtus femelle,

(h) des moyens pour comparer lesdits comptes de bacs normalisés dans ledit échantillon d'essai auxdits comptes normalisés dans lesdits échantillons de référence de fœtus mâle par un troisième test de Wilcoxon pour lesdits bacs de ladite séquence du chromosome Y qui sont statistiquement différents, l'hypothèse nulle dudit second test de Wilcoxon étant que la moyenne desdits comptes de bacs normalisés dans chacun desdits bacs qui est statistiquement différent dans ledit échantillon d'essai est significativement inférieure à la moyenne desdits comptes de bacs normalisés dans chacun desdits bacs qui est statistiquement différent dans lesdits échantillons de référence de fœtus mâle,

(i) des moyens pour déterminer des bacs mâles lorsque lesdites deux hypothèses nulles desdits second et troisième tests de Wilcoxon sont vérifiées et déterminer des bacs femelles lorsque au moins une desdites hypothèses nulles desdits second et troisième tests de Wilcoxon n'est pas vérifiée,

(j) des moyens pour déterminer le nombre desdits bacs mâles et femelles et déterminer la présence du chromosome Y, le nombre desdits bacs mâles étant supérieur au nombre desdits bacs femelles, et déterminer l'absence du chromosome Y dans le cas contraire, ladite présence ou absence du chromosome Y indiquant le sexe fœtal.

**FIGURE 1**

**FIGURE 2**

EP 3 884 502 B1

# FIGURE 3

**Calculation of the reference random forest
model for predicting the percentage of fetal DNA**

| 11 | Obtain sequence reads, by massive sequencing, of DNA libraries of reference samples |
|---|---|

| 12 | Align sequence reads against a reference genome |
|---|---|

| 13 | Divide the sequence of each chromosome into first bins with a first sliding bin and obtain bin counts by counting the number of sequence reads in each first bin in the reference samples |
|---|---|

| 14 | Obtain normalised bin counts by normalising the bin counts obtained in the previous step based on the total counts in all first bins in the reference samples |
|---|---|

| 15 | Calculate a regression for each first bin in the reference samples using the normalised number of sequence reads in each bin as independent variable; the percentage of fetal DNA as dependent variable and using gestation week, mother's height, mother's weight and mother's intake of heparin as co-variables |
|---|---|

| 16 | Identify genomic regions enriched in fetal DNA |
|---|---|

| 17 | Obtain a density of normalised bin counts for each reference sample which map to the genomic regions enriched in fetal DNA vs insert size of reads in the reference samples which map to the genomic regions enriched in fetal DNA and obtain a density curve from the values obtained, wherein the density curve is a reference nucleosome profile |
|---|---|

| 18 | Divide the reference nucleosome profile into second bins of insert sizes with a second sliding bin and calculate the value of first variables selected from the group consisting of mean, minimum, maximum and the accumulative area under the curve for each second bin |
|---|---|

| 19 | Determine the variable importance of the first variables in the references samples by a random forest classification and determining a set of first predictive variables in the reference samples, wherein the predictive variables in the set are the n-most important variables as classified by said variable importance |
|---|---|

| 20 | Generate a first matrix containing the values in the reference samples of second predictive variables, wherein the second predictive variables comprise the first predictive variables and further comprise gestation week, mother's height, mother's weight and mother's intake of heparin |
|---|---|

| 21 | Calculate a reference random forest regression model for predicting percentage of fetal DNA by growing random forest regression trees for said reference samples using part of the reference samples as a training set and part of the reference samples as a test set and using the first matrix containing the values of said second predictive variables as input to the reference random forest regression model, and by obtaining an average based on the trees obtained |
|---|---|

52

## FIGURE 3 (CONT.)

**Determination of the percentage of fetal
DNA in a test sample**

22 | Obtain sequence reads, by massive sequencing, of DNA libraries of a test sample

23 | Align sequence reads against a reference genome

24 | Divide the sequence of each chromosome into bins and obtain bin counts by counting the number of sequence reads in each first bin in the test sample

25 | Obtain normalised bin counts by normalising the bin counts based on the total counts of all first bins in the test sample

26 | Obtain a a density of normalised bin counts of reads for the test sample which map to said genomic regions enriched in fetal DNA against insert size of reads in the test sample which map to the genomic regions enriched in fetal DNA and obtain a density curve from the values obtained, wherein the density curve is a test nucleosome profile

27 | Divide the test nucleosome profile into second bins with a second sliding bin and calculate the value of the first variables selected from the group consisting of mean, minimum, maximum and the accumulative area under the curve for each second bin

28 | Generate a second matrix containing the values in the test sample of the second predictive variables

29 | Determine the value of the percentage of fetal DNA in the test sample by growing random forest regression trees using the reference random forest model for predicting percentage of fetal DNA, using the second matrix containing the values in the test sample of second predictive variables as input to the reference random forest model and obtain estimated values of the percentage of fetal DNA for each tree in the test sample, the average of the estimated values being the percentage of fetal DNA in said test sample

FIGURE 4

**FIGURE 5**

**FIGURE 6**

**FIGURE 7**

**FIGURE 8**

EP 3 884 502 B1

FIGURE 9

Obtain sequence reads, by massive sequencing, of DNA libraries of a test sample

Align sequence reads against a reference genome

Determine the percentage of fetal DNA in the test sample by nucleosome profiling

Divide each chromosome into bins and obtain bin counts by counting the number of sequence reads in each bin in the test sample

Recover bin counts for male fetus and female fetus euploid reference samples

Determine normally distributed bins: for first iteration, all autosomal bins are determined as normally distributed bins; for subsequent iterations, bins having a copy number of 2 in the test sample are determined as normally distributed bins

Obtain coverage normalisation coefficients in the test and reference samples, by dividing the total counts of all normally distributed bins by the mean of the total counts of test and reference samples for all normally distributed bins

Obtain normalised bin counts in the test and reference samples, by multiplying bin counts by the normalisation coefficients

Calculate GC-content models in the test sample and the reference samples by regression using as independent variable the GC-content of the normally distributed bins and as dependent variable the normalised bin counts and obtaining predicted bin counts of all bins with the GC-content models

Obtain residuals by calculating differences between the normalised bin counts of all bins and the bin counts of all bins predicted with the GC-content models in the test sample and the reference samples

Obtain groups of contiguous bins by segmentation, thereby obtaining segments having the same copy number, whereby when any given segment of an autosomal chromosome has a copy number greater than 2.5 or less than 1.5, said fetus is classified as being at risk of having alterations in the number of at least one chromosome and/or at least one chromosome fragment

YES

Convergence of normalisation coefficients?

NO

Determine bin copy numbers (real numbers) by multiplying first-scaled standardised residuals by a second scaling coefficient an adding a shifting coefficient

Obtain first-scaled standardised residuals in the test sample by multiplying standardised residuals by the inverse of the percentage of fetal DNA determined previously by nucleosome profiling

Standardise the residuals of all bins in the test sample by means of a Z-score obtained for every bin by comparing the residual in the test sample to the residuals of the reference samples

59

**FIGURE 10**

FIGURE 11

**FIGURE 12**

**FIGURE 13**

## Determination of the presence or absence
## of the Y-chromosome in a test sample

| | |
|---|---|
| 51 | Obtain sequence reads of the Y-chromosome, by massive sequencing, of DNA libraries in a test sample |

| | |
|---|---|
| 52 | Align sequence reads of the Y-chromosome against a sequence of the Y-chromosome of a reference genome |

| | |
|---|---|
| 53 | Divide the sequence of the Y-chromosome into fourth bins and obtain bin counts by counting the number of sequence reads in each fourth bin of the chromosome in the test sample |

| | |
|---|---|
| 54 | Recover bin counts of Y-chromosome for male fetus and female fetus euploid reference samples |

| | |
|---|---|
| 55 | Obtain normalised bin counts in the test sample and the reference samples by normalising said bin counts of all bins based on the mean of the total counts of the test sample and the reference samples for all normal bins in the test sample |

| | |
|---|---|
| 56 | Determine bins of said sequence of the Y-chromosome that are statistically different by a first Wilcoxon statistical hypothesis test comparing the normalised bin counts in the female fetus reference samples to normalised bin counts in the male fetus reference samples, wherein the null hypothesis of the first Wilcoxon test is that the mean of the normalised bin counts in each fourth bin in the male fetus reference samples is significantly greater than the mean of the normalised bin counts in each fourth bin in the female fetus reference samples and wherein bins are determined statistically different when the null hypothesis of the first Wilcoxon test is verified as true |

| | |
|---|---|
| 57 | For the bins of the sequence of the Y-chromosome that are statistically different, compare the normalised bin counts in the test sample to the normalised counts in the female fetus reference samples by a second Wilcoxon test, wherein the null hypothesis of the second Wilcoxon test is that the mean of the normalised bin counts in each bin that is statistically different in the test sample is significantly greater than the mean of the normalised bin counts in each bin that is statistically different in the female fetus reference samples |

| | |
|---|---|
| 58 | For the bins of the sequence of the Y-chromosome that are statistically different, compare the normalised bin counts in the test sample to the normalised counts in the male fetus reference samples by a third Wilcoxon test, wherein the null hypothesis of the third Wilcoxon test is that the mean of the normalised bin counts in each bin that is statistically different in the test sample is significantly less than the mean of said normalised bin counts in each bin that is statistically different in male fetus reference samples |

| | |
|---|---|
| 59 | Determine male bins when both null hypotheses of the second and third Wilcoxon tests are verified as true and determine female bins when at least one of the null hypotheses of the second and third Wilcoxon tests are not verified as true |

| | |
|---|---|
| 60 | Determine the number of said male and female bins and determine the presence of the Y-chromosome wherein the number of the male bins is higher than the number of the female bins and determine the absence of the Y-chromosome otherwise |

**FIGURE 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013132305 A1
- WO 2015061359 A1
- WO 2013109981 A1
- US 2018089364 A1

**Non-patent literature cited in the description**

- **LAM WKJ et al.** Sequencing-based counting and size profiling of plasma Epstein-Barr virus DNA enhance population screening of nasopharyngeal carcinoma. *Proc. Natl. Acad. Sci. USA.,* 2018, vol. 115 (22), E5115-E5124
- **LOVE MI et al.** Moderated estimation of fold change and dispersion for RNA-seq data with DESeq2. *Genome Biology,* 2014, vol. 15, 550
- **KUHN S et al.** Building blocks for automated elucidation of metabolites: machine learning methods for NMR prediction. *BMC Bioinformatics,* 2008, vol. 9, 400
- **LIAW A ; WIENER M.** Classification and Regression by randomForest. *R News,* 2002, vol. 2 (3), 18-22
- **HUDECOVA I et al.** Maternal plasma fetal DNA fractions in pregnancies with low and high risks for fetal chromosomal aneuploidies. *PLoS One,* 2014, vol. 9 (2